# EUROPEAN PATENT APPLICATION

(11) **EP 4 703 381 A1**
(43) Date of publication of application: **04.03.2026**
(21) Application number: 24382946.2
(22) Date of filing: 03.09.2024
(51) Int. Cl.: C07K 16/28, A61P 29/00, A61P 35/00, A61K 39/00

(54) **ANTIBODIES AGAINST ICAM-1 AND APPLICATIONS THEREOF**

(71) Applicant: Institució Catalana de Recerca i Estudis Avançats, 08010 Barcelona (ES); Fundació Institut de Bioenginyeria de Catalunya (IBEC), 08028 Barcelona (ES)
(72) Inventor: MURO GALINDO, Silvia, E-08028 Barcelona (ES); VIGO, Marco, E-08028 Barcelona (ES); PLACCI, Marina, E-08028 Barcelona (ES)
(74) Representative: ABG Intellectual Property Law, S.L.

(57) **Abstract**

The invention relates to antibodies and to fragments thereof that bind specifically to the ICAM-1 protein, and to carriers comprising said antibodies or fragments thereof. The invention also relates to uses thereof in the diagnosis, prognosis, prevention and/or treatment of a disease or condition associated to the ICAM-1 protein expression using said antibodies and to methods, pharmaceutical compositions, kits and uses thereof.

## Description

### FIELD OF THE INVENTION

The present invention relates to antibodies binding specifically to the ICAM-1 protein, to uses thereof, and to methods and kits using said antibodies.

### BACKGROUND OF THE INVENTION

Intercellular adhesion molecule 1 (ICAM-1), also called BB2, CD54, or P3.58, is a glycoprotein of the immunoglobulin (Ig) superfamily. ICAM-1 has a large extracellular segment comprising five Ig-like domains, named D1 to D5, which is glycosylated at various sites. This is followed by a single transmembrane region and a short cytoplasmic tail that interacts with other proteins and relays signals intracellularly. The ICAM-1 protein is mostly expressed on the plasma membrane of vascular and immune cells. Additionally, soluble ICAM-1 has been identified in the circulation, extracellular space, and other body fluids. ICAM-1 expression levels at all these sites vary: its basal expression in most physiological conditions is generally low, while many types of stimulations and pathological alterations can increase ICAM-1 expression. In other instances, ICAM-1 levels can decrease by physical (e.g. light) or chemical (e.g. nitric oxide) signals. Such stimulatory or downregulatory signals can result from either endogenous body processes or exogenously applied factors or agents.

ICAM-1 is involved in numerous cellular and body functions. For instance, ICAM-1 facilitates the recruitment of immune cells to inflamed, damaged, or infected sites in the body due to its role as an adhesion molecule mediating the binding of leukocytes to endothelial cells lining the vasculature, which helps leukocytes migrate into the affected tissue to initiate an adaptive or innate immune response. ICAM-1 also contributes to immune surveillance by facilitating the interaction between immune cells, enabling them to recognize and respond to potential threats. This protein is involved in the immune synapse and antigen (Ag) presentation, which is crucial for the activation of specific immune responses. Additionally, ICAM-1 is indirectly involved in thrombosis and blood clotting, which can be mediated by interaction with platelets, fibrinogen-derived fragments, and ICAM-1 association with a pro-inflammatory state that can alter the hemostatic balance. ICAM-1 has also been related with tumorigenesis, cancer progression, metastasis, and the immune response against cancer. It plays a role in infection, not only through respective function in inflammation and the immune response, but also through interaction with certain pathogens or pathogen-infected cells or vesicles. Given its expression in inflammation, ICAM-1 is involved in oxidative stress, metabolic syndromes, pulmonary and cardiovascular diseases, neurological conditions, genetic maladies, cancers, infections, and diseases associated with any of the tissues and/or functions in which ICAM-1 is expressed and involved.

ICAM-1 targeting can be achieved using ICAM-1 binders, such as antibodies (Ab) or their fragments, either applied as such, coupled to agents, or coupled to delivery systems carrying them. The binding of ICAM-1 binders or ICAM-1-binder-bearing systems to the extracellular domain of this molecule generally renders cellular signaling events involved in diverse phenomena related to ICAM-1 functions, such as ICAM-1 binders or derivative systems.

Therefore, any tools capable of binding to ICAM-1 could be used: (i) to detect, measure, and/or alter its expression, (ii) as markers and/or modifiers of the patho-physiological processes in which ICAM-1 participates, (iii) to detect and/or modify ICAM-1's structural regions and/or their associated functions; whereas all of this could be used for either research, diagnostic, and/or therapeutic purposes. Therefore, there is a need to provide agents recognizing ICAM-1 specifically that are suitable for the diagnosis, prognosis, prevention and/or treatment of a disease or condition concomitant with the expression of the ICAM-1 protein.

### SUMMARY OF THE INVENTION

In a first aspect, the invention relates to an antibody binding specifically to the ICAM-1 protein or an antigen-binding fragment thereof, wherein the antibody or antigen-binding fragment thereof is selected from the group consisting of:
(a) an antibody or antigen-binding fragment thereof, wherein the antibody comprises the CDR1, CDR2 and CDR3 within the heavy chain of the variable region comprising respectively the sequences set forth in SEQ ID NO: 5, 6 and 7, or a functionally equivalent variant thereof, and the CDR1, CDR2 and CDR3 within the light chain comprising respectively the sequences set forth in SEQ ID NO: 8, 9 and 10, or a functionally equivalent variant thereof; and
(b) an antibody or antigen-binding fragment thereof, wherein the antibody comprises the CDR1, CDR2 and CDR3 within the heavy chain of the variable region comprising respectively the sequences set forth in SEQ ID NO: 11, 12 and 13, or a functionally equivalent variant thereof, and the CDR1, CDR2 and CDR3 within the light chain comprising respectively the sequences set forth in SEQ ID NO: 14, 15 and 16, or a functionally equivalent variant thereof.

In a second aspect, the invention relates to a carrier further comprising the antibody or antigen-binding fragment thereof according to the first aspect.

In a third aspect, the invention relates to a nucleic acid selected form the group consisting of:
(i) a nucleic acid coding for the antibody or antigen-binding fragment thereof according to the first aspect; and
(ii) a complementary nucleic acid of a nucleic acid as defined in (i).

In a fourth aspect, the invention relates to a gene construct comprising the nucleic acid according to the third aspect.

In a fifth aspect, the invention relates to an expression cassette comprising the nucleic acid according to the third aspect or the gene construct according to the fourth aspect.

In a sixth aspect, the invention relates to a vector comprising the nucleic acid according to the third aspect, the gene construct according to the fourth aspect, or the expression cassette according to the fifth aspect.

In a seventh aspect, the invention relates to a cell comprising the nucleic acid according to the third aspect, the gene construct according to the fourth aspect, the expression cassette according to the fifth aspect, or the vector according to the sixth aspect.

In an eighth aspect, the invention relates to a living organism comprising the nucleic acid according to the third aspect, the gene construct according to the fourth aspect, the expression cassette according to the fifth aspect, or the vector according to the sixth aspect

In a ninth aspect, the invention relates to a method for producing the antibody or antigen-binding fragment thereof according to the first aspect, comprising growing the cell according to the seventh aspect, or the living organism according to the eighth aspect, under conditions permitting the production of said antibody or antigen-binding fragment thereof.

In a tenth aspect, the invention relates to an *in vitro* method for the diagnosis and/or prognosis of a disease or condition associated to the ICAM-1 protein expression in a subject, comprising:
(a) contacting the antibody or antigen-binding fragment thereof according to the first aspect, or the carrier according to the second aspect, with a sample from said subject;
(b) detecting and/or quantifying the level and/or location of said antibody or antigen-binding fragment thereof, or said carrier bound to the ICAM-1 protein in said sample;
(c) comparing the level and/or location of said antibody or antigen-binding fragment thereof, or said carrier bound to the ICAM-1 protein detected in step (b) with that of a reference value; and
(d) correlating the result obtained with the presence of a disease or condition associated to the ICAM-1 protein expression.

In an eleventh aspect, the invention relates to an *in vitro* method for monitoring the response to a treatment of a disease or condition associated to the ICAM-1 protein expression in a subject under treatment, comprising:
(a) contacting the antibody or antigen-binding fragment thereof according to the first aspect, or the carrier according to the second aspect, with a first sample from said subject taken at a first time-point;
(b) detecting and/or quantifying the level and/or location of said antibody or antigen-binding fragment thereof, or said carrier bound to the ICAM-1 protein in said first sample;
(c) contacting the antibody or antigen-binding fragment thereof according to the first aspect, or the carrier according to the second aspect, with a second sample from said subject taken at a second time-point;
(d) detecting and/or quantifying the level and/or location of said antibody or antigen-binding fragment thereof, or said carrier bound to the ICAM-1 protein in said second sample;
(e) comparing the level and/or location of said antibody or antigen-binding fragment thereof, or said carrier bound to the ICAM-1 protein detected in step (b) with the level and/or location of said antibody or antigen-binding fragment thereof, or said carrier bound to the ICAM-1 protein detected in step (d); and
(f) correlating the result obtained with the response to the treatment of a disease or condition associated to the ICAM-1 protein expression.

In a twelfth aspect, the invention relates to an *in vitro* method for detecting, localizing and/or quantifying the presence of the ICAM-1 protein, or cells expressing the ICAM-1 protein, in a sample, comprising:
(i) contacting the test sample with the antibody or antigen-binding fragment thereof according to the first aspect, or the carrier according to the second aspect; and
(ii) detecting, localizing and/or quantifying the formation of immune complexes with said antibody or antigen-binding fragment thereof, or said carrier.

In a thirteenth aspect, the invention relates to a pharmaceutical composition comprising the antibody or antigen-binding fragment thereof according to the first aspect, or the carrier according to the second aspect, and a pharmaceutically acceptable excipient.

In a fourteenth aspect, the invention relates to the antibody or antigen-binding fragment thereof according to the first aspect, the carrier according to the second aspect, or the pharmaceutical composition according to the thirteenth aspect, for use in medicine.

In a fifteenth aspect, the invention relates to the antibody or antigen-binding fragment thereof according to the first aspect, the carrier according to the second aspect, or the pharmaceutical composition according to the thirteenth aspect, for use in the treatment and/or prevention of a disease or condition associated to the ICAM-1 protein expression.

In a sixteenth aspect, the invention relates to the antibody or antigen-binding fragment thereof according to the first aspect, the carrier according to the second aspect, or the pharmaceutical composition according to the thirteenth aspect, for use in a method of diagnosis and/or prognosis *in vivo* of a disease or condition associated to the ICAM-1 protein expression.

In a seventeenth aspect, the invention relates to the use of the antibody or antigen-binding fragment thereof according to the first aspect, the carrier according to the second aspect, or the pharmaceutical composition according to the thirteenth aspect, for targeting a drug to a specific site wherein said specific site comprises the presence of the ICAM-1 protein or said specific site comprises cells expressing ICAM-1 (ICAM-1⁺ cells).

In an eighteenth aspect, the invention relates to the use of the antibody or antigen-binding fragment thereof according to the first aspect, or the carrier according to the second aspect, as a tool in biotechnology techniques for detection, localization and/or quantification of the ICAM-1 protein in a sample, or in the detection, localization and/or quantification of the ICAM-1 protein, or cells expressing the ICAM-1 protein, present in a sample.

In a nineteenth aspect, the invention relates to a kit comprising at least one antibody or antigen-binding fragment thereof according to the first aspect, the carrier according to the second aspect, or the pharmaceutical composition according to the thirteenth aspect.

In a twentieth aspect, the invention relates to the use of the kit of the nineteenth aspect:
- for the diagnosis and/or prognosis of a disease or condition associated to the ICAM-1 protein expression; or
- for monitoring the response to a treatment of a disease or condition associated to the ICAM-1 protein expression in a subject under treatment; or
- for detecting, localizing and/or quantifying the ICAM-1 protein, or cells expressing the ICAM-1 protein, present in a sample; or
- for the treatment and/or prevention of a disease or condition associated to the ICAM-1 protein expression; or
- for targeting a drug to a specific site wherein said specific site comprises the presence of the ICAM-1 protein or said specific site comprises cells expressing ICAM-1 (ICAM-1⁺ cells); or
- as a tool in biotechnology techniques for detection, localization and/or quantification of the ICAM-1 protein in a sample, or in the detection, localization and/or quantification of the ICAM-1 protein, or cells expressing the ICAM-1 protein, present in a sample.

### DESCRIPTION OF THE FIGURES

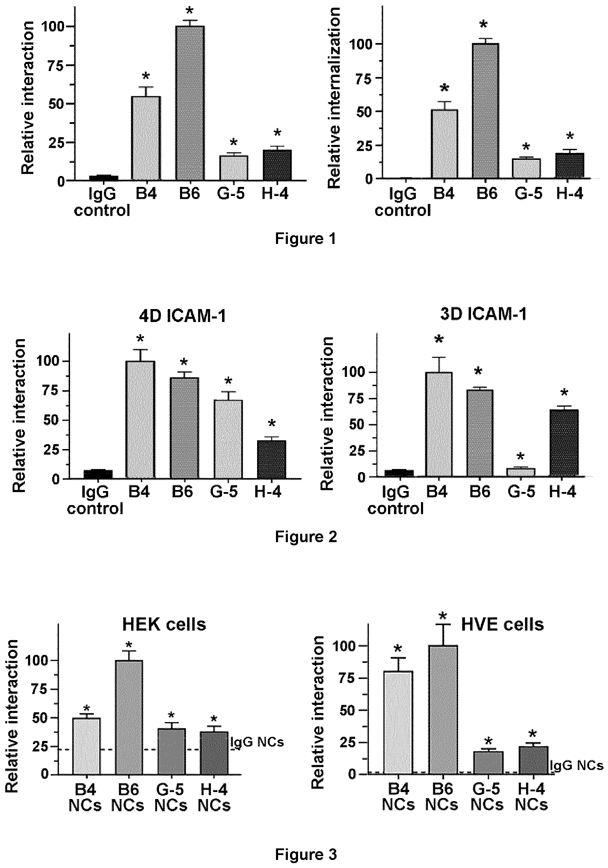

**Figure 1****: Specific interaction and uptake of anti-ICAM-1 Abs in ICAM-1-expressing cells.** Quantification by fluorescence microscopy of new anti-ICAM-1 Abs B4 and B6 vs. commercial anti-ICAM-1 Abs G-5 and H-4, associated with ICAM-1-expressing HEK-293T cells, both compared to a non-specific Ab (IgG control). (Left) Total amount of Abs associated with cells, measured as sum intensity per cell. (Right) Ab amounts found intracellularly, measured as internalized sum intensity per cell. Data are mean±standard error of the mean, calculated as relative values compared to the best performer, which is given a value of 100. (* p < 0.05 compared to IgG control, by Student's t-test).
**Figure 2****: Interaction of anti-ICAM-1 Abs with cells expressing different ICAM-1 variants.** Quantification by fluorescence microscopy of the interaction of new anti-ICAM-1 Abs B4 and B6 vs. commercial anti-ICAM-1 Abs G-5 and H-4, both compared to a non-specific Ab (IgG control), with HEK-293T cells that express (left) ICAM-1 missing one extracellular domain (4D ICAM-1) or (right) ICAM-1 missing two extracellular domains (3D ICAM-1). Data were analyzed as sum intensity per cell, calculated as relative values compared to the best performer, which is given a value of 100, and expressed as mean±standard error of the mean. (* p < 0.05 compared to IgG control, by Student's t-test).
**Figure 3****: Specific interaction of nanoparticles coated with anti-ICAM-1 Abs in cell expressing ICAM-1.** Quantification by fluorescence microscopy of the total number of nanoparticles (herein called nanocarriers; NCs) coated with non-specific IgG control (dashed line) vs. anti-ICAM-1 Abs (bars), either new (B4 or B6) or commercial (G-5 or H-4). (Left) Total number of NCs interacting with HEK-293T cells recombinantly expressing ICAM-1. (Right) Similar data in human vascular endothelial (HVE) cells treated with TNFα to mimic an inflammatory condition. Data are mean±standard error of the mean, calculated as relative values compared to the best performer, which is given a value of 100. (* p < 0.05 by Student's t-test against nanoparticles coated with non-specific IgG control).

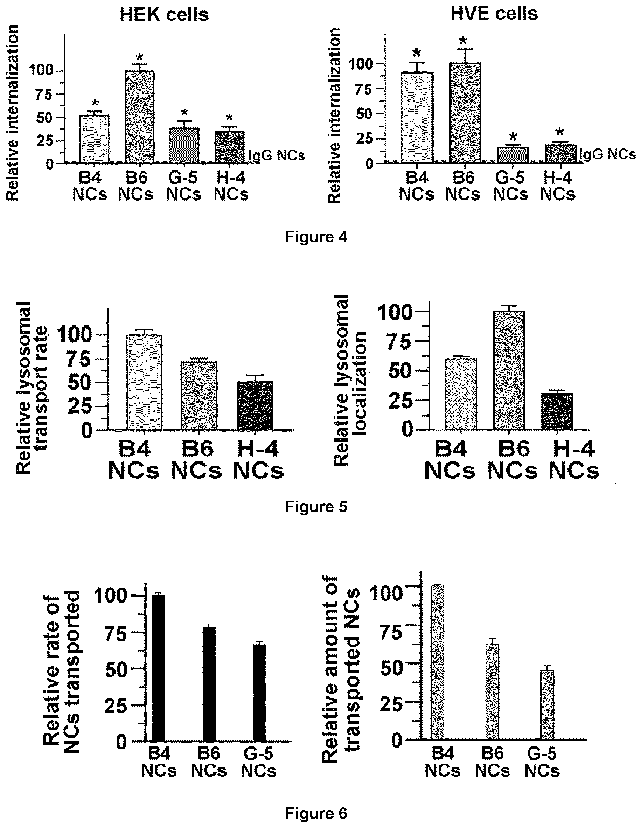

**Figure 4****: Specific uptake of nanoparticles coated with anti-ICAM-1 Abs in cell expressing ICAM-1.** Quantification by fluorescence microscopy of the number of nanoparticles (called nanocarriers; NCs) coated with non-specific IgG control (dashed line) vs. anti-ICAM-1 Abs (bars), either new (B4 or B6) or commercial (G-5 or H-4), which were found inside of: (Left) each HEK-293T cell recombinantly expressing ICAM-1 or (Right) each human vascular endothelial (HVE) cell treated with TNFα to mimic an inflammatory condition. Data are mean±standard error of the mean, calculated as relative values compared to the best performer, which is given a value of 100. (* p < 0.05 by Student's t-test against nanoparticles coated with non-specific IgG control).
**Figure 5****: Transport into lysosomes of nanoparticles coated with anti-ICAM-1 Abs in cell expressing ICAM-1.** Fluorescence microscopy quantification of the amount of nanoparticles (herein called nanocarriers; NCs) coated with new anti-ICAM-1 Abs B4 or B6 compared to commercial Ab H-4, which colocalized with anti-LAMP-1-labeled lysosomes, examined after 1 hour incubation with HEK293T cells expressing ICAM-1. (Left) Results calculated as a percentage of nanoparticles colocalizing with lysosomes out of the total number of nanoparticles interacting with cells, which represents the transport rate. (Right) Absolute number of nanoparticles found in the lysosomes of each cell. Data are mean±standard error of the mean, calculated as relative values compared to the best performer, which is given a value of 100.
**Figure 6****: Transport of nanoparticles coated with anti-ICAM-1 Abs across a blood-brain cell barrier model.** Radiometric quantification of the transport of ¹²⁵I-labeled Ab-coated nanoparticles, also called nanocarriers (NCs), from the apical chamber above a monolayer of TNFα-treated human brain endothelial cells to the basolateral chamber below. (Left) Rate of transport represented as the percentage of crossing nanoparticles out of all interacting nanoparticles after 1 hour incubation. (Right) Absolute number of nanoparticles that crossed the cellular barrier after 24 hours. Data are mean±standard error of the mean, calculated as relative values compared to the best performer, which is given a value of 100.

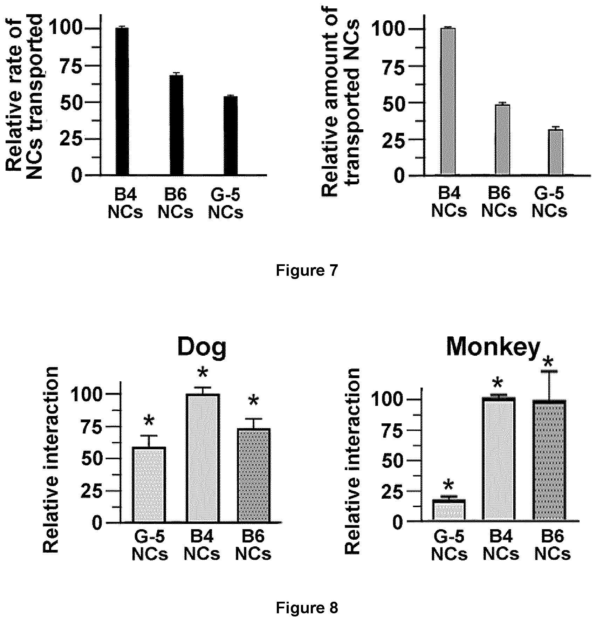

**Figure 7****: Transport of nanoparticles coated with anti-ICAM-1 Abs across a blood-lung cell barrier model.** Radiometric quantification of the transport of ¹²⁵I-labeled Ab-coated nanoparticles, also called nanocarriers (NCs), from the apical chamber above a monolayer of TNFα-treated human pulmonary endothelial cells to the basolateral chamber below. (Left) Rate of transport represented as the percentage of crossing nanoparticles out of all interacting nanoparticles after 1 hour incubation. (Right) Absolute number of nanoparticles that crossed the cellular barrier after 24 hours. Data are mean±standard error of the mean, calculated as relative values compared to the best performer, which is given a value of 100.
**Figure 8****: Species cross-reactivity of nanoparticles coated with anti-ICAM-1 Abs.** Quantification by fluorescence microscopy of the total amount of nanoparticles (also called nanocarriers; NCs) coated with new anti-ICAM-1 Abs B4 or B6 or commercial Ab G-5 as an example, which interacted with each HEK-293T cell expressing ICAM-1 from either one of two other species, namely dog (left) or Cynomolgus monkey (right) (all previous figures referred to human ICAM-1), after 1 hour incubation. Data are mean±standard error of the mean, calculated as relative values compared to the best performer, which is given a value of 100. (* p < 0.05 by Student's t-test, compared to control IgG NCs which is not shown here for simplicity).

### DETAILED DESCRIPTION OF THE INVENTION

The authors of the present invention have discovered two new antibodies with unexpectedly improved functions compared to commercial antibodies. These improved functions encompass: (a) recognition of ICAM-1 as a purified protein or a protein expressed by/on/in cells; (b) binding to ICAM-1 from human and other animal species; (c) recognition of ICAM-1 variants; (d) transport into cells, to subcellular compartments such as lysosomes, and/or across cells; and (e) activity as single molecules or coupled constructs. These new antibodies can be valuable tools in ICAM-1-related research, diagnostic, and/or therapeutic purposes.

### Antibodies of the invention

In a first aspect, the invention relates to an antibody binding specifically to the ICAM-1 protein or an antigen-binding fragment thereof, hereinafter referred to as the "antibody of the invention", selected from the group consisting of:
(a) an antibody or an antigen-binding fragment thereof, wherein the antibody comprises the CDR1, CDR2 and CDR3 within the heavy chain of the variable region comprising respectively the sequences set forth in SEQ ID NO: 5, 6 and 7, or a functionally equivalent variant thereof, and the CDR1, CDR2 and CDR3 within the light chain comprising respectively the sequences set forth in SEQ ID NO: 8, 9 and 10, or a functionally equivalent variant thereof; and
(b) an antibody or an antigen-binding fragment thereof, wherein the antibody comprises the CDR1, CDR2 and CDR3 within the heavy chain of the variable region comprising respectively the sequences set forth in SEQ ID NO: 11, 12 and 13, or a functionally equivalent variant thereof, and the CDR1, CDR2 and CDR3 within the light chain comprising respectively the sequences set forth in SEQ ID NO: 14, 15 and 16, or a functionally equivalent variant thereof.

The term "antibody" (Ab), in the context of the present invention, refers to an immunoglobulin (Ig) molecule, a fragment of an immunoglobulin molecule, or a derivative of either thereof, which has the ability to specifically bind to an antigen under typical physiological conditions. The term "antibody" relates to a monomeric or multimeric protein which comprises at least one polypeptide having the capacity for binding to a determined antigen and comprising all or part of the light or heavy chain variable region of an immunoglobulin molecule.

It is well known that the basic structural unit of an antibody comprises a tetramer. Each tetramer is constituted by two identical pairs of polypeptide chains, each of which is composed by a light chain (25 KDa) and by a heavy chain (50-75 KDa). The amino-terminal region of each chain includes a variable region of about 100-110 or more amino acids, which is involved in antigen recognition. The carboxy-terminal region of each chain comprises the constant region that mediates the effector function. The term "variable region" or "variable domain" refers to the domain of an antibody heavy or light chain that is involved in binding the antibody to antigen. The variable regions of each pair of light (VL) and heavy chains (VH) form the binding site of the antibody. They are characterized by the same general structure constituted by relatively preserved regions called frameworks (FR) joined by three hypervariable regions (HVR) called complementarity determining regions (CDR). The term "Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following order in VH-FR1, VH-FR2, VH-FR3, VH-FR4 and VL-FR1, VL-FR2, VL-FR3, VL-FR4. The term "CDRs", "hypervariable region", "HVR", "complementarity determining regions" or as used herein, refers to each of the regions of an antibody variable domain which are hypervariable in sequence and/or form structurally defined loops ("hypervariable loops"). Generally, native four-chain antibodies comprise six CDRs; three in the VH (H1, H2, H3), and three in the VL (L1, L2, L3). Thus, CDRs determine the protein's affinity (roughly, bonding strength) and specificity for specific antigens. The CDRs of the two chains of each pair are aligned by the framework regions, acquiring the function of binding a specific epitope. Consequently, both the heavy variable chain and the light variable chain are characterized by three CDRs, respectively VH-CDR1, VH-CDR2, VH-CDR3 and VL-CDR1, VL-CDR2, VL-CDR3.

In a particular embodiment, the antibody of the invention comprises:
(a) the FR1, FR2, FR3 and FR4 regions within the heavy chain of the variable region of the antibody or antigen-binding fragment thereof according to the first aspect of the invention option (a) comprising respectively the sequences set forth in SEQ ID NO: 17, 18, 19 and 20, or a functionally equivalent variant thereof, and the FR1, FR2, FR3 and FR4 regions within the light chain of the variable region of the antibody or antigen-binding fragment thereof according to the first aspect of the invention option (a) comprising respectively the sequences set forth in SEQ ID NO: 21, 22, 23 and 24, or a functionally equivalent variant thereof; and
(b) the FR1, FR2, FR3 and FR4 within the heavy chain of the variable region of the antibody or antigen-binding fragment thereof according to the first aspect of the invention option (b) comprising respectively the sequences set forth in SEQ ID NO: 25, 26, 27 and 28, or a functionally equivalent variant thereof, and the FR1, FR2, FR3 and FR4 regions within the light chain of the variable region of the antibody or antigen-binding fragment thereof according to the first aspect of the invention option (b) comprising respectively the sequences set forth in SEQ ID NO: 29, 30, 31 and 32, or a functionally equivalent variant thereof.

In another particular embodiment, the antibody of the invention comprises:
(a) at least a heavy chain of the variable region (VH) of the antibody or antigen-binding fragment thereof according to the first aspect of the invention option (a) comprising the sequence set forth in SEQ ID NO: 1 or a functionally equivalent variant thereof, and at least a light chain of the variable region (VL) according to the first aspect of the invention option (a) comprising the sequence set forth in SEQ ID NO: 2 or a functionally equivalent variant thereof; and
(b) at least a heavy chain of the variable region (VH) according to the first aspect of the invention option (b) comprising the sequence set forth in SEQ ID NO: 3 or a functionally equivalent variant thereof, and at least a light chain of the variable region (VL) according to the first aspect of the invention option (b) comprising the sequence set forth in SEQ ID NO: 4 or a functionally equivalent variant thereof.

The sequences set forth in SEQ ID NOs: 1 to 32 are defined in Table 1.

**Table 1. Sequences of the heavy and light chain of the variable regions of the antibodies of the invention and sequences of the CDRs and FRs of said heavy and light chain of the variable regions of the antibodies of the invention.**

| SEQ ID NO: | Region | Sequence |
|---|---|---|
| 1 | VH | QVQLVESGGGLVQPGGSLRLSCSASGFTFSSYAMHWV RQAPGKGLEYVSAISSNGGSTYYADSVKGRFTISRDNSK NTLYLQMSSLRAEDTAVYYCVKGSNTKQWLAGGYYYYG MDVWGQGTTVTVSS |
| 2 | VL | AIRMTQSPSSVSASVGDRVTITCRASQGISSWLAWYQQ KPGKAPKLLIYAASSLQSGVPSRFSGSGSGTDFTLTISSL QPEDFATYYCQQANSFPYTFGQGTKVDIK |
| 3 | VH | QMQLVQSGGGVVQPGRSLRLSCAASGLTFSSYGMHWV RQAPGKGLEWVAVISYDGSNKYYADSVKGRFTISRDNS KNTLYLQMNSLRAEDTAVYYCAKDFYNWNDWHDAFDI WGQGTMVTVSS |
| 4 | VL | DIVMTQSPSSLSASVGDSVTITCRASQDISNYLAWYQQK PGKAPKLLIFAASTLQSGVPSRFSGSGSGTDFALTISSLQ PEDVASYYCQKYNHVPQTFGPGTQVDIK |
| 5 | VH-CDR1 | GFTFSSYA |
| 6 | VH-CDR2 | ISSNGGST |
| 7 | VH-CDR3 | VKGSNTKQWLAGGYYYYGMDV |
| 8 | VL-CDR1 | QGISSW |
| 9 | VL-CDR2 | AAS |
| 10 | VL-CDR3 | QQANSFPYT |
| 11 | VH-CDR1 | GLTFSSYG |
| 12 | VH-CDR2 | ISYDGSNK |
| 13 | VH-CDR3 | AKDFYNWNDWHDAFDI |
| 14 | VL-CDR1 | QDISNY |
| 15 | VL-CDR2 | AAS |
| 16 | VL-CDR3 | QKYNHVPQT |
| 17 | VH-FR1 | QVQLVESGGGLVQPGGSLRLSCSAS |
| 18 | VH-FR2 | MHWVRQAPGKGLEYVSA |
| 19 | VH-FR3 | YYADSVKGRFTISRDNSKNTLYLQMSSLRAEDTAVYYC |
| 20 | VH-FR4 | WGQGTTVTVSS |
| 21 | VL-FR1 | AIRMTQSPSSVSASVGDRVTITCRAS |
| 22 | VL-FR2 | LAWYQQKPGKAPKLLIY |
| 23 | VL-FR3 | SLQSGVPSRFSGSGSGTDFTLTISSLQPEDFATYYC |
| 24 | VL-FR4 | FGQGTKVDIK |
| 25 | VH-FR1 | QMQLVQSGGGVVQPGRSLRLSCAAS |
| 26 | VH-FR2 | MHWVRQAPGKGLEWVAV |
| 27 | VH-FR3 | YYADSVKGRFTISRDNSKNTLYLQMNSLRAEDTAVYYC |
| 28 | VH-FR4 | WGQGTMVTVSS |
| 29 | VL-FR1 | DIVMTQSPSSLSASVGDSVTITCRAS |
| 30 | VL-FR2 | LAWYQQKPGKAPKLLIF |
| 31 | VL-FR3 | TLQSGVPSRFSGSGSGTDFALTISSLQPEDVASYYC |
| 32 | VL-FR4 | FGPGTQVDIK |

In an embodiment, the CDR1 within the heavy chain of the variable region of the antibody or antigen-binding fragment thereof according to the first aspect of the invention option (a) consists on SEQ ID NO: 5, the CDR2 within the heavy chain of the variable region of the antibody or antigen-binding fragment thereof according to the first aspect of the invention option (a) consists on SEQ ID NO: 6, the CDR3 within the heavy chain of the variable region of the antibody or antigen-binding fragment thereof according to the first aspect of the invention option (a) consists on SEQ ID NO: 7, the CDR1 within the light chain of the variable region of the antibody or antigen-binding fragment thereof according to the first aspect of the invention option (a) consists on SEQ ID NO: 8, the CDR2 within the light chain of the variable region of the antibody or antigen-binding fragment thereof according to the first aspect of the invention option (a) consists on SEQ ID NO: 9, the CDR3 within the light chain of the variable region of the antibody or antigen-binding fragment thereof according to the first aspect of the invention option (a) consists on SEQ ID NO: 10.

In another embodiment, the CDR1 within the heavy chain of the variable region of the antibody or antigen-binding fragment thereof according to the first aspect of the invention option (b) consists on SEQ ID NO: 11, the CDR2 within the heavy chain of the variable region of the antibody or antigen-binding fragment thereof according to the first aspect of the invention option (b) consists on SEQ ID NO: 12, the CDR3 within the heavy chain of the variable region of the antibody or antigen-binding fragment thereof according to the first aspect of the invention option (b) consists on SEQ ID NO: 13, the CDR1 within the light chain of the variable region of the antibody or antigen-binding fragment thereof according to the first aspect of the invention option (b) consists on SEQ ID NO: 14, the CDR2 within the light chain of the variable region of the antibody or antigen-binding fragment thereof according to the first aspect of the invention option (b) consists on SEQ ID NO: 15, the CDR3 within the light chain of the variable region of the antibody or antigen-binding fragment thereof according to the first aspect of the invention option (b) consists on SEQ ID NO: 16.

The term "antibody" includes any type of known antibody, such as, for example, polyclonal antibodies, monoclonal antibodies, and genetically engineered antibodies, such as chimeric antibodies, humanized antibodies, primatized antibodies, human antibodies, antibodies of non-human origin, bispecific antibodies, single domain antibodies, VHH antibodies, caninized antibodies, felinized antibodies, canine antibodies, and feline antibodies. "Monoclonal antibodies" (mAbs) are homogenous, highly specific antibody populations directed against a single site or antigenic "determinant". "Polyclonal antibodies" include heterogeneous antibody populations directed against different antigenic determinants.

In an embodiment, the antibody of the invention is an antibody of non-human origin, preferably of murine origin. In a particular embodiment, the antibody of the invention is an antibody of human origin or a humanized antibody. In a particular embodiment, the antibody of the invention is an animal antibody, a chimeric antibody, or a humanized antibody. In an embodiment, the antibody of the invention is a monoclonal antibody. In another embodiment, the antibody of the invention is a polyclonal antibody.

The antibody of the invention can be of any isotype. The choice of isotype typically will be guided by the desired effector functions, such as ADCC induction. Exemplary isotypes are IgGI, IgG2, IgG3, and IgG4. Either of the human light chain constant regions, kappa or lambda, may be used. If desired, the class of an ICAM-1 antibody of the present invention may be switched by known methods.

The antibody of the invention binds specifically to the ICAM-1 protein. By the term "specifically binds" or "binding specifically", as used herein, is meant a molecule, such as an antibody, which recognizes and binds to a cell surface molecule or feature, but does not substantially recognize or bind other molecules or features in a sample. The term "ICAM-1", as used herein, refers to the "Intercellular adhesion molecule 1". Other terms to designate ICAM-1 include BB2, CD54, or P3.58, which are interchangeably, and include as well variants, isoforms, species homologs of human or murine ICAM-1, and analogs having at least one common epitope with human or murine ICAM-1. The term includes functionally equivalent variants of ICAM-1, i.e., any molecule sharing with ICAM-1 one or more of the functions described in the present invention associated with ICAM-1, and having a minimal identity in the amino acid sequence. The term also includes all the physiologically relevant post-translational chemical modification forms, for example, glycosylation, phosphorylation or acetylation, etc., provided that the functionality of the protein is maintained. Said term encompasses the ICAM-1 of any mammal species, including but not being limited to, domestic and farm animals (cows, horses, pigs, sheep, goats, dogs, cats or rodents), primates and humans. Preferably, the ICAM-1 is human. The mRNA sequence for human ICAM-1 has the Genbank accession number NM_000201 (version of 24 June 2024). "Human ICAM-1" is undersood as the protein defined by the sequence of the UniProt database with accession number P05362 (version 2, release of 24 July 2024).

The variants of ICAM-1 can be both natural and artificial. The expression "natural variant" refers to all those variants of human ICAM-1 mentioned above which occur naturally in other species, i.e., ICAM-1 orthologs. Said natural variants include but are not limited to, ICAM-1 of dogs, corresponding to the predicted sequence with GenBank accession number L31625.1 (version of 06 April 1994) or to the predicted sequence with GenBank accession number NM_001003291.1 (version of 11 October 2020), or the protein defined under UniProt accession number P33729 (version 2, release of 24 July 2024); ICAM-1 of rats, corresponding to the predicted sequence with GenBank accession number NM_012967.1 (version of 02 June 2024), or the protein defined under UniProt accession number Q00238 (version 1, release of 24 July 2024); ICAM-1 of mouse, corresponding to the predicted sequence with GenBank accession number NM_010493.3 (version of 09 July 2024), or the protein defined under UniProt accession number P13597 (version 1, release of 24 July 2024); ICAM-1 of monkeys, corresponding to the predicted sequence with GenBank accession number NM_001047135.1 (version of 02 April 2024), or the protein defined under UniProt accession number Q5NKV6 (version 1, release of 24 July 2024); ICAM-1 of pigs, corresponding to the predicted sequence with GenBank accession number NM_213816.1 (version of 01 July 2020), or the protein defined under UniProt accession number Q9MZU5 (version 1, release of 24 July 2024). The natural variants of ICAM-1 can also be derived from said sequences by means of insertion, substitution (such as conservative substitution) or deletion of one or more amino acids and include natural alleles, variants resulting from alternative processing and secreted and truncated forms occurring naturally. The ICAM-1 can also be an artificial variant produced by recombinant and/or synthetic means.

Additionally, the functionally equivalent variants of ICAM-1 include polypeptides showing at least 60%, 65%, 70%, 72%, 74%, 76%, 78%, 80%, 90%, 95%, 97%, 99% similarity or identity with the diferent natural variants of ICAM-1 mentioned above. The degree of identity between two polypeptides is determined using algorithms implemented in a computer and methods which are widely known by the persons skilled in the art. The identity between two amino acid sequences is preferably determined using the BLASTP algorithm (BLAST Manual, Altschul, S. et al., NCBI NLM NIH Bethesda, Md. 20894, Altschul, S., et al., J., 1990, Mol. Biol. 215:403-410).

The ICAM-1 is a glycoprotein of the immunoglobulin (Ig) superfamily encoded by the *icam1* gene, which is located on chromosome 19 (19p13.2 site). ICAM-1 is mostly expressed on the plasma membrane of vascular and immune cells, including but not limited to endothelial cells and leukocytes, and also many other cell types, such as epithelial cells, neurons, astrocytes, fibroblasts, myocytes, hepatocytes, and others. Soluble ICAM-1 has been identified, including but not limited to the circulation, extracellular space, and other body fluids.

ICAM-1 has a large extracellular segment, which is glycosylated at various sites and comprises five Ig-like domains, named D1 (the most membrane-distal one) through D5 (the most membrane-proximal one). This is followed by a single transmembrane region that anchors the protein to the membrane and a short cytoplasmic tail that interacts with other membrane, cytoplasmic, or cytoskeletal proteins and relays signals intracellularly, most commonly upon ligand binding on the extracellular region. Such ICAM-1 ligands include ICAM-1 cognate receptors expressed on leukocytes (LFA-1 and Mac-1), a peptide contained in fibrinogen and fibrin, a peptide found in mucin 1 core protein, hyaluronan, and others. In addition, various pathogens and pathogen-infected cells can bind to ICAM-1, including but not limited to major class human Rhinoviruses, A-type coxsackieviruses, *Plasmodium falciparum*-infected erythrocytes displaying PfEMP1, among others. Several types of membrane-enveloped viruses, extracellular vesicles, and cancer or metastatic cells can either bind to ICAM-1 through the presence of ICAM-1 ligands on their surface or, *vice versa,* contain ICAM-1 molecules of their membranes with which they can bind to ICAM-1 ligands. This facilitates their penetration into/through cells and tissues in the body. Apart from these ligand-binding and adhesion properties of ICAM-1 extracellular domains, these domains are also involved in ICAM-1 dimerization and oligomerization. This, in turn, can alter ICAM-1 affinity to its ligands and partner proteins, consequently affecting their derived signals and/or functions. These interactions and such derived signals and functions can also be affected and/or modulated depending on the glycosylation pattern found on ICAM-1 extracellular region. Interestingly, although most studies on ICAM-1 expression, structure, and function have been conducted on the full-length form of this protein or its soluble derivatives, several studies have identified shorter forms of this protein, which lack different extracellular domains and mostly arise from alternative splicing (herein referred to as isoforms for simplicity). These isoforms were identified first in mice recombinantly modified to express human ICAM-1 and then were associated with the endogenous expression of mouse ICAM-1 in mice. Their expression, binding properties, functions, and implication in disease have been tested and/or speculated in several reports. Therefore, any new tools that can bind to ICAM-1 structural regions can be used to detect and/or modify: them, their binding and/or oligomerization sites, their interaction with extracellular binders, membrane and/or intracellular partners, as well as any signal, function or associated changes derived from such structure and/or interactions.

The antibody of the invention may be directed to ICAM-1 antigens from various mammalian species. Non-limitative examples of mammals suitable for this invention include mouse, rat, rabbit, goat, donkey, or non-human primate such as monkey (e.g., cynomologous or rhesus monkey) or ape (e.g., chimpanzee) and human. In a particular embodiment, the ICAM-1 is of human origin.

As used herein, the antibody of the invention encompasses not only full length antibodies (e.g., IgG), but also antigen-binding fragments thereof, for example, Fv, Fab, Fab', F(ab')₂, fragments, human antibodies, humanised antibodies, chimeric antibodies, antibodies of a non-human origin, recombinant antibodies, and polypeptides derived from immunoglobulins produced by means of genetic engineering techniques, for example, single chain Fv (scFv), diabodies, heavy chain or fragments thereof, light chain or fragment thereof, V_{H} or dimers thereof, V_{L} or dimers thereof, Fv fragments stabilized by means of disulfide bridges (dsFv), molecules with single chain variable region domains (Abs), minibodies, nanobodies, scFv-Fc, (scFv)₂, and fusion proteins comprising an antibody, or any other modified configuration of the immunoglobulin molecule that comprises an antigen recognition site of a desired specificity. The antibody of the invention may also be a bispecific antibody. An antibody fragment may refer to an antigen binding fragment. An antibody includes an antibody of any class, namely IgA, IgD, IgE, IgG (or sub-classes thereof), and IgM, and the antibody need not be of any particular class. In addition, the antibody of the invention may be also conjugated to a further compound, such as a marker, a tracer, a therapeutic agent, a toxin and the like.

Thus, in an embodiment, the antibody of the invention is selected from the group consisting of Fv, Fab, F(ab')₂, Fab', scFv, (scFv)₂, scFv-Fc, minibody, nanobody, and diabody.

Numerous approaches make use of the molecular biology and genetic techniques such as the good knowledge of the genetics and structure of the immunoglobulins to construct different modifications of immunoglobulin molecule with the aim of improve its properties for clinical or other uses. Some of them tend to reduce the immunogenicity of the molecule in the species in which should be used and the resultant molecule has a sequence more homologous with this species. Various methods have been used to obtain monoclonal antibodies (mAbs) of human origin avoiding the non-ethically admissible proceedings in healthy humans. In other approaches the molecular weight and size are reduced e.g. in order to improve the distribution of the molecule into solid tumours. Other possibilities are conjugation in a molecule of binding domains for more than one target molecule (bispecific antibody or also triespecific, etc.) or the conjugation of an antibody or a fragment with another molecule with the desired function e.g. a toxic agent, a hormone, growth factor, an immunomodulating agent (immunosuppressor or immunostimulator), an inhibitor of cell growth, etc. In general, all the resultant molecules retain at least one variable domain of an antibody, which gives the high specificity and affinity characteristic of the antigen-antibody binding.

Such antibodies may be produced in a variety of ways, including hybridoma cultures, recombinant expression in prokaryotic or eukaryotic cells, such as but not limited to bacteria or mammalian cell cultures, and recombinant expression in transgenic organisms, including fungi, algae, plants, or animals. There is abundant guidance in the literature for selecting a particular production methodology, e.g., Chadd and Chamow, Curr. Opin. Biotechnol., 12:188-194 (2001). The choice of manufacturing methodology depends on several factors including the antibody structure desired, the importance of carbohydrate moieties on the antibodies, ease of culturing and purification, and cost. Many different antibody structures may be generated using standard expression technology, including full-length antibodies, antibody fragments, such as Fab and Fv fragments, as well as chimeric antibodies comprising components from different species. Antibody fragments of small size, such as Fab and Fv fragments, having no effector functions and limited pharmokinetic activity may be generated in a bacterial expression system. Single chain Fv fragments show low immunogenicity and are cleared rapidly from the blood.

In a particular embodiment, the antibody of the invention is a monoclonal antibody or a fragment of said antibody which retains the capacity to bind to ICAM-1. Said antibodies are preferably human or humanized antibodies.

Thus, in a particular embodiment, the antibody of the invention is a human antibody. In another particular embodiment, the antibody of the invention is a humanized antibody. In another particular embodiment, the antibody of the invention is a chimeric antibody.

### Humanized antibodies

By "humanized antibody" is meant an antibody derived from a non-human antibody, typically a murine antibody, that retains the antigen-binding properties of the parent antibody, but which is less immunogenic in humans. This may be achieved by various methods, including (a) grafting the entire non-human variable domains onto human constant regions to generate chimeric antibodies; (b) grafting only the non-human complementarity determining regions (CDRs) into human framework and constant regions with or without retention of critical framework residues; and (c) transplanting the entire non-human variable domains, but "cloaking" them with a human-like section by replacement of surface residues.

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain.

Humanization can be essentially performed following the method of Winter and coworkers (Jones et al., Nature, 321:522-525 (1986); Reichmann et al., Nature, 332:323-327 (1988); Verhoeyen et al., Science, 239:1534-1536 (1988)), by substituting hypervariable region sequences for the corresponding sequences of a human antibody. In practice, humanized antibodies are typically human antibodies in which some hypervariable region residues and possibly some framework region (FR) residues are substituted by residues from analogous sites in rodent antibodies. The choice of human variable domains, both light and heavy, to be used in making the humanized antibodies is very important to reduce immunogenicity retaining the specificity and affinity for the antigen. According to the so called "best-fit" method, the sequence of the variable domain of a rodent antibody is screened against the entire library of known human variable-domain sequences. The human sequence which is closest to that of the rodent is then accepted as the human framework region (FR) for the humanized antibody (Suns et al., J. Immunol., 151:2296 (1993); Chothia et al., J. Mol. Biol, 196:901 (1987)). Another method uses a particular framework region derived from the consensus sequence of all human antibodies of a particular subgroup of light or heavy chains. The same framework may be used for several different humanised antibodies (Carter et al., Proc. Natl. Acad. Sci. USA, 89:4285 (1992); Presta et al., J. Immunol., 151:2623 (1993)).

It is further important that antibodies are humanized, with retention of high affinity for the antigen and other favourable biological properties. To achieve this goal, according to a preferred method, humanized antibodies are prepared by a process of analysis of the parental sequences and various conceptual humanised products using three-dimensional models of the parental and humanized sequences.

A further step in this approach, to make an antibody more similar to humans, is to prepare the so called primatised antibodies, i.e.. a recombinant antibody which has been engineered to contain the variable heavy and light domains of a monkey (or other primate) antibody, in particular, a cynomolgus monkey antibody, and which contains human constant domain sequences, preferably the human immunoglobulin gamma 1 or gamma 4 constant domain (or PE variant). The preparation of such antibodies is described in Newman et al., Biotechnology, 10: 1458-1460 (1992); US 5,658,570 and US 6,113,898. These antibodies have been reported to exhibit a high degree of homology to human antibodies, i.e., 85-98%, display human effector functions, have reduced immunogenicity, and may exhibit high affinity to human antigens. Another highly efficient means for generating recombinant antibodies is disclosed by Newman, Biotechnology, 10: 1455-1460 (1992).

### Human antibodies

By "human antibody" is meant an antibody containing entirely human light and heavy chains as well as constant regions, produced by any of the known standard methods.

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region PH gene in chimeric and germ-line mutant mice results in the complete inhibition of endogenous antibody production. Transfer of the human germ-line immunoglobulin gene array in such germ line mutant mice will result in the production of human antibodies after immunization. See, e.g., Jakobovits et al., Proc. Mad. Acad. Sci. USA, 90:255 1 (1993); Jakobovits et al., Nature, 362:255-258 (1993), Lonberg, 2005, Nature Biotech. 23:1117-25.

Human antibodies may also be generated by in vitro activated B cells or SCID mice with its immune system reconstituted with human cells.

Once a human antibody is obtained, its coding DNA sequences can be isolated, cloned and introduced into an appropriate expression system, i.e., a cell line, preferably from a mammal, which subsequently express and liberate it into a culture media from which the antibody can be isolated.

### Antibody fragments

An antibody fragment is a fragment of an antibody such as, for example, Fab, F(ab')₂, Fab' and scFv. Various techniques have been developed for the production of antibody fragments. Traditionally, these fragments were derived via proteolytic digestion of intact antibodies but more recently these fragments can be produced directly by recombinant host cells. In other embodiments, the antibody of choice is a single chain Fv (scFv) fragment which additionally may be monospecific or bispecific.

Papain digestion of antibodies produces two identical antigen-binding fragments, called "Fab" fragments, each with a single antigen-binding site, and a residual "Fc" fragment, which name reflects its ability to crystallize readily. Pepsin treatment yields an F(ab')₂ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind the antigen, although with lower affinity than the entire binding site.

The "Fab" fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments which have hinge cysteines between them. Other chemical couplings of antibody fragments are also known.

"Single-chain Fv" or "scFv" antibody fragments comprise the V_{H} and V_{L} domains of an antibody, wherein these domains are present in a single polypeptide chain. Preferably, the Fv polypeptide further comprises a polypeptide linker between the VH and VL domains which enables the scFv to form the desired structure for antigen binding. For a review of scFv see Pluckthun in The Pharmacology of Monoclonal Antibodies, vol. 113, Rosenburg and Moore eds., Springer-Verlag, N.Y., pp. 269-315 (1994).

The term "diabodies" refers to small antibody fragments with two antigen-binding sites, those fragments comprising a heavy-chain variable domain (V_{H}) connected to a light chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

The term "minibodies" includes antibodies that include an antibody fragment as a partial structural unit. The minibodies of the present disclosure are not particularly limited by their structure nor their method of production, so long as they have antigen binding activity. Some minibodies have an activity greater than that of a whole antibody (Orita et al., Blood (2005) 105:562-566).

The term "nanobodies" designates small sized entities (15 kDa) formed solely by the antigen binding region of the heavy chain (VH fragment) of immunoglobulins. Said nanobodies are mainly produced after immunizing animals of the Camelidae family, such as camels, llamas and dromedaries, mainly llamas; and also of the shark family, which have the particularity of having antibodies which naturally lack the light chain and recognize the antigen by the heavy chain variable domain. Nevertheless, the nanobodies derived from these sources require a humanization process for their therapeutic application. Another potential source for obtaining nanobodies is from antibodies derived from different human samples by separating the VH and VL domains of the variable region. Nanobodies present advantages such as a production cost reduction with respect to whole antibodies, stability and the reduction of immunogenicity.

Functional fragments of antibodies which bind to ICAM-1 included within the present invention retain at least one binding function and/or modulation function of the full-length antibody from which they are derived. Preferred functional fragments retain an antigen-binding function of a corresponding full-length antibody (e.g., the ability to bind a mammalian ICAM-1).

### Bispecific antibodies

Bispecific antibodies are antibodies that have binding specificities for at least two different epitopes. Exemplary bispecific antibodies may bind to two different epitopes of the ICAM-1 protein. Other such antibodies may bind the ICAM-1 protein (a first antigen) and further bind a second protein or marker (second antigen). Bispecific antibodies may also be used to localize cytotoxic agents to the ICAM-1-expressing cells. These antibodies possess an anti-ICAM-1-binding arm and an arm which binds the cytotoxic agent (e.g. saporin, anti-interferon-α, vinca alkaloid, ricin A chain, methotrexate or a radioactive isotope). Bispecific antibodies can be prepared as full length antibodies or antibody fragments (e.g. F(ab)₂ bispecific antibodies, minibodies, diabodies).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

Techniques for generating bispecific antibodies from antibody fragments have also been described in the literature. For example, bispecific antibodies can be prepared using chemical linkage.

The fragments with the capacity to bind to ICAM-1 can also be obtained by conventional methods known by persons having ordinary skill in the art. Said methods can involve isolating DNA that encodes the polypeptide chains (or a fragment thereof) of a monoclonal antibody of interest and manipulating DNA by means of recombinant DNA technology. DNA can be used to generate another DNA of interest, or an altered DNA, (for example by means of mutagenesis) for adding, removing or substituting one or more amino acids, for example, the DNA that encodes the polypeptide chains of an antibody (e.g., the heavy or light chains, the variable region or the whole antibody) can be isolated from murine B cells from immunized mice with ICAM-1. The DNA can be isolated and amplified by conventional methods, for example by means of PCR.

The single-chain antibodies can be obtained by conventional methods by binding the variable region of the heavy and light chains (Fv region) by means of an amino acid bridge. The scFvs can be prepared by fusing the DNA encoding a linker peptide between the DNAs encoding the polypeptides of the variable regions (V_{L} and V_{H}). The production of scFvs is described in a number of documents, for example, in US patent US 4,946,778, Bird. Science 242: 423, 1988, Huston et al. Proc. Natl. Acad Sci USA 85: 5879, 1988 and Ward et al. Nature 334: 544, 1989.

The person skilled in the art will understand that the amino acid sequences of the antibodies of the invention can include one or more amino acid substitutions such that, even though the primary sequence of the polypeptide is altered, the capacity of the antibody to bind to ICAM-1 is maintained. Said substitution can be a conservative substitution and is generally applied to indicate that the substitution of one amino acid with another amino acid with similar properties (for example, the substitution of glutamic acid (negatively charged amino acid) with aspartic acid would be a conservative amino acid substitution).

The present invention also contemplates variants of the sequences of the heavy and light chains, CDRs and/or FRs identified in this description, which fall within the scope of the present invention. As it is used herein, the term "variant" or "functional variant" or "functionally equivalent variant", as used herein, refers to substantially similar sequences that substantially maintains its capacity to bind to its cognate antigen, i.e., its affinity/avidity and/or the specificity/selectivity. The variants generally have the same biological activity from the qualitative viewpoint as the native sequence. A variant of a heavy and light chain, CDR or FR of an antibody can be a polypeptide sequence derivative identified in this description comprising the addition, deletion or substitution of one or more amino acids. According to the invention, variants of a heavy or light variable chain comprising the amino acid sequence set forth in one of SEQ ID NO: 1 to 4, a CDR comprising the amino acid sequence set forth in one of SEQ ID NO: 5 to 16, or a FR comprising the amino acid sequence set forth in one of SEQ ID NO: 17 to 32 include variable chains, CDRs or FRs comprising amino acid sequences having at least approximately 70% sequence identity with the corresponding amino acid sequence shown in one of SEQ ID NOs: 1 to 32, preferably at least 75%, 80%, 85%, 90%, 91%, 92%, 93%, 94%, 95%, 96%, 97%, 98% or 99% sequence identity with the corresponding amino acid sequences shown in one of SEQ ID NOs: 1 to 32. It is also contemplated that variants comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or the C-terminus, or both the N- and C-terminus of the variable chain, CDR and/or FR. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or the C-terminus, or both the N- and C-terminus of the variable chain, CDR and/or FR. Functional variants of an antibody according to the invention will preferably have a capacity to bind to its cognate target of at least 50%, at least 60%, at least 70%, at least 80%, at least 90%, at least 91%, at least 92%, at least 93%, at least 94%, at least 95%, at least 96%, at least 97%, at least 98%, or at least 99% the capacity to bind to its cognate target of said antibody.

The capacity of the antibody of the invention to bind to ICAM-1 can be determined by a number of assays that are available in the art. Preferably, the binding specificity of monoclonal antibodies produced by a clone of hybridoma cells is determined by immunoprecipitation or by an *in vitro* binding assay, such as radioimmunoassay (RIA), enzyme-linked immunoabsorbent assay (ELISA), surface plasmon resonance or by immunofluorescent techniques such as immunohistochemistry (IHC), fluorescence microscopy or flow cytometry.

Amino acid sequence modification(s) of the antibody described herein are contemplated. For example, it may be desirable to improve the binding affinity and/or other biological properties of the antibody. Amino acid sequence variants of the antibody are prepared by introducing appropriate nucleotide changes into the antibody encoding nucleic acid, or by peptide synthesis. Such modifications include, for example, deletions from, and/or insertions into and/or substitutions of, residues within the amino acid sequences of the antibody. Any combination of deletion, insertion, and substitution is made to achieve the final construct, provided that the final construct possesses the desired characteristics. The amino acid changes may also alter post-translational processes of the protein, such as changing the number or position of glycosylation sites.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a peptide with an N-terminal methionyl residue or the antibody polypeptidic chain fused to a cytotoxic polypeptide. Other insertional variants of the molecule include the fusion to the N- or C-terminus of an enzyme, or a polypeptide which increases its serum half-life.

Another type of variant is an amino acid substitution variant. These variants have at least one amino acid residue in the molecule replaced by a different residue. The sites of greatest interest for substitution mutagenesis of antibodies include the hypervariable regions, but framework (FR) regions alterations are also contemplated.

Another type of amino acid variant of the antibody alters the original glycosylation pattern of the antibody. By altering is meant deleting one or more carbohydrate moieties found in the molecule, and/or adding one or more glycosylation sites that are not present in it. Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of any of these tripeptide sequences in a polypeptide creates a potential glycosylation site. O-linked glycosylation refers to the attachment of one of the monosaccharides or monosaccharide dereivatives N-aceylgalactosamine, galactose, or xylose to a hydroxyamino acid, most commonly serine or threonine, although 5-hydroxyproline or 5-hydroxylysine may also be used. Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites). Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

In an embodiment, the amino acid sequence variants of the antibody of the invention conserve the capacity to transport to lysosomes. In another embodiment, the amino acid sequence variants of the antibody of the invention conserve the capacity to recognize the ICAM-1 of dogs and primates, particularly of dogs, monkey and humans.

It may be desirable to modify the antibodies used in the invention to improve effector function, e.g. so as to enhance ADCC (antibody dependent cell-mediated cytotoxicity) and/or CDC (complement-dependent cytotoxicity) of the antibody. This may be achieved by introducing one or more amino acid substitutions in an Fc region of an antibody.

Glycosyl groups added to the aminoacid backbone of glycoproteins e.g. antibodies are formed by several monosaccharides or monosaccharide derivatives in resulting in a composition which can be different in the same antibody produced in cell from different mammals or tissues. In addition, it has been shown that different composition of glycosil groups can affect the potency in mediating antigen-dependent cell-mediated cyotoxicity (ADCC) and/or complement dependent cytotoxicity (CDC) of the antibody. Therefore it is possible to improve those properties by mean of studying the pattern of glycosilation of antibodies from different sources. An example of such approach is Niwa et al., Cancer Res. 2004 Mar 15;64(6):2127-33.

Thus, in another particular embodiment, the antibody of the invention is glyco-engineered to reduce fucose and thus enhance ADCC. Antibodies lacking fucosyl residues can be prepared according to US 8,207,303.

In another particular embodiment, the antibody of the invention has been engineered to enhance complement activation.

Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumour activity may also be prepared using heterobifunctional cross-linkers as described in Wolff et al. Cancer Research 53:2560-2565 (1993). Alternatively, an antibody which has dual Fc regions can be engineered and may thereby have enhanced complement lysis and ADCC capabilities. See Stevenson et al. Anti-Cancer Drug Design 3:2 19-230 (1989), and Davis et al., Protein Eng Des Sel 23:195-202 (2010).

In order to increase the serum half-life of the antibody, one may incorporate a salvage receptor binding epitope into the antibody (especially an antibody fragment) as described in US 5,739,277, for example. As used herein, the term "salvage receptor binding epitope" refers to an epitope of the Fc region of an IgG molecule (e.g., IgGI, IgG2, IgG3, or lgG4) that is responsible for increasing the *in vivo* serum half-life of the IgG molecule.

Preferably, the antibody of the invention, e.g., monoclonal antibodies, scFv fragments, Fab fragments, or other binding compositions derived from the monoclonal antibody of the invention, have a high affinity to ICAM-1 receptor. The affinity of monoclonal antibodies and related molecules to the ICAM-1 receptor may be measured by conventional techniques.

The affinity of an antibody for an antigen can be defined as the effectiveness of the antibody for binding such antigen. Antigen-antibody binding is a reversible binding and so, when both molecules are in dilution in the same solution after sufficient time, this solution reaches an equilibrium in which the concentrations of antigen-antibody complex (AgAb), free antigen (Ag) and free antibody (Ab) are constant. Therefore, the ratio [AgAb]/[Ag]*[Ab] is also a constant defined as association constant named Ka which can be used to compare the affinity of some antibodies for its respective epitope.

The common way to measure affinity is to experimentally determine a binding curve. This involves measuring the amount of antibody-antigen complex as a function of the concentration of the free antigen. There are two common methods of performing this measurement: (i) the classical equilibrium dialysis using Scatchard analysis and (ii) the surface plasmon resonance method in which either antibody or antigen are bound to a conductive surface and binding of antigen or antibody respectively affects the electrical properties of this surface.

Often it is only necessary to determine relative affinities of two or more antibodies that join the same epitope, such as in the case of the antibody of the invention and a functional variant thereof. In this case a competitive assay can be performed in which a serial dilution of one of the antibodies is incubated with a constant quantity of a ligand, and the second antibody labelled with any suitable tracer is then added. After binding of this mAb and washing the non-bounded antibodies, the concentration of the second antibody is measured and plotted in relation to concentrations of the first antibody and analysed with Scatchard method. An example is Tamura et al., J. Immunol. 163: 1432-1441 (2000). Alternatively, when the ligand is a membrane bound antigen as is the case of the present invention, the soluble ligand may be displayed to the antibodies on the surface of cells expressing said antigen. Therefore, varying quantities of antibodies are incubated with a constant quantity of a ligand.

The affinity of the antibody of the invention for ICAM-1 is at least 10⁻⁷ M, at least 10⁻⁸ M, at least 10⁻⁹ M, at least 10⁻¹⁰ M, at least 10⁻¹¹ M or at least 10⁻¹² M.

### Carrier of the invention

In a second aspect, the invention relates to a carrier, hereinafter referred to as the "carrier of the invention", further comprising the antibody of the invention according to the first aspect.

The "antibody of the invention" has been defined in the context of the previous aspects of the invention and apply equally to this aspect of the invention.

The term "carrier", as described herein, refers to a molecular or supramolecular structure, either natural, derived from a natural one, artificial, or comprising any or all these types of components, to which the antibody of the invention can be incorporated or associated. In an embodiment, the antibody of the invention is coupled or attached to, onto, or in the carrier. Coupling or attachment can be achieved genetically or synthetically, by coating, complexation, conjugation, absorption, adsorption, encapsulation, or synthesis or fabrication as a part of the same structure or through any type of passive or active interaction or association that join or bring together the antibody and the carrier. In a particular embodiment, the antibody of the invention is displayed on the surface of the carrier of the invention. Methods to display the antibody of the invention on the surface of the carrier have been described in the art. Non-limiting methods include covalent or non-covalent attachment, conjugation, or recombinant linkage, or the antibody fragment could be a structural part of the carrier.

In another particular embodiment, the carrier of the invention comprising the antibody of the invention further comprises at least an agent. The term "agent", as used herein, refers to a molecular or supramolecular entity that induces a desired effect. The term "agent" is not to be construed narrowly but extends to chemical elements, small molecules, substances, compounds, macromolecules, supramolecular structures, lipids, hormones, vitamins, proteinaceous molecules such as peptides, polypeptides and proteins, including but not limited to other antibodies, as well as compositions comprising them and genetic molecules such as RNA, DNA and mimetics and chemical analogs thereof, as well as cellular agents. In a more particular embodiment, the carrier of the invention comprises an agent selected from the group consisting of therapeutic agent, diagnostic agent, tracer agent, reporter, marker, cell, cell fragment, cell part, virus, viral fragment, viral part, conjugate and fusion protein. In a preferred embodiment of the carrier of the invention, the agent is a therapeutic agent.

The term "therapeutic agent", as used herein, refers to an agent useful in the treatment and/or prevention of a disease. The term "diagnostic agent", as used herein, is understood to be an agent whose presence or absenc may be detected after being administered. The term "tracer agent", as used herein, refers to an agent used to track or follow in imaging, visualization, or any type of detection technique. The term "reporter", as used herein, refers to any identifiable tag, label, or moiety that allows tracing or detection. The term "marker", as used herein, refers to an agent for which the presence, the absence, the modification of expression or distribution can be measured. The term "cell" as used herein, refers to a prokaryotic or eukaryotic cell which can be used as an agent. The terms "cell fragment" and "cell part", as used herein, refer to a fragment or part of a prokaryotic or eukaryotic cell, respectively, which can be used as an agent. The term "virus", as used herein, refers to an infectious, attenuated or inactivated virus to be used as an agent. The terms "viral fragment" and "viral part", as used herein, refer to a fragment or a part of a virus, respectively, which can be used as an agent. The term "conjugate", as used herein, refers to any substance formed from the joining together of two parts, such as, but not limited to, a chemical element and a molecule, a small molecule and a large molecule, two large molecules, a chemical element or molecule and a macromolecular structure, like a chemical element and a peptide or protein or lipid or nucleic acid or polymer, or two peptides, or a peptide and a protein, or two proteins, or a peptide or protein and a lipid, or a peptide or protein and a nucleic acid, or a peptide or protein and a polymer. The term "fusion protein", as used herein, refers to an proteinaceous construct that does not exist in nature as such and means a protein comprising at least two different amino acid sequences which are defined by their respective origin and/or by respective special structures and/or functions.

Suitable carriers of the invention are capable of, but not limited to, carrying and/or delivering the antibody of the invention to its target. Numerous suitable carriers are known, and include, but are not limited to, delivery system, conjugate, fusion protein, cell, cell-derived membrane, cell-derived vesicle, virus, viral-like particle or bacteria. The term "delivery system", as disclosed herein, refers to a system capable of carrying and/or delivering the antibody of the invention, to its target. The terms "conjugate" and "fusion protein", have been described above and apply equally as suitable carriers of the antibody of the invention. The term "cell", as used herein, refers to a prokaryotic or eukaryotic cell which can de used as a carrier of the antibody of the invention. The term "cell-derived membranes", as used herein, refers to a membrane comprising a cell membrane component of a native cell membrane with modification or with an additional component. The term "cell-derived vesicle", or "CDV" as used herein, refers to a vesicle generated from cells, and is generally a type of, but not limited to, cell organelle. The vesicle is originated from the cell membrane, either the plasma membrane or internal membranes, in almost all types of cells, and having a form of a phospholipid bilayer membrane, which is the structure of external or internal cell membranes. The term "virus", as used herein, refers to an infectious, attenuated or inactivated virus to be used as a carrier of the antibody of the invention. The term "viral-like particle" or "VLP", as used herein, relates to particles resembling viruses that do not contain any viral genetic material. VLPs are the result of the expression of viral structural proteins, such as capsid proteins, and their self-assembly. The term "bacteria", as used herein, refers to single-celled prokaryotes that can be used as a carrier of the antibody of the invention.

Thus, in a particular embodiment, the carrier of the invention is selected from a group consisting of delivery system, conjugate, fusion protein, cell, cell-derived membrane, cell-derived vesicle, virus, viral-like particle and bacteria. In a more particular embodiment, the carrier of the invention is a delivery system.

Numerous suitable delivery systems are known and include, but are not limited to, polymer, polymeric nanoparticle, lipid nanoparticle, protein nanoparticle, nucleic acid nanoparticle, inorganic nanoparticle, polymeric microparticle, lipid microparticle, protein microparticle, nucleic acid microparticle, inorganic microparticle, liposome, polymersome, micelle and dendrimer.

The term "polymer" refers to both linear and branched polymers derived from one or more monomer units, which may or may not be crosslinked or grafted. Non-limiting examples of polymers include copolymers, terpolymers, tetramers, and the like, wherein the polymer is random, block, or alternating polymer. Polymers such as polyethylenglycol (PEG), hydroxypropyl methacrylate (HPMA), chitosan, dextran, polynucleotides, poly(2-oxazoline) (POx), polyglutamate (PGA), copolymers of ethylene glycol/propylene glycol, polyvinyl alcohol, carboxymethylcellulose, polyvinyl pyrrolidone, poly-1,3-dioxolane, poly-1,3,6-trioxane, ethylene/maleic anhydride copolymer, polyaminoacids, polyacetals, polyketals, etc., may be used as a suitable carrier comprising the antibody of the invention.

In the context of the present invention, "nanoparticle" must be understood as a particle, preferably, but not limited to, a spherical particle, with a hydrodynamic size ranging from 1 to 1000 nm, preferably from 1 nm to 300 nm, even more preferably between 2 nm and 200 nm, still more preferably between 2 nm and 150 nm.

In the context of the present invention, "microparticle" must be understood as a particle, preferably, but not limited to, a spherical particle, with a hydrodynamic size ranging from 1 to 1000 µm, preferably from 1 µm to 200 µm, even more preferably between 3 µm and 50 µm, still more preferably between 5 µm and 30 µm, and most preferably between 20 µm and 60 µm.

As used herein, the term "polymeric nanoparticle" and "polymeric microparticle" refer to a nanoparticle or microparticle, respectively, as defined herein, that comprises or consists of one or more polymers. In one embodiment, the polymeric nanoparticle or polymeric microparticle forms a colloidal suspension or dispersion in aqueous solution. Non-limiting examples of polymeric nanoparticles or polymetic microparticles include polystyrene, polylactic acid, poly(lactic-co-glycolic acid), polycaprolactone, chitosan, alginate, poly(methyl methacrylate), poly(P-amino ester), ethyl cellulose, and semiconducting polymer nanoparticles or polymeric microparticles.

Herein, the term "lipid nanoparticle" and "lipid microparticle" are defined as a molecule spherical or spherical-like in shape, and comprising a solid lipid core stabilized by a surfactant. The core lipids can be steroids, fatty acids, acylglycerols, waxes, and combinations of them. Surfactants may be biological membrane lipids such as phospholipids, sphingomyelins and bile salts (e.g., sodium taurocholate). All of these may be utilized as stabilizers in the lipid nanoparticle or lipid microparticles, respectively.

The term "protein nanoparticle" and "protein microparticle", as used herein, are spherical, spherical-like, elongated, or filamentous protein particles formed by the self-assembly a protein or proteins.

As used herein, the term "nucleic acid nanoparticle" and "nucleic acid microparticle" refer to a single- or multi-chain polynucleotide molecule or supramolecular composite comprising a compacted three-dimensional structure.

As used herein, the term "inorganic nanoparticle" and "inorganic microparticle" refer to a nanoparticle or microparticle, respectively, comprising at least one inorganic element. Non-limiting examples of inorganic nanoparticles or inorganic microparticles include metal nanoparticles or microparticles (such as, but not limited to, iron oxide, gold or silver nanoparticles or microparticles), mesoporous silica nanoparticles or microparticles, quantum dots, or carbon nanotubes or microtubes.

As used herein, the term "liposome" is used to define a variety of single and multilamellar lipid vehicles formed by the generation of enclosed lipid bilayers or aggregates. Liposomes may be characterized as having vesicular structures with a bilayer membrane, generally comprising but not limited to one or more phospholipids, with or without cholesterol, and an inner medium that generally comprises an aqueous composition. Liposomes preferably have diameters of less than 10 µm. Liposomes having diameters between 200 nanometers and 1 µm are preferred. Liposomes having diameters between 50 and 150 nanometers are yet preferred. Suitable types of liposomes may comprise one or more of the following lipids: cholesterol, phosphatidylcholine, hydrogenated soy phosphatidylcholine, dipalmitoyl phosphatidylcholine, phosphatidylglycerol, phosphatidic acid, phosphatidylethanolamine, 2-distearyl-sn-glycero-3-phosphoethanolamine, phosphatidylserine, N-[1-(2,3-dioleyloxy)propyl]-N,N,N-triethylammonium (DOTMA), and 1,2-dioleoyl-3-trimethylammoniopropane (DOTAP).

The term "polymersome", as used herein, refers to a particle which has a bilayer membrane structure with "hydrophilic domain-hydrophobic domain-hydrophobic domain-hydrophilic domain," and includes, but is not limited to, a polymer or copolymer in the hydrophobic and hydrophilic regions. The polymer or copolymer may include an artificially synthesized polymer or copolymer and a lipid, or only an artificially synthesized polymer or copolymer. The artificially synthesized polymer or copolymer refers to any polymer or copolymer, not made of natural lipid. The polymersome comprises a single membrane having a bilayer structure, or a multi membrane having two or more single layer structure.

The term "micelle", as used herein, refers to a particle having a hydrophobic core and a hydrophilic shell. In an aqueous medium, the lipophilic domains of the molecule aggregate are oriented towards the inside of the micelle and the hydrophilic domains are in contact with the medium. In one embodiment, the micelle may be a micelle of a surfactant or a polymer self-assembled micelle. The micelle made by self assembly may have various shapes according to the type of polymer forming a copolymer, but the present invention is not limited thereto. The micelle also includes a multi membrane including two or more mono layers. The shape of the micelle may be, but not limited to, for example, a sphere, ellipsoid or cylinder. A probe with the micelle structure may further include components such as a marker and an inorganic particle in a hydrophobic core region.

The term "dendrimer" or "dendrimeric structure", as used herein, refers to a macromolecule having a core and having multiple shells of branching structures emanating from the core. The shape and size of a dendritic carrier can vary. In some instances, the dendritic carrier can be approximately spherical or globular in shape. Furthermore, the dendritic carrier can have a diameter in the range of about 15 angstroms (A) to about 250 A, with a corresponding range of molecular weights, e.g., from about 500 Daltons to about 2 million Daltons, but they can also exceed this size range amply, such as but not limited to dendrimers made of nucleic acids, such as DNA, which can achieve sizes in the range of 20 nm to 500 nm. Dendrimers can be obtained commercially from various sources (e.g., Dendritech, Midland, Michigan) or synthesized by methods known to those skilled in the art. Dendritic molecules can roughly be divided into the low-molecular weight and the high-molecular weight species. The first category includes dendrimers and dendrons whereas the second encompasses dendronized polymers, hyperbranched polymers, and brush-polymers (also called bottle-brushes). Dendrimers and dendrons are repeatedly branched, monodisperse, and usually highly symmetric compounds. There is no apparent difference in defining dendrimer and dendron. A dendron usually contains a single chemically addressable group that is called the focal point. Because of the lack of the molar mass distribution high-molar-mass dendrimers and dendrons are macromolecules but not polymers. The properties of dendrimers are dominated by the functional groups on the molecular surface. Dendritic encapsulation of functional molecules allows for the isolation of the active site, a structure that mimics the structure of active sites in biomaterials because dendritic scaffolds separate internal and external functions. For example, a dendrimer can be water-soluble when its end-group is a hydrophilic group, like a carboxyl group.

Dendrimers may be generally characterised by the following features: (i) an initiator core (I) which may have one or more reactive sites and be point-like or of significant size so as to effect the final topology of the dendrimer; (ii) one or more layers of branched repeating units attached to the initiator core; (iii) functional terminal groups, such as anionic or cationic groups, which can be a part of the dendrimer or can be attached to it, optionally through linking groups, to the surface of the dendrimer.

Dendrimers contemplated herein may comprise lysine, or lysine analogue building units. The term "lysine analogue" refers to a molecule which has a single apex carboxyl group for attachment to the previous layer of building units, and two or three primary amine groups to which can be attached further building units, blocking groups, linkers or aryl acid groups. Examples of "lysine analogues" contemplated herein are described in PCT/AU2007/000352, for example glycyl-lys. In some particular examples, the dendrimer comprises only lysine or one type of lysine analogue as the building unit.

Other dendrimers contemplated herein include those comprising polyamidoamine (PAMAM), poly(etherhydroxylamine) (PEHAM) or polypropyleneimine building units. In particular examples thereof, the dendrimer has only polyamidoamine (PAMAM), poly(etherhydroxylamine) (PEHAM) or polypropyleneimine as the building unit.

The core moiety may contain only 1 point of attachment for a building unit or may contain 2, 3 or more points, which may or may not be further utilized for the attachment of building units. Typically, the point of attachment is a free amino group. Core moieties may consist of, comprise or be derived from a building unit or may be a molecule different to the building units. Exemplary core moieties are illustrated herein and described in PCT/AU2007/000352.

Other dendrimers contemplated herein include dendrimers made of a nucleic acid, such as, but not limited to DNA, comprising natural or modified or artificial nucleic acids. In a particular embodiment, the dendrimers are DNA dendrimers. DNA dendrimers are complex, highly branched molecules built from interconnected natural or synthetic DNA subunits. A DNA dendrimer is constructed from partially double stranded DNA monomers, each of which is made from two single stranded DNA molecules that share a region of sequence complementarity located in the central portion of each strand. In order to prevent DNA dendrimers from "falling apart" over time, chemical "spot welds" are sometimes added to the growing assembly during the process using UV light via the intercalation and activation of psoralen cross-linkers. Monomers are combined during the manufacturing process to prepare DNA dendrimers of different sizes and shapes. Dendrimers have been historically purified according to their size and molecular weight on denaturing sucrose gradients after ultracentrifugation. DNA dendrimers are described in detail in WO2010017544. An example of a DNA dendrimer is the 3DNA^{®} dendrimer comprised of DNA (WO2011106481).

Thus, in a particular embodiment of the carrier of the invention, the delivery system is selected from the group consisting of polymer, polymeric nanoparticle, lipid nanoparticle, protein nanoparticle, nucleic acid nanoparticle, inorganic nanoparticle, polymeric microparticle, lipid microparticle, protein microparticle, nucleic acid microparticle, inorganic microparticle, liposome, polymersome, micelle and dendrimer. In a more particular embodiment, the delivery system is a polymeric nanoparticle. In a particular embodiment of the carrier of the invention, the delivery system is a polystyrene nanoparticle. In a more particular embodiment, the polymeric nanoparticle is a polystyrene nanoparticle. In a preferred embodiment of the carrier of the invention, the delivery system is a polystyrene nanoparticle and the agent is a therapeutic agent.

In an embodiment, the antibody of the invention is coupled or attached to, onto, or in the delivery system. Coupling or attachment can be achieved genetically or synthetically, by coating, complexation, conjugation, absorption, adsorption, encapsulation, or synthesis or fabrication as a part of the same structure or through any type of passive or active interaction or association that join or bring together the antibody and the delivery system. In a particular embodiment, the antibody of the invention is displayed on the surface of the delivery system of the invention.

In another embodiment, other materials and devices can be loaded or coated with the antibody of the invention. Non-limiting examples of materials and devices include cardiovascular stents or materials used for implants or tissue regeneration. In a particular embodiment, said materials and devices can further comprise at least an agent, such as those defined in the context of the carrier of the invention.

All the terms and embodiments described in the first aspect of the invention are equally applicable to the second aspect of the invention.

### Nucleic acids, gene constructs, expression cassettes, vectors, cells and living organisms of the invention

In a third aspect, the present invention relates to a nucleic acid, hereinafter referred to as the "nucleic acid of the invention" selected form the group consisting of:
(i) a nucleic acid coding for the antibody of the invention; and
(ii) a complementary nucleic acid of a nucleic acid as defined in (i).

The term "nucleic acid" or "polynucleotide", as used herein, refers to deoxyribonucleic acids (DNA) or ribonucleic acids (RNA) and their polymers in either single or double stranded form. Unless specifically limited, the term encompasses nucleic acids containing known analogs of natural nucleotides that have functional capabilities similar to those of a reference nucleic acid and which are metabolized similarly or not to naturally occurring nucleotides. Unless otherwise indicated, a specific nucleic acid sequence also implies conservatively modified variants (e.g., substitutions with degenerate codons), alleles, orthologs, SNPs and complementary sequences, as well as sequences indicated in direct form. In particular, substitutions with degenerate codons can be obtained by creating sequences in which the third position of one or more selected (or all) codons is replaced by residues with mixed bases and/or deoxyinosine residues.

The "antibody of the invention" has been defined in the context of the previous aspects of the invention and apply equally to this aspect of the invention.

In an embodiment, the nucleic acid of the invention encodes an antibody or an antigen-binding fragment thereof, selected from the group consisting of:
(a) an antibody or an antigen-binding fragment thereof, wherein the antibody comprises the CDR1, CDR2 and CDR3 within the heavy chain of the variable region comprising respectively the sequences set forth in SEQ ID NO: 5, 6 and 7, or a functionally equivalent variant thereof, and the CDR1, CDR2 and CDR3 within the light chain comprising respectively the sequences set forth in SEQ ID NO: 8, 9 and 10, or a functionally equivalent variant thereof, which corresponds to the antibody identified as B4 in the examples; and
(b) an antibody or an antigen-binding fragment thereof, wherein the antibody comprises the CDR1, CDR2 and CDR3 within the heavy chain of the variable region comprising respectively the sequences set forth in SEQ ID NO: 11, 12 and 13, or a functionally equivalent variant thereof, and the CDR1, CDR2 and CDR3 within the light chain comprising respectively the sequences set forth in SEQ ID NO: 14, 15 and 16, or a functionally equivalent variant thereof, which corresponds to the antibody identified as B6 in the examples.

In a particular embodiment, the nucleic acid of the invention encodes an antibody or an antigen-binding fragment thereof, selected from the group consisting of:
(a) an antibody or an antigen-binding fragment thereof, wherein the antibody comprises the FR1, FR2, FR3 and FR4 regions (FRs) within the heavy chain of the variable region comprising respectively the sequences set forth in SEQ ID NO: 17, 18, 19 and 20, or a functionally equivalent variant thereof, and the FR1, FR2, FR3 and FR4 regions (FRs) within the light chain of the variable region comprising respectively the sequences set forth in SEQ ID NO: 21, 22, 23 and 24, or a functionally equivalent variant thereof, which corresponds to the antibody identified as B4 in the examples; and
(b) an antibody or an antigen-binding fragment thereof, wherein the antibody comprises the FR1, FR2, FR3 and FR4 regions (FRs) within the heavy chain of the variable region comprising respectively the sequences set forth in SEQ ID NO: 25, 26, 27 and 28, or a functionally equivalent variant thereof, and the FR1, FR2, FR3 and FR4 regions within the light chain of the variable region comprising respectively the sequences set forth in SEQ ID NO: 29, 30, 31 and 32, or a functionally equivalent variant thereof, which corresponds to the antibody identified as B6 in the examples.

In another particular embodiment, the nucleic acid of the invention encodes an antibody or an antigen-binding fragment thereof, selected from the group consisting of:
(a) an antibody or an antigen-binding fragment thereof, wherein the antibody comprises at least a heavy chain of the variable region (VH) comprising the sequence set forth in SEQ ID NO: 1, or a functionally equivalent variant thereof, and at least a light chain of the variable region (VL) comprising the sequence set forth in SEQ ID NO: 2, or a functionally equivalent variant thereof, which corresponds to the antibody identified as B4 in the examples; and
(b) an antibody or an antigen-binding fragment thereof, wherein the antibody comprises at least a heavy chain of the variable region (VH) comprising the sequence set forth in SEQ ID NO: 3, or a functionally equivalent variant thereof, and at least a light chain of the variable region (VL) comprising the sequence set forth in SEQ ID NO: 4, or a functionally equivalent variant thereof, which corresponds to the antibody identified as B6 in the examples.

Said nucleic acid of the invention can contain a regulatory sequence and/or a sequence codifying for an additional functional or structural component, operatively linked for the expression of the nucleotide sequence encoding the antibody of the invention, thereby forming a gene construct, hereinafter referred to as the "gene construct of the invention".

The term "gene construct" or "nucleic acid construct" as used herein relates to a functional unit required to transfer, and preferably express, a gene or genes of interest in a host cell. This term refers to a nucleic acid molecule, either single- or doublestranded, which is modified to contain segments of nucleic acids in a manner that would not otherwise exist in nature.

Thus, in a fourth aspect, the invention relates to the gene construct of the invention comprising the nucleic acid of the invention. As used herein, the term "operatively linked" means that the antibody encoded by the nucleic acid sequence of the invention is expressed in the correct reading frame under control of the expression control or regulating sequences. In a fifth aspect, the invention relates to an expression cassette, hereinafter referred to as the "expression cassette of the invention", comprising the nucleic acid of the invention or the gene construct of the invention.

The term "expression cassette" refers to a nucleotide molecule capable of affecting expression of a structural gene (i.e., a protein coding sequence, such as an antibody chain) in a host compatible with such sequences. Expression cassettes include at least a promoter operably linked with the polypeptide-coding sequence and, optionally, with other sequences, e.g., transcription termination signals. Additional regulatory elements necessary or helpful in effecting expression may also be used, e.g., enhancers. Thus, expression cassettes include plasmids, expression vectors, recombinant viruses, any form of recombinant "naked DNA" vector, and the like.

In an embodiment, the expression cassette of the invention comprises the gene construct of the invention operatively linked to an expression control sequence. The gene construct of the invention can be obtained through the use of techniques widely known in the prior art (Sambrook et al., 2001 "Molecular cloning: to Laboratory Manual", 3a ed., Cold Spring Harbor Laboratory Press, N.Y., Vol. 1-3).

Control sequences are sequences that control and regulate transcription and, where appropriate, the translation of said antibody, and include promoter sequences, transcriptional regulators encoding sequences, ribosome binding sequences (RBS) and/or transcription terminating sequences. The expression cassette of the present invention may additionally include an enhancer, which may be adjacent to or distant from the promoter sequence and can function to increase transcription from the same. In a particular embodiment, said expression control sequence is functional in prokaryotic cells and organisms, such as bacteria, etc. Whereas in another particular embodiment, said expression control sequence is functional in eukaryotic cells and organisms, for example, insect cells, nematode cells, plant cells including those from algae, fungal cells, mammalian cells, etc.

Any available promoter can be used in this methodology. In a preferred embodiment of the present invention, the promoter used in the nucleic acid construct of the present invention is active in the specific cell population to be transfected. Illustrative, non-limiting examples of ubiquitous promoters which can be present in the expression cassette of the invention include the human cytomegalovirus promoter (hCMV), SV40 promoter, the EF1-alpha promoter to, and the ubiquitin promoter C. Illustrative, non-limiting examples of cell-type specific promoters and/or tissue specific promoters such as albumin include which is specific for liver [Pinkert et al. (1987) Genes Dev 1 :268-277], lymphoid-specific promoters [Calame et al. (1988) Adv. Immunol. 43:235-275], in particular promoters of T cell receptors [Winoto et al. (1989) EMBO J. 8:729-733] and immunoglobulins [Banerji et al. (1983) Cell 33729-740], neuron-specific promoters such as neurofilament promoter [Byrne et al. (1989) Proc. Natl. Acad. Sci USA 86:5473-5477], pancreas-specific promoters [Edlunch et al. (1985) Science 230:912-916] or mammary gland-specific promoters such as the milk whey promoter (US Patent No. 4,873,316 and EP No. 264,166). The expression cassette CAG-GS is composed of a CMV enhancer element, the promoter of the chicken β-actin and the post-transcriptional regulatory element (WPRE) virus woodchuck hepatitis (Woodchuck Hepatitis Virus, WHP) [Niwa et al. (1991) Gene 108: 193-9]. The combination of promoter and this element favors high expression levels of the transgene *in vivo.*

Advantageously, the expression cassette of the invention further comprises a marker or gene encoding a motif or phenotype which allows selecting the transformed host cell with said expression cassette. Illustrative examples of said markers that could be present in the expression cassette of the invention include antibiotic resistance genes, genes for resistance to toxic compounds, and in general, all those that allow selecting the genetically transformed cells.

The nucleic acid of the invention, the gene construct of the invention or the expression cassette of the invention can be inserted into appropriate vectors. Thus, in a sixth aspect, the invention relates to a vector, hereinafter referred to as the "vector of the invention", comprising said nucleic acid, said gene construct or said expression cassette of the invention. In an embodiment, the vector of the invention is an expression vector.

The term "vector", as used herein, refers to a construct capable of delivering, and preferably additionally expressing, one or more polynucleotides of interest into a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. This term also relates to targeting constructs, which allow for random or site-directed integration of the targeting construct into genomic DNA. Such targeting constructs, preferably, comprise DNA of sufficient length for either homologous recombination or heterologous integration. In a particular embodiment, the vector is an expression vector. The term "expression vector" refers to a replicative DNA construct used for expressing the nucleic acid construct of the invention in a cell, preferably a eukaryotic cell, more preferably a mammalian cell. The expression vector also preferably contains an origin of replication in prokaryotes, necessary for vector propagation in bacteria. Additionally, the expression vector can also contain a selection gene for bacteria, for example, a gene encoding a protein conferring resistance to an antibiotic, for example, ampicillin, kanamycin, chloramphenicol, etc. The expression vector can also contain one or more multiple cloning sites.

The choice of vector depends on the host cell in which it will be subsequently introduced. As an example, the vector into which is inserted the said nucleic acid sequences may be a plasmid or a vector which, when introduced into a host cell, either prokaryotic or eukaryotic, is integrated or not in the genome of said cell. Obtaining this vector can be performed by conventional methods known to those skilled in the art (Sambrook et al. 2001, cited *supra*)*.* In a particular embodiment, said recombinant vector is a vector useful to transfect fungal, plant or animal cells.

Said vector can be used to transform, transfect, transduce or infect cells susceptible of being transformed, transfected, transduced or infected by said vector. Such cells can be prokaryotic or eukaryotic. Therefore, in a seventh aspect, the invention relates to a cell, hereinafter referred to as the "cell of the invention", transformed, transfected, transduced or infected with a vector of the invention. Said transformed, transfected, transduced or infected cell comprises, therefore, the nucleic acid of the invention, the gene construct of the invention, the expression cassette of the invention, or the vector of the invention.

Transformed, transfected, transducted or infected cells may be obtained by conventional methods known to those skilled in the art (Sambrook *et al.* 2001, cited *supra*)*.* Cells suitable for performing the invention include, without limitation, mammalian, plant, insect, fungal and bacterial cells. Bacterial cells include, without limitation, cells from Gram positive bacteria such as species of the genus *Bacillus, Streptomyces* and *Staphylococcus* and Gram-negative bacterial cells such as cells of the genus *Escherichia* and

*Pseudomonas.* Fungal cells preferably include yeast cells such as *Saccharomyces, Pichia pastoris* and *Hansenula polymorpha.* Insect cells include, without limitation, *Drosophila* cells and Sf9 cells. Plant cells include, among others, cells of crop plants such as tobacco, cereals, medicinal, ornamental or bulbs., as well as algae (understood as eukaryotic and autotrophic organisms capable of photosynthesis just like plants), Mammalian cells suitable for the present invention include any cell that form a part of a mammal, wether located in the body or isolated from the body, and whether isolated from the body and later returned to or implanted or transplanted in the body. Not limiting examples are endothelial cells, epithelial cell lines, osteosarcoma cell lines, neuroblastoma cell lines, epithelial carcinomas, neural cells, glial cells, any blood cells, mesenchymal cells, hepatic cell lines, CHO (Chinese Hamster Ovary) cells, COS cells, BHK cells, HeLa cells, 911 cells, AT1080 cells, A549 cells, 293 and 293T cells, PER.C6 cells, NTERA-2 human ECCs cells, D3 cells of the mESCs line, human embryonic cells or stem cells such as HEK293, HS293, hMSCs and BGV01, SHEF1, SHEF2 and HS181, NIH3T3 cells, REH and MCF-7 cells.

In a particular embodiment, said cell is a fungal, plant or animal cell transformed, transfected, transduced or infected with a suitable vector, said transformed, transfected, transduced or infected fungal, plant or animal cell being capable of expressing the antibody of the invention, therefore said vectors can be used for expressing the antibodies of the invention in fungal, plant or animal cells.

As such, additionally to all cell types described above, the term "cell of the invention", by extension, also includes hybridoma cells producing the antibody of the invention. The term "hybridoma", as used herein, refers to the hybrid cell line formed by fusing a specific antibody-producing B cell with a myeloma (B cell cancer) cell that is selected for its ability to grow in tissue culture and for an absence of antibody chain synthesis. The antibodies produced by the hybridoma are usually of a single specificity and are therefore monoclonal antibodies (in contrast to polyclonal antibodies). The production of monoclonal antibodies was invented by César Milstein and Georges J. F. Köhler in 1975 (Köhler and Milstein, 1975, Nature 256:495-7).

In the context of the present invention, any living organism, such as unicellular organisms described above as prokaryotic or eukaryotic cells, and also pluricellular organisms, including fungi, plants or animals, can produce the antibody of the invention. Thus, in an eighth aspect, the invention relates to a living organism, hereinafter referred to as the "living organism of the invention", comprising the nucleic acid of the invention, the gene construct of the invention, the expression cassette of the invention, or the vector of the invention. In an embodiment, the living organism of the invention is selected from the group consisting of fungus, plant and animal. In another embodiment, the living organism of the invention is a non-human living organism. The terms "fungus", "plant" or "animal", as used herein, refer to a transgenic fungus, transgenic plant, including algae, or transgenic animal. As used herein, the term "transgenic fungus" refers to a fungus comprising, in one or more of its cells, an exogenous nucleic acid. Herein, the term "transgenic plant" refers to a plant comprising, in one or more of its cells, an exogenous nucleic acid. Herein, the term "transgenic animal" means a genetically modified animal whose genome has been altered by transfer of a gene or genes from other species or breed. Said genetical modification in the fungus, plant or animal is performed by way of human intervention, such as by transgenic techniques well known in the art.

The term "antibody" and its particulars have been described in detail in the context of the antibody of the invention and are used with the same meaning in the context of the nucleic acids, gene constructs, expression cassettes, vectors, cells and living organisms of the invention.

The nucleic acid, gene construct, expression cassette, vector, cell and living organism of the invention can be used to produce an antibody of the invention. In a particular embodiment, the antibody of the invention expressed or produced by the nucleic acid, gene construct, expression cassette, vector, cell, or living organism of the invention are the antibodies identified as B4 or B6 in the examples. In an embodiment, the living organism of the invention includes a fungus, plant, including algae, or animal. In another embodiment, the living organism of the invention is a non-human living organism. In another particular embodiment, the antibody of the invention is the antibody identified as B4 or the antibody identified as B6 in the examples.

Therefore, in a ninth aspect, the invention relates to a method for producing the antibody of the invention, which comprises growing the cell of the invention, or the living organism of the invention, under conditions permitting the production of said antibody of the invention. The conditions for optimising the culture of said cell will depend on the cell used. Likewise, the conditions for optimizing the development of said living organism will depend on the living organism used. In an embodiment, the conditions for optimizing the development of the fungus, plant, including algae, or animal will depend on the fungus, plant, including algae, or animal used. If desired, the method for producing the antibody of the invention further includes the isolation and purification of said antibody. In an embodiment, the living organism of the method for producing the antibody of the invention is a non-human living organism. In another embodiment, the method for producing the antibody of the invention comprising growing the cell of the invention, or the living organism of the invention, under conditions permitting the production of said antibody of the invention, wherein the method is not a method for treatment of the human or animal body by surgery, therapy or diagnostic method practiced on the human or animal body.

All the terms and embodiments previously described are equally applicable to these aspects of the invention.

### Methods for diagnosis and/or prognosis of a disease or condition associated to the ICAM-1 protein expression

The antibody of the invention can be used for the *in vitro* diagnosis and/or prognosis of a disease or condition associated to the ICAM-1 protein expression. Thus, in another aspect, the invention relates to the use of an antibody of the invention for the *in vitro* diagnosis and/or prognosis of a disease or condition associated to the ICAM-1 protein expression.

Further, in a tenth aspect, the invention relates to an *in vitro* method for the diagnosis and/or prognosis of a disease or condition associated to the ICAM-1 protein expression in a subject, hereinafter referred to as the "first method of the invention" comprising:
(a) contacting the antibody of the invention or the carrier of the invention with a sample from said subject;
(b) detecting and/or quantifying the level and/or location of said antibody or antigen-binding fragment thereof, or said carrier bound to the ICAM-1 protein in said sample;
(c) comparing the level and/or location of said antibody or antigen-binding fragment thereof, or said carrier bound to the ICAM-1 protein detected in step (b) with that of a reference value; and
(d) correlating the result obtained with the presence of a disease or condition associated to the ICAM-1 protein expression.

The terms "antibody", "ICAM-1", "carrier" and their particulars have been described in detail in the context of the antibody of the invention and carrier of the invention and are used with the same meaning in the context of the first method of the invention.

The term "subject" or "individual" or "patient" refers to members of mammalian species, and includes but is not limited to domestic animals, primates and humans; the subject is preferably a male or female human being of any age or race.

The term "diagnosing", as used herein, refers both to the process of attempting to determine and/or identify a possible disease in a subject, i.e. the diagnostic procedure, and to the opinion reached by this process, i.e. the diagnostic opinion. As will be understood by those skilled in the art, the diagnosis of a disease or condition associated to the ICAM-1 protein expression in a subject, although preferred to be, need not be correct for 100% of the subjects to be diagnosed or evaluated. The term, however, requires that a statistically significant portion of subjects are diagnosed correctly. The term "prognosis", as used herein, refers to the likelihood to predict the outcome of a subject suffering from a disease or condition associated to the ICAM-1 protein expression. As will be understood by those skilled in the art, the prognosis of said disease or condition, although preferred to be, need not be correct for 100% of the subjects to be diagnosed or evaluated. However, it requires that a statistically significant number of samples analyzed are classified correctly. In the context of both diagnosis and prognosis, whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.05, 0.01, 0.005 or lower.

To put the first method of the invention into practice, a sample, such as a biological sample, is obtained from the subject to be studied. The term "sample" or "biological sample", as used herein, includes different types of biological fluids, organs, tissues or sections of tissues of the affected organs, etc. In a particular embodiment, the sample comprises cells from the subject under study. In another particular embodiment, the sample does not comprise cells from the subject under study. Illustrative, non-limiting examples of said samples include different types of biological fluids, such as extracellular fluids, peritoneal fluid, pleural fluid, synovial fluid, urine, saliva, blood, semen, serum, sputum, etc. These biological fluid samples can be obtained by any conventional method known by persons having ordinary skill in the art. Alternatively, said sample can also be a section of an affected organ tissue sample, for example from the lymphoid gland, breast, prostate, skin, small bowel, large bowel, pancreas, ovary, lung, bladder, kidney, liver, brain, spinal cord, stomach, heart, muscle, bone, etc., which can be obtained by any conventional method, for example by means of biopsy, cystoscopy, surgical resection, etc., as well as frozen sections taken for histological purposes. In another embodiment, the ICAM-1 protein or a part of it can be present in the circulation, in extracellular fluids or in cellular o acellular implants inserted in the body.

The samples to be analysed can be obtained from subjects who have been previously diagnosed, or not diagnosed, with a disease or condition associated to the ICAM-1 protein expression, or also from a subject undergoing treatment, or who has been previously treated, for a disease or condition associated to the ICAM-1 protein expression.

According to step (a) of the first method of the invention, the antibody of the invention or the carrier of the invention is contacted with a sample from the subject under study under suitable conditions known by the skilled person in the art.

The antibody of the invention or the carrier of the invention is incubated with the sampe at a suitable temperature and for a time sufficient to allow the binding of the antibody of the invention or the carrier of the invention to the ICAM-1 protein that may be present in the sample. The temperature is preferably, but not limited to, between 20°C and 37°C. For example, the antibody of the invention or the carrier of the invention is incubated with the sample for at least 5 min, at least 10 minutes, at least 15 minutes at least 20 minutes, at least 30 minutes, at least 60 minutes, at least 120 min or more.

Optionally, in an embodiment, the first method of the invention may have an additional step between steps (a) and (b), wherein, once the antibody of the invention or the carrier of the invention is bound to the ICAM-1 protein that may be present in the sample, said antibody or said carrier not bound to the sample is separated. This step may be carried out by any method suitable for this purpose. For example, sequential washes with a suitable buffer may be carried out on the sample.

The person skilled in the art can use a number of conventional methods to detect and/or quantify the the level and/or location of the antibody of the invention or the carrier of the invention bound to the ICAM-1 protein in said sample, which are suitable for carrying out step (b) of the first method of the invention. Particularly useful are immunological and inflammatory methods. Thus, in a particular embodiment, the first method of the invention comprises contacting the antibody of the invention or the carrier of the invention with a sample comprising tumour or inflamed cells from said individual in order to detect and/or quantify the level and/or location of the antibody of the invention or the carrier of the invention bound to the ICAM-1 protein in said sample. As a result of step (b) of the first method of the invention, a value of the level or a distribution of the location of the antibody of the invention or the carrier of the invention bound to the ICAM-1 protein present in the sample is obtained.

The antibody of the invention to be used in these assays can be labelled or not labelled. The term "detectable label" or "labelling agent", as used herein, refers to a molecular label which allows the detection, localization and/or identification of the molecule to which it is attached, using suitable procedures and equipment for detection, for example by spectroscopic, photochemical, biochemical, immunochemical or chemical means. Labelling agents that are suitable for labelling the antibodies include radionuclides, enzymes, fluorophores, chemiluminescent reagents, enzyme substrates or cofactors, enzyme inhibitors, particles, dyes and derivatives, and the like. As the person skilled in the art will understand, antibodies that are not labelled need to be detected with an additional reagent, for example, a secondary antibody that is labelled. This is particularly useful in order to increase the sensibility of the detection method, since it allows the signal to be amplified.

There is a wide range of conventional assays that can be used in the present invention which use an antibody of the invention that is not labelled (primary antibody) followed by a labelled antibody (secondary antibody) for detection of the antibody of the invention (primary antibody), or the antibody of the invention that is labelled and does not require a secondary antibody for detection; these techniques include Western blot or immunoblot, ELISA (Enzyme-Linked Immunosorbent Assay), RIA (Radioimmunoassay), competitive EIA (Competitive Enzyme Immunoassay), DAS-ELISA (Double Antibody Sandwich-ELISA), immunocytochemical and immunohistochemical techniques, optical microscopy and electron microscopy, flow cytometry, fluorescence-activated cell sorting (FACS) or multiplex detection techniques based on using protein microspheres, biochips or microarrays which include the antibody of the invention. Other ways of detecting and quantifying the ICAM-1 protein using the antibody of the invention or the carrier of the invention include, but are not limited to, affinity chromatography techniques, ligand binding assays or lectin binding assays.

The first method of the invention also comprises, under step (c), the step of comparing the level and/or location of said antibody or antigen-binding fragment thereof, or said carrier bound to the ICAM-1 protein detected in step (b) with that of a reference value or location.

The term "reference value" refers to a predetermined criteria used as a reference for evaluating the values location, or distribution data obtained from the samples collected from a subject. The reference value or reference level can be an absolute value, a relative value, a value that has an upper or a lower limit, a range of values, an average value, a median value, a mean value, or a value as compared to a particular control or baseline value. The reference value according to the diagnostic and/prognostic method of the invention can be obtained from the values of the level of the antibody of the invention or the carrier of the invention bound to the ICAM-1 protein present in a sample obtained from one or more healthy subjects or subjects who do not suffer from said disease or condition associated to the ICAM-1 protein expression.

In the context of the invention, the level of the antibody of the invention or the carrier of the invention bound to the ICAM-1 protein is considered to be "increased" when the level of said antibody or carrier bound to the ICAM-1 protein in a sample is higher than a reference value. The level of said antibody or carrier bound to the ICAM-1 protein is considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more, higher than its reference value.

Likewise, the level of the antibody of the invention or the carrier of the invention bound to the ICAM-1 protein is considered to be "decreased" when the level of said antibody or carrier bound to the ICAM-1 protein in a sample is lower than a reference value. The level of said antibody or carrier bound to the ICAM-1 protein is considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more, lower than its reference value.

In an embodiment, step (b) of the first method of the invention comprises detecting and/or quantifying the ICAM-1 protein in said sample by methods known to a person skilled in the art, and which have been previously mentioned. In another embodiment, the first method of the invention further comprises a step (c) comprises comparing the presence and/or amount and/or distribution of said ICAM-1 protein detected or determined in said sample, with that of the ICAM-1 protein detected or determined in the control sample. The terms "control sample" or "reference sample", as used herein, refer to a sample that does not comprise ICAM-1 protein expression or does not comprise ICAM-1-expressing cells or comprises ICAM-1 expression or ICAM-1-expressing cells under normal physiological conditions or under conditions associated with known ICAM-1 expression levels or distribution, and therefore it can be used for establishing a reference value.

In a particular embodiment, the reference sample is taken from a healthy subject, although it may also be taken from other subjects, including subjects who do not have a clinical history of a disease or condition associated to the ICAM-1 protein expression, the same subject but from a tissue or part thereof which is under a normal physiological condition or healthy.

In a particular embodiment, the control sample is a sample from a healthy subject. In another particular embodiment, the control sample is a sample from an individual who does not have a clinical history of a disease or condition associated to the ICAM-1 protein expression, in which case the results obtained could have a diagnostic or predictive value of the malignancy, invasiveness, stage and/or severity of said a disease or condition associated to the ICAM-1 protein expression in the subject. In another particular embodiment, the control sample is a sample from the same subject but from a tissue or part thereof which is healthy. A positive control is, for example, a sample that expresses or comprises the ICAM-1 protein or a cell line that expresses the ICAM-1 protein, and/or a negative control is, for example, a sample that does not express or does not comprise the ICAM-1 protein or a cell line that does not express the ICAM-1 protein, which can additionally be used if desired. In another embodiment, the control sample or reference sample refers to a sample collection formed preferably by a mixture of samples that do not comprise ICAM-1 expression or ICAM-1-expressing cells or comprise ICAM-1 expression or ICAM-1-expressing cells under normal physiological conditions, or a sample that comprises ICAM-1 or cells expressing ICAM-1 in levels that are known, and therefore can be used for establishing a reference value. Said reference value can be determined by means of techniques well known in the state of the art, for example determining the mean levels of the ICAM-1 protein measured in samples that do not comprise ICAM-1 expression or ICAM-1-expressing cells or comprise ICAM-1 expression or ICAM-1-expressing cells under normal physiological conditions or under conditions associated with known ICAM-1 expression levels or in samples taken from healthy subjects. In a particular embodiment, the reference sample is taken from a group of healthy subjects, although it may also be taken from other subjects, including subjects who do not have a clinical history of a disease or condition associated to the ICAM-1 protein expression.

In a particular embodiment, the control sample is a sample from healthy subjects. In another particular embodiment, the control sample is a sample from individuals who do not have a clinical history of a disease or condition associated to the ICAM-1 protein expression, in which case the results obtained could have a diagnostic or predictive value of the malignancy, invasiveness, stage and/or severity of said a disease or condition associated to the ICAM-1 protein expression in the subject. A positive control, for example a sample that expresses the ICAM-1 protein or a cell line that expresses the ICAM-1 protein, and/or a negative control, for example a sample that does not express the ICAM-1 protein or a cell line that does not express the ICAM-1 protein, can additionally be used if desired.

The term "distribution", as used herein, refers to the ICAM-1 protein expression in a sample without cells or to the population or type of cells where the ICAM-1 protein is expressed in a sample. Thus, the ICAM-1 protein may be expressed in a sample with no cells or in one or more than one particular cell population or type. By cell population or cell type it is understood morphologically and phenotypically distinct cells, such as T cells, B cells, macrophages, epithelial cells, muscle cells, osteoclasts, osteoblasts, neurons, etc. Thus, by comparing the distribution of the ICAM-1 protein between the sample to be analysed and the reference sample, one is comparing the presence of the ICAM-1 protein in the different cell populations present in the said samples.

The first method of the invention additionally comprises, as step (d), the step of correlating the result obtained from the comparison of step (c) with the presence of a disease or condition associated to the ICAM-1 protein expression, the determination or prognosis of the malignancy, invasiveness, stage and/or severity of said disease or condition associated to the ICAM-1 protein expression in said subject.

In a particular embodiment, an increased level of an antibody or antigen-binding fragment thereof, or carrier bound to the ICAM-1 protein detected in step (b) of the first method of the invention when compared to the reference value is indicative of the presence of a disease or condition associated to the ICAM-1 protein expression.

In another particular embodiment, a decreased level of an antibody or antigen-binding fragment thereof, or carrier bound to the ICAM-1 protein detected in step (b) of the first method of the invention when compared to the reference value is indicative of the presence of a disease or condition associated to the ICAM-1 protein expression.

In a particular embodiment, the presence of the ICAM-1 protein in the sample being analysed when the ICAM-1 protein is not present in the reference sample is indicative of the subject having a disease or condition associated to the ICAM-1 protein expression.

In another particular embodiment, an altered distribution of the ICAM-1 protein in the sample being analysed in respect with that of the ICAM-1 protein in the reference sample is indicative of the subject having a disease or condition associated to the ICAM-1 protein expression. It is considered that the distribution of the ICAM-1 protein is "altered" when the ICAM-1 protein is expressed differently in a sample without cells, or in a different cellular location with respect to the reference sample, or in a different cell population with respect to the reference sample, or when the ICAM-1 protein is expressed in at least one different additional cell population with respect to the reference sample.

In another particular embodiment, an increase in the amount said the ICAM-1 protein in the sample from said subject with respect to the amount of the ICAM-1 protein in said reference sample is indicative of a disease or condition associated to the ICAM-1 protein expression. It is considered that the amount of the ICAM-1 protein in the sample being analysed is "increased" in respect to the amount of the ICAM-1 protein in the reference sample when it increases in at least 1%, at least 2%, at least 3%, at least 4%, at least 5%, at least 10%, at least 15%, at least 20%, at least 25%, at least 30%, at least 40%, at least 50%, at least 60%, at least 70%, at least 80%, at least 90%, at least 100%, at least 110%, at least 120%, at least 150%, at least 200%, at least 500%, at least 1000% or more in respect to the reference sample.

In another particular embodiment, a decrease in the amount said the ICAM-1 protein in the sample from said subject with respect to the amount of the ICAM-1 protein in said reference sample is indicative of a disease or condition associated to the ICAM-1 protein expression. It is considered that the amount of the ICAM-1 protein in the sample being analysed is "decreased" in respect to the amount of the ICAM-1 protein in the reference sample when it decreases in at least 1%, at least 2%, at least 3%, at least 4%, at least 5%, at least 10%, at least 15%, at least 20%, at least 25%, at least 30%, at least 40%, at least 50%, at least 60%, at least 70%, at least 80%, at least 90%, at least 100%, at least 110%, at least 120%, at least 150%, at least 200%, at least 500%, at least 1000% or more in respect to the reference sample.

Said information can also be used to determine and/or evaluate and/or predict the malignancy, invasiveness, stage and/or severity of said disease or condition associated to the ICAM-1 protein expression in said subject; in this sense, the person skilled in the art, in view of the experience that he/she has acquired, will be able to correlate the amount of the ICAM-1 protein determined in the sample from the subject under study with the malignancy, invasiveness, stage and/or severity of said disease or condition associated to the ICAM-1 protein expression in said subject. Likewise, when the control sample is a sample from the subject himself/herself diagnosed with a disease or condition associated to the ICAM-1 protein expression, analysed before or during the administration of a therapy for treating said disease or condition associated to the ICAM-1 protein expression, the information relating to the detection or to the variation in the distribution and/or amount of said the ICAM-1 protein in the sample from said subject at a given time after the administration of said therapy, can serve for monitoring or evaluating the effect or efficacy of the therapy administered to said subject who suffers from said disease or condition associated to the ICAM-1 protein expression, as it will be described below, and, if said therapy is not effective, evaluating the possibility of changing it.

As it will be understood by those skilled in the art, the prediction, although preferred to be, need not be correct for 100% of the subjects to be diagnosed or evaluated. The term, however, requires that a statistically significant portion of subjects can be identified as having an increased probability of having a given outcome. Whether the data obtained from a subject is statistically significant can be determined without further ado by the person skilled in the art using various well known statistic evaluation tools, e.g., determination of confidence intervals, p-value determination, cross-validated classification rates and the like etc. Details are found in Dowdy and Wearden, Statistics for Research, John Wiley & Sons, New York 1983. Preferred confidence intervals are at least 50%, at least 60%, at least 70%, at least 80%, at least 90% or at least 95%. The p-values are, preferably, 0.01, 0.005 or lower.

The term "disease or condition associated to the ICAM-1 protein expression", as used herein, as used herein, refers to a disease or condition in which the ICAM-1 protein is expressed in an altered way, location or distribution, or in altered amount, for example a higher value or a lower value, with respect to normal or reference physiological conditions or reference values. As such, the term "disease or condition associated to the ICAM-1 protein expression" is substantially equivalent to "disease or condition wherein ICAM-1 protein expression is, directly or indirectly, implied" or similar.

In another embodiment, the disease or condition associated to the ICAM-1 protein expression can be defined as a disease or condition wherein cells expressing the ICAM-1 protein participate. The term "disease or condition wherein cells expressing the ICAM-1 protein participate", as used herein, refers to a disease or condition in which cells expressing the ICAM-1 protein are directly or indirectly involved independently that the ICAM-1 protein is, or not, the responsible of the disease or condition, including, for example, diseases or conditions in which the ICAM-1 protein is expressed in an altered way, location or distribution, or in altered amount, for example a higher value or a lower value, with respect to normal or reference physiological conditions or reference values. As such, the term "disease or condition wherein cells expressing the ICAM-1 protein participate" is substantially equivalent to "disease or condition wherein cells expressing the ICAM-1 protein are, directly or indirectly, implied" or similar.

In a particular embodiment, said disease or condition associated to the ICAM-1 protein expression is selected from the group consisting of a tumour disease and a non-tumour disease.

In a preferred embodiment, said disease or condition associated to the ICAM-1 protein expression is selected from the group consisting of cancer, an infectious disease, an inflammatory disease, a metabolic disease, a pulmonary disease, a cardiovascular disease, a neurological disease, a genetic disease, and an immune disease.

The term "cancer", as used herein, refers to a disease characterized by uncontrolled cell division (or by an increase of survival or apoptosis resistance) and by the ability of said cells to invade other neighbouring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. As used herein, the term cancer includes, but is not limited to, the following types of cancer: breast cancer; biliary tract cancer; bladder cancer; brain cancer including glioblastomas, in particular glioblastoma multiforme, and medulloblastomas; cervical cancer; head and neck carcinoma; choriocarcinoma; colon cancer, colorectal cancer; endometrial cancer; esophageal cancer; gastric cancer; hematological neoplasms including acute lymphocytic and myelogenous leukemia; T-cell acute lymphoblastic leukemia/lymphoma; hairy cell leukemia; chronic myelogenous leukemia, multiple myeloma; AIDS-associated leukemias and adult T-cell leukemia/lymphoma; intraepithelial neoplasms including Bowen's disease and Paget's disease; liver cancer, hepatoma; lung cancer, pleural mesothelioma; lymphomas including Hodgkin's disease and lymphocytic lymphomas; neuroblastomas; oral cancer including squamous cell carcinoma; parotid gland cancer; ovarian cancer including those arising from epithelial cells, stromal cells, germ cells and mesenchymal cells; pancreatic cancer; prostate cancer; kidney cancer, suprarenal cancer; rectal cancer; sarcomas including leiomyosarcoma, rhabdomyosarcoma, liposarcoma, fibrosarcoma, and osteosarcoma; skin cancer including melanoma, Merkel cell carcinoma, Kaposi's sarcoma, basal cell carcinoma, and squamous cell cancer; cervix cancer, endometrial cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor. Other cancers will-be known to one of ordinary skill. In a particular embodiment, the cancer is as gastric, duodenal, liver, hematologic, breast, glioma, bladder, pancreatic, prostate, colorectal, cervical, nasopharyngeal, lung, kidney, metastasis, melanoma, sarcoma, neural, or skin cancer. Other cancers will-be known to one of ordinary skill in the art. In a particular embodiment, the cancer is as lung cancer, mesothelioma, brain cancer, breast cancer, pancreatic cancer, colon cancer, ovarian cancer, prostate cancer, head and neck cancer, liver cancer, or leukemia.

The term "infectious disease", as used herein, refers to relates to diseases caused by pathogens such as viruses, bacteria, fungi, protozoa, and parasites. Infectious diseases may be caused by viruses including rhinovirus, A-type coxsackievirus, adenovirus, cytomegalovirus, dengue, Epstein-Barr, hanta, hepatitis A, hepatitis B, hepatitis C, herpes simplex type I, herpes simplex type II, human immunodeficiency virus (HIV), human papilloma virus (HPV), influenza, measles, mumps, papova virus, polio, respiratory syncytial virus, rinderpest, rhinovirus, rotavirus, rubella, SARS virus, smallpox, viral meningitis, and the like. Infectious diseases may also be caused by bacteria including *Bacillus antracis, Borrelia burgdorferi, Campylobacter jejuni, Chlamydia trachomatis, Clostridium botulinum, Clostridium tetani, Diptheria, E. coli, Legionella, Helicobacter pylori, Mycobacterium rickettsia, Mycoplasma nesisseria, Pertussis, Pseudomonas aeruginosa, S. pneumonia, Streptococcus, Staphylococcus, Vibria cholerae, Yersinia pestis,* and the like. Infectious diseases may also be caused by fungi such as *Aspergillus fumigatus, Blastomyces dermatitidis, Candida albicans, Coccidioides immitis, Cryptococcus neoformans, Histoplasma capsulatum, Penicillium marneffei,* and the like. Infectious diseases may also be caused by protozoa and parasites such as *chlamydia, kokzidioa, leishmania, malaria (Plasmodiun falciparum), rickettsia, trypanosoma,* and the like. In a particular embodiment, the infectious disease is common cold, acquired immunodeficiency syndrome (AIDS), malaria, a respiratory viral infection, or an enteric viral infection. In a particular embodiment, the infectious disease is as common cold, COVID-19, herpes, acquired immunodeficiency syndrome (AIDS), influenza, tuberculosis, malaria, or an enteric viral infection.

The term "inflammatory disease", as used herein, refers to a condition in a subject characterized by inflammation, e.g., chronic inflammation. Illustrative, non-limiting examples of inflammatory disorders include, but are not limited to, Celiac Disease, rheumatoid arthritis (RA), Inflammatory Bowel Disease (IBD), asthma, encephalitis, chronic obstructive pulmonary disease (COPD), inflammatory osteolysis, allergic disorders, septic shock, pulmonary fibrosis (e.g., idiopathic pulmonary fibrosis), inflammatory vacultides (e.g., polyarteritis nodosa, Wegner's granulomatosis, Takayasu's arteritis, temporal arteritis, and lymphomatoid granulomatosus), post-traumatic vascular angioplasty (e.g., restenosis after angioplasty), undifferentiated spondyloarthropathy, undifferentiated arthropathy, arthritis, inflammatory osteolysis, chronic hepatitis, and chronic inflammation resulting from chronic viral or bacteria infections. The term "inflammatory disease" also includes immune mediated inflammatory disease, which shall be taken to mean any disease mediated by the immune system and characterized by chronic or acute inflammation, resulting from, associated with or triggered by, a dysregulation of the normal immune response e.g. Crohn's disease, type 1 diabetes mellitus, rheumatoid arthritis, inflammatory bowel disease, psoriasis, psoriatic arthritis, ankylosing spondylitis, systemic lupus erythematosus, Hashimoto's disease, graft-versus-host disease, Sjogren's syndrome, pernicious anemia, Addison disease, scleroderma, Goodpasture's syndrome, ulcerative colitis, autoimmune hemolytic anemia, sterility, myasthenia gravis, multiple sclerosis, Basedow's disease, thrombopenia purpura, Guillain-Barré syndrome, allergy, asthma, atopic disease, arteriosclerosis, myocarditis, cardiomyopathy, glomerular nephritis, hypoplastic anemia, and rejection after organ transplantation. In a particular embodiment, the inflammatory disease is as asthma, diabetes, lupus, ulcerative colitis, psoriasis, chronic obstructive pulmonary disease, vasculitis, arthritis, hepatitis, systemic sclerosis, gout, myositis, or scleroderma.

The term "metabolic disease" or "metabolic disorder", as used herein, is understood as all types of disorders that lead to errors and imbalances in the metabolism as well as to metabolic processes taking place in a sub-optimal form. The expression also relates to disorders that can be treated through metabolism modulation, although the disease in itself may not have been caused by a metabolic disorder. In a particular embodiment, the metabolic disease is as glycogen storage disease, hypercholesterolemia, obesity, diabetes, atherosclerosis, lysosomal storage disorder, fatty liver disease, or inborn errors of metabolism.

The term "pulmonary disease", as used herein, relates to those types of diseases that affect the lungs and other parts of the respiratory system, thus the entire respiratory tract that are involved in breathing. These organs include the nose, throat, larynx, trachea, bronchi, and lungs. In a particular embodiment, the pulmonary disease is as asthma, cystic fibrosis, acid sphingomyelinase deficiency, Fabry disease, pneumonia, bronchitis, pulmonary edema, pulmonary fibrosis, interstitial lung disease, pneumothorax, mesothelioma, lung cancers, acute respiratory distress syndrome, chronic obstructive pulmonary disease, emphysema, bronchitis, or pulmonary thrombosis.

The term "cardiovascular disease" or "cardiovascular disorder", as used herein, relates to diseases affecting the heart or blood vessels or both or associated with the cardiopulmonary and circulatory systems including but not limited to ischemia, angina, edematous conditions, artherosclerosis, Coronary Heart Disease, LDL oxidation, adhesion of monocytes to endothelial cells, foam-cell formation, fatty-streak development, platelet adherence, and aggregation, smooth muscle cell proliferation, reperfusion injury, high blood pressure, thrombotic disease, arrhythmia (atrial or ventricular or both); cardiac rhythm disturbances; myocardial ischemia; myocardial infarction; cardiac or vascular aneurysm; vasculitis, stroke; peripheral obstructive arteriopathy of a limb, an organ, or a tissue; reperfusion injury following ischemia of the brain, heart or other organ or tissue, endotoxic, surgical, or traumatic shock; hypertension, valvular heart disease, heart failure, abnormal blood pressure; shock; vasoconstriction (including that associated with migraines); vascular abnormality, inflammation, insufficiency limited to a single organ or tissue. In a particular embodiment, the cardiovascular disease is as stroke, aneurysm, hypertension, cardiomyopathy, heart failure, heart attack, coronary artery disease, peripheral arterial disease, angina, heart valve disease, atrial fibrillation, arrhythmia, thrombosis, endocarditis, cerebrovascular disease, or atherosclerosis.

The term "neurological disease" or "neurological condition" as used herein, includes neurological related maladies such as Parkinson's disease, Alzheimer's disease, spinal cord injury, amyotrophic lateral sclerosis, ataxia, epilepsy, depression, bipolar disorder, schizophrenia, or congenital diseases that affect the central nervous system.

The term "genetic disease" or "genetic disorder", as used herein, refers to any disease or abnormality that results from inherited factors. The term "genetic disease" includes any of those diseases listed in the online catalogue of Mendelian Disease that is entitled "Online Mendelian Inheritance in Man" (known as OMIM, www.omim.org/). In a particular embodiment, the genetic disease is as cystic fibrosis, familiar hypercholesterolemia, lysosomal storage disease, Gaucher disease, Pompe disease, Muccopolysaccharidosis, Niemann-Pick disease, acid sphingomyelinase disease, Fabry disease, Duchenne muscular dystrophy, sickle cell disease, congenital glycosylation disorders, or congenital heart disease.

The term "immune disease", as used herein, refers to a disease caused by the excessive response of the immune system in the human body, and typical examples of immune disease include allergic diseases. Illustrative, non-limiting examples of immune diseases include inflammatory bowel disease, lupus, asthma, psoriasis, rheumatoid arthritis, multiple sclerosis, Hashimoto thyroiditis, Grave's disease, Type 1 diabetes, vitiligo, host versus graft disease, Crohn's disease, ulcerative colitis, celiac disease, Sjogren's syndrome, Kawasaki disease, or allergy.

In another particular embodiment, the cells in which the expression of the ICAM-1 protein is altered comprise a subpopulation of lymphoid, myeloid, dendritic, plasmacytoid cells, which are defined by additional at least one of the markers including CD2, CD3, CD4, CD5, CD8, CD11, CD14, CD19, CD20, CD25, CD51, CD81, CD146, CD228, CD231, TCR and combinations thereof.

A preferred embodiment comprises a rapid test in tissue in a procedure where histological tissue sample is stained with antibodies of the invention fluorescently labelled. In a further preferred process the antibody of the invention, preferably an IgG isotype antibody, is combined with an additional antibody, which specifically recognizes CD2, CD3, CD4, CD5, CD8, CD11, CD14, CD19, CD20, CD25, CD51, CD81, CD146, CD228, CD231, TCR. In a further preferred embodiment the antibodies are directly labelled with different fluorescent dyes, such as Cy3 and Cy5 or Cy3 and FITC.

In an embodiment, in which a signal enhancement is advantageous, antibodies and/or recognition molecules are increased by labelled secondary antibodies or biotin-streptavidin detection systems. It is therefore advantageous to use isotypes and/or sequences of different species in the constant region of the antibody. The technologies and methods used in this document, for example immunohistology, and the choice of the appropriate format of the recognition molecules are known to the skilled artisan.

Additionally, the antibody of the invention, in view of its ability to specifically bind target cells (i.e., cells expressing the ICAM-1 protein, or ICAM-1⁺ cells), can also be used for *in vivo* diagnosis and/or prognosis of a disease or condition associated to the ICAM-1 protein expression or a disease or condition associated to the ICAM-1 protein expression; by illustrative, said antibodies can be used in medical imaging, i.e., a set of techniques and processes used to create images of a body (or parts and function thereof), e.g., a human body, for clinical purposes, such as medical procedures seeking to reveal, diagnose or examine a disease, or for medical science, including the study of normal anatomy and physiology.

Therefore, in another aspect, the invention relates to the use of the antibody of the invention for *in vivo* or *in vitro* diagnosis and/or prognosis of a disease or condition associated to the ICAM-1 protein expression, or to the use of the antibody of the invention in a method for *in vivo* or *in vitro* diagnosis and/or prognosis of a disease or condition associated to the ICAM-1 protein expression; alternatively, this aspect can be expressed as the use of the antibody of the invention in the manufacture of a composition for *in vivo* or *in vitro* diagnosis and/or prognosis of a disease or condition associated to the ICAM-1 protein expression. In another alternative wording, this aspect can be expressed as the antibody of the invention for use in an *in vivo* or *in vitro* diagnosis and/or prognosis of a disease or condition associated to the ICAM-1 protein expression, or as the antibody of the invention for use in a method for *in vivo* or *in vitro* diagnosis and/or prognosis of a disease or condition associated to the ICAM-1 protein expression.

To this end, antibodies of the invention are labelled by suitable methods known in the art, and are provided as agents for diagnostic imaging methods, such as radioimmunodiagnostics, positron emission tomography (PET), endoscopy immunofluorescent methods, etc., for example by means of coupling and/or loading with appropriate molecules, for example radioactive isotopes or fluorescent dyes. In a preferred embodiment, the antibody of the invention is coupled to gamma-emitting istotopes, for example, ^{99m}Tc, ¹²³I, and ¹¹¹In, and used in radioimmunoscintigraphy using gamma cameras or single-photon emission computed tomography. In another preferred embodiment, the antibody of the invention is coupled to positron emitters, for example, ¹⁸F, ⁶⁴Cu, ⁶⁸Ga, ⁸⁶Y, and ¹²⁴I, and used in PET. In another preferred embodiment, the antibody of the invention is coupled to fluorescent dyes, such as Cy3, Cy2, Cy5 or FITC, and used in endoscopy immunofluorescent methods. The antibodies modified as described are administered by any suitable route, for example, intravenously, at an appropriate dose for the individual and the location of ICAM-1 is detected, determined or measured by processes well known in the art. The methods and technologies used herein, including diagnostic imaging, are known to the skilled artisan, who can also provide suitable dose formulations.

In a further preferred embodiment, the antibody of the invention is radioactively labelled, for instance with ¹¹¹In, and is provided as diagnostic agent which can be given locally in the tumour or in the blood vessels of the tumour afferent or efferent or systemically. This serves in one embodiment for the determination of tumour size and a further embodiment for the determination of the lymph nodes. The methods and technologies used herein, including diagnostic imaging, are known to the skilled artisan, who can also provide suitable dosage formulations.

The radiolabelled recognition molecules of the invention used in diagnostic assays or as diagnostic agents may also be administered by other routes of application. For this, preferred but not limiting routes are intraperitoneal, intranodal, intrathecal, intratumoural, intraarticular, intraarterial and intravenous routes.

Further, the antibody of the invention will be formulated in a pharmaceutical composition suitable for its administration to a subject. To that end, the pharmaceutical composition will include the necessary pharmaceutically acceptable excipients or carriers. Illustrative, non-limiting, examples of said compositions will be described below in the aspect of the pharmaceutical compositions and are incorporated herein by reference.

All the terms and embodiments previously described are equally applicable to this aspect of the invention.

### Methods for monitoring the response to a treatment of a disease or condition associated to the ICAM-1 protein expression

In an eleventh aspect, the invention relates to an *in vitro* method for monitoring the response to a treatment of a disease or condition associated to the ICAM-1 protein expression in a subject under treatment, hereinafter referred to as the "second method of the invention", comprising:
(a) contacting the antibody of the invention or the carrier of the invention with a first sample from said subject taken at a first time-point;
(b) detecting and/or quantifying the level and/or location of said antibody or antigen-binding fragment thereof, or said carrier bound to the ICAM-1 protein in said first sample;
(c) contacting the antibody of the invention or the carrier of the invention with a second sample from said subject taken at a second time-point;
(d) detecting and/or quantifying the level and/or location of said antibody or antigen-binding fragment thereof, or said carrier bound to the ICAM-1 protein in said second sample;
(e) comparing the level and/or location of said antibody or antigen-binding fragment thereof, or said carrier bound to the ICAM-1 protein detected in step (b) with the level and/or location of said antibody or antigen-binding fragment thereof, or said carrier bound to the ICAM-1 protein detected in step (d); and
(f) correlating the result obtained with the response to the treatment of a disease or condition associated to the ICAM-1 protein expression.

The terms "antibody", "ICAM-1", "carrier", "sample", "subject", "reference sample", "disease or condition associated to the ICAM-1 protein expression", "distribution", "altered" and their particulars have been described in detail in the context of the antibody of the invention, carrier of the invention and in the first method of the invention and are used with the same meaning in the context of the second method of the invention.

The term "treatment", as used herein, refers to the therapy suitable for the remediation of the disease or condition associated to the ICAM-1 protein expression. The term "treatment" or "therapy" can be used indistinctly and refer to clinical intervention in an attempt to prevent, cure, delay, reduce the severity of, or ameliorate one or more symptoms of the disease or disorder or recurring disease or disorder, or in order to prolong the survival of a patient beyond that expected in the absence of such treatment. Suitable treatments for a disease or condition associated to the ICAM-1 protein expression are conventional and well-known by the person skilled in the art.

In a preferred embodiment, said disease or condition associated to the ICAM-1 protein expression is selected from the group consisting of cancer, an infectious disease, an inflammatory disease, a metabolic disease, a pulmonary disease, a cardiovascular disease, a neurological disease, a genetic disease, and an immune disease.

According to step (a) of the second method of the invention, the antibody of the invention or the carrier of the invention is contacted with a first sample from said subject taken at a first time-point. As the person skilled in the art will appreciate, step (a) of the second method of the invention is identical to step (a) of the first method of the invention, which has been described in detail in the first method of the invention. Thus, the particulars of step (a) of the first method of the invention are incorporated herein by reference.

Optionally, in an embodiment, the second method of the invention may have an additional step between steps (a) and (b), which comprises separating the antibody of the invention or the carrier of the invention not bound to the first sample. This optional step of separation between steps (a) and (b) of the second method of the invention is identical to the optional step of separation between steps (a) and (b) of the first method of the invention, which has been described in detail in the first method of the invention. Thus, the particulars of the optional step of separation between steps (a) and (b) of the first method of the invention are incorporated herein by reference.

Step (b) of the second method of the invention comprises detecting and/or quantifying the level and/or location of said antibody or antigen-binding fragment thereof, or said carrier bound to the ICAM-1 protein in said first sample. Step (b) of the second method of the invention is identical to step (b) of the first method of the invention, which has been described in detail in the first method of the invention. Thus, the particulars of step (b) of the first method of the invention are incorporated herein by reference.

The second method of the invention further comprises steps (c) and (d) comprising contacting the antibody of the invention or the carrier of the invention with a second sample (step (c)), and detecting and/or quantifying the level and/or location of said antibody or antigen-binding fragment thereof, or said carrier bound to the ICAM-1 protein in said second sample, wherein said second sample is taken at a second time-point (step (d)).

Optionally, in an embodiment, the second method of the invention may have an additional step between steps (c) and (d), which comprises separating the antibody of the invention or the carrier of the invention not bound to the second sample. This optional step of separation between steps (c) and (d) of the second method of the invention is identical to the optional step of separation between steps (a) and (b) of the first method of the invention, thus, the particulars of the optional step of separation between steps (a) and (b) of the first method of the invention are incorporated herein by reference.

The term "time point", as used herein, refers to a moment in time at which a sample is taken from the subject being monitored. In the context of the second method of the invention, there is a first or initial time point, at which the first sample is taken, and a second or later time point, at which the second sample is taken. The first and second time points are separated in time by at least 1 day, at least 2 days, at least 3 days, at least 4 days, at least 5 days, at least 10 days, at least 20 days, at least 30 days, at least 40 days, at least 50 days, at least 60 days, at least 70 days, at least 80 days, at least 90 days, at least 100 days, at least 6 months, at least 1 year, at least 2 years, at least 5 years, at least 10 years, or more. In a particular embodiment, the first time point happens before the second time point, so that the first sample is taken before the second sample.

As the person skilled in the art will realise, in a particular embodiment, it is particularly useful that the first sample is taken before the subject begins treatment for a disease or condition associated to the ICAM-1 protein expression in order to fully monitor the response to said treatment. Thus, in a particular embodiment, the first time point occurs before treatment for a disease or condition associated to the ICAM-1 protein expression has begun. In another particular embodiment, the first time point occurs when treatment for a disease or condition associated to the ICAM-1 protein expression has begun. Likewise, in a particular embodiment, the first sample is taken before treatment for a disease or condition associated to the ICAM-1 protein expression has begun, whereas in another particular embodiment, the first sample is taken when treatment for a disease or condition associated to the ICAM-1 protein expression has begun.

In order to monitor the response of said subject to said treatment, it will be necessary that the second sample is taken once the subject has begun said treatment. Thus, in another particular embodiment, the second time point occurs after treatment for a disease or condition associated to the ICAM-1 protein expression has begun. In a particular embodiment, the second time point occurs at least 1 day, at least 2 days, at least 3 days, at least 4 days, at least 5 days, at least 10 days, at least 20 days, at least 30 days, at least 40 days, at least 50 days, at least 60 days, at least 70 days, at least 80 days, at least 90 days, at least 100 days, at least 6 months, at least 1 year, at least 2 years, at least 5 years, at least 10 years, or more after treatment for a disease or condition associated to the ICAM-1 protein expression has begun. Likewise, the second sample is taken after treatment for a disease or condition associated to the ICAM-1 protein expression has begun. In a particular embodiment, the second sample is taken at least 1 day, at least 2 days, at least 3 days, at least 4 days, at least 5 days, at least 10 days, at least 20 days, at least 30 days, at least 40 days, at least 50 days, at least 60 days, at least 70 days, at least 80 days, at least 90 days, at least 100 days, at least 6 months, at least 1 year, at least 2 years, at least 5 years, at least 10 years, or more after treatment for a disease or condition associated to the ICAM-1 protein expression has begun.

The second method of the invention also comprises the step (e) of comparing the level and/or location of said antibody or antigen-binding fragment thereof, or said carrier bound to the ICAM-1 protein detected in step (b) with the level and/or location of said antibody or antigen-binding fragment thereof, or said carrier bound to the ICAM-1 protein detected in step (d) prior to the correlation step [step (f)] wherein the result obtained from the comparison of step (e) is correlated with the response to treatment of a disease or condition associated to the ICAM-1 protein expression.

In the context of the invention, the level of the antibody of the invention or the carrier of the invention bound to the ICAM-1 protein is considered to be "increased" when the level of said antibody or carrier bound to the ICAM-1 protein in the first sample (step (b)) is higher than the level of the antibody of the invention or the carrier of the invention bound to the ICAM-1 protein detected in the second sample (step (d)). The level of said antibody or carrier bound to the ICAM-1 protein in the fist sample is considered to be higher than the level of said antibody or carrier bound to the ICAM-1 protein in the second sample when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more, higher than the level of said antibody or carrier bound to the ICAM-1 protein in the second sample.

Likewise, the level of the antibody of the invention or the carrier of the invention bound to the ICAM-1 protein is considered to be "decreased" when the level of said antibody or carrier bound to the ICAM-1 protein in the first sample (step (b)) is lower than the level of the antibody of the invention or the carrier of the invention bound to the ICAM-1 protein detected in the second sample (step (d)). The level of said antibody or carrier bound to the ICAM-1 protein in the fist sample is considered to be lower than the level of said antibody or carrier bound to the ICAM-1 protein in the second sample when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more, lower than the level of said antibody or carrier bound to the ICAM-1 protein in the second sample.

In a particular embodiment, an increased level of an antibody or antigen-binding fragment thereof, or carrier bound to the ICAM-1 protein detected in step (b) of the second method of the invention when compared to the level of the antibody or antigen-binding fragment thereof, or carrier bound to the ICAM-1 protein detected in the second sample (step (d)) is indicative of the presence of a disease or condition associated to the ICAM-1 protein expression.

In a particular embodiment, an decreased level of an antibody or antigen-binding fragment thereof, or carrier bound to the ICAM-1 protein detected in step (b) of the second method of the invention when compared to the level of the antibody or antigen-binding fragment thereof, or carrier bound to the ICAM-1 protein detected in the second sample (step (d)) is indicative of the presence of a disease or condition associated to the ICAM-1 protein expression.

In another embodiment, step (b) of the second method of the invention comprises detecting and/or quantifying the ICAM-1 protein in said first sample by methods known to a person skilled in the art, and which have been previously mentioned.

In an embodiment, the second method of the invention further comprises steps (c) and (d) comprising contacting the antibody of the invention or the carrier of the invention with a second sample (step (c)), and detecting and/or quantifying the ICAM-1 protein in said second sample, wherein said second sample is taken at a second time-point (step (d)).

In an embodiment, the second method of the invention comprises the step of comparing the presence and/or amount and/or distribution of the ICAM-1 protein detected in said first and second samples [step (e)] prior to the correlation step [step (f)] wherein the result obtained from the comparison of step (e) is correlated with the response to treatment of a disease or condition associated to the ICAM-1 protein expression.

In a particular embodiment, the lack of presence or reduced presence of the ICAM-1 protein in the second sample in respect to the first sample is, or may be, indicative of a favourable response to said treatment. Alternatively, the presence or increased presence of the ICAM-1 protein in the second sample in respect to the first sample is, or maybe, indicative of an unfavourable response to said treatment.

In another embodiment, the lack of presence or reduced presence of the ICAM-1 protein in the second sample in respect to the first sample is, or may be, indicative of an unfavourable response to said treatment. Alternatively, the presence or increased presence of the ICAM-1 protein in the second sample in respect to the first sample is, or maybe, indicative of a favourable response to said treatment.

In another particular embodiment, an altered distribution of the ICAM-1 protein in the second sample in respect to that of the first sample, where the ICAM-1 protein is less expressed in the second sample with respect to the first sample, or where the ICAM-1 protein is expressed in different cellular or non-cellular sites, or in fewer or less cell populations in the second sample with respect to the first sample, is indicative of a favourable response to said treatment. On the other hand, an unaltered distribution of the ICAM-1 protein in the second sample in respect with that of the first sample, or an altered distribution of the ICAM-1 protein in the second sample in respect to that of the first sample, where the ICAM-1 protein is more expressed in the second sample with respect to the first sample, or where the ICAM-1 protein is expressed in different cellular or non-cellular sites, or in more cell populations in the second sample with respect to the first sample, is indicative of an unfavourable response to said treatment.

In another particular embodiment, an altered distribution of the ICAM-1 protein in the second sample in respect to that of the first sample, where the ICAM-1 protein is less expressed in the second sample with respect to the first sample, or where the ICAM-1 protein is expressed in different cellular or non-cellular sites, or in fewer or less cell populations in the second sample with respect to the first sample, is indicative of an unfavourable response to said treatment. On the other hand, an altered distribution of the ICAM-1 protein in the second sample in respect to that of the first sample, where the ICAM-1 protein is more expressed in the second sample with respect to the first sample, or where the ICAM-1 protein is expressed in different cellular or non-cellular sites, or in more cell populations in the second sample with respect to the first sample, is indicative of a favourable response to said treatment.

In another particular embodiment, a decrease in the amount of the ICAM-1 protein in the second sample with respect to the amount of the ICAM-1 protein in the first sample is indicative of a favourable response to said treatment. It is considered that the amount of the ICAM-1 protein in the second sample is decreased in respect to the amount of the ICAM-1 protein in the first sample when it decreases in at least 1%, at least 2%, at least 3%, at least 4%, at least 5%, at least 10%, at least 15%, at least 20%, at least 25%, at least 30%, at least 40%, at least 50%, at least 60%, at least 70%, at least 80%, at least 90%, at least 100%, at least 110%, at least 120%, at least 150%, at least 200%, at least 500%, at least 1000% or more in respect to the first sample. Likewise, an increase in the amount of the ICAM-1 protein in the second sample with respect to the amount of the ICAM-1 protein in the first sample is indicative of an unfavourable response to said treatment. It is considered that the amount of the ICAM-1 protein in the second sample is increased in respect to the amount of the ICAM-1 protein in the first sample when it increases in at least 1%, at least 2%, at least 3%, at least 4%, at least 5%, at least 10%, at least 15%, at least 20%, at least 25%, at least 30%, at least 40%, at least 50%, at least 60%, at least 70%, at least 80%, at least 90%, at least 100%, at least 110%, at least 120%, at least 150%, at least 200%, at least 500%, at least 1000% or more in respect to the first sample.

In another particular embodiment, a decrease in the amount of the ICAM-1 protein in the second sample with respect to the amount of the ICAM-1 protein in the first sample is indicative of an unfavourable response to said treatment. It is considered that the amount of the ICAM-1 protein in the second sample is decreased in respect to the amount of the ICAM-1 protein in the first sample when it decreases in at least 1%, at least 2%, at least 3%, at least 4%, at least 5%, at least 10%, at least 15%, at least 20%, at least 25%, at least 30%, at least 40%, at least 50%, at least 60%, at least 70%, at least 80%, at least 90%, at least 100%, at least 110%, at least 120%, at least 150%, at least 200%, at least 500%, at least 1000% or more in respect to the first sample. Likewise, an increase in the amount of the ICAM-1 protein in the second sample with respect to the amount of the ICAM-1 protein in the first sample is indicative of a favourable response to said treatment. It is considered that the amount of the ICAM-1 protein in the second sample is increased in respect to the amount of the ICAM-1 protein in the first sample when it increases in at least 1%, at least 2%, at least 3%, at least 4%, at least 5%, at least 10%, at least 15%, at least 20%, at least 25%, at least 30%, at least 40%, at least 50%, at least 60%, at least 70%, at least 80%, at least 90%, at least 100%, at least 110%, at least 120%, at least 150%, at least 200%, at least 500%, at least 1000% or more in respect to the first sample.

The term "response to a treatment", as used herein, refers to the evolution of a disease or condition associated to the ICAM-1 protein expression that is being treated, and it may be a favourable response or an unfavourable response. Generally, a favourable response to treatment involves a partial or complete amelioration or disappearance of the symptoms and/or lesions caused by said disease or condition, whereas an unfavourable response to treatment involves an aggravation or deterioration of the symptoms and/or lesions caused by said disease or condition. There are various ways of evaluating the response to treatment of a disease or condition associated to the ICAM-1 protein expression, which are conventional and well-known by the person skilled in the art. By way of illustrative, non-limiting example, when the disease or condition associated to the ICAM-1 protein expression is a type of cancer, in particular a solid tumour, the response to treatment may be evaluated as:
- Complete Response (CR): Disappearance of all target lesions;
- Partial Response (PR): At least a 30% decrease in the sum of the longest diameter (LD) of target lesions, taking as reference the baseline sum of the LD;
- Stable Disease (SD): Neither sufficient shrinkage to qualify for PR nor sufficient increase to qualify for PD, taking as reference the smallest sum LD since the treatment started; or
- Progressive Disease (PD): At least a 20% increase in the sum of the LD of target lesions, taking as reference the smallest sum LD recorded since the treatment started or the appearance of one or more new lesions;
wherein CR and PR are favourable responses to treatment, and PD is an unfavourable response to treatment.

All the terms and embodiments previously described are equally applicable to this aspect of the invention.

### Methods for detecting and/or quantifying the presence of the ICAM-1 protein, or cells expressing the ICAM-1 protein, in a sample

In a twelfth aspect, the invention relates to an *in vitro* method for detecting and/or quantifying the presence of the ICAM-1 protein, or cells expressing the ICAM-1 protein, in a sample, hereinafter referred to as the "third method of the invention", comprising:
(i) contacting the test sample with the antibody of the invention or the carrier of the invention; and
(ii) detecting, localizing and/or quantifying the formation of immune complexes with said antibody of the invention or said carrier.

The terms "antibody", "ICAM-1", "carrier", "sample", and their particulars have been described in detail in the context of the antibody of the invention, the carrier of the invention and in the first method of the invention and are used with the same meaning in the context of the third method of the invention.

The term "test sample", as used herein, refers to the sample in which the detection, localization and/or quantification of the presence of the ICAM-1 protein, or cells expressing the ICAM-1 protein, is performed.

The term "immune complex" or "antigen-antibody complex", as used herein, describes the result from the interaction between an epitope (antigen) with an antibody directed against this epitope, i.e. the interaction between the antigen with the antibody of the invention or the carrier of the invention.

One can use any of a wide variety of formats of immunochemical analysis using the method of the present invention. Analysis such immunochemical techniques include, without limitation, ELISA, immunoassay strip or LFIA, immunosensors, immunoaffinity extraction systems, immunoprecipitation, Western blot, dot blot, radioimmunoassay, immunofluorescence, immunogold labelling for electron microscopy, flow cytometry, immunocytochemistry and immunohistochemistry.

All the terms and embodiments previously described are equally applicable to this aspect of the invention.

### Pharmaceutical compositions

In a thirteenth aspect, the invention relates to a pharmaceutical composition, hereinafter referred to as the "pharmaceutical composition of the invention", comprising the antibody of the invention or the carrier of the invention, and a pharmaceutically acceptable excipient. In an embodiment, the pharmaceutical composition of the invention comprises a therapeutically effective amount of the antibody of the invention, or a pharmaceutically derivative or prodrug thereof, together with a pharmaceutically acceptable excipient, carrier, adjuvant, or vehicle, for administration to a subject.

The terms "antibody", "ICAM-1", "carrier", "subject", "disease or condition associated to the ICAM-1 protein expression" and their particulars have been described in detail in the context of the antibody of the invention, the carrier of the invention and in the first method of the invention and are used with the same meaning in the context of the pharmaceutical composition of the invention.

The term "pharmaceutical composition", as used herein, relates to a composition comprising a therapeutically effective amount of at least the antibody of the invention or the carrier of the invention together with at least one pharmaceutically acceptable excipient. Pharmaceutical compositions according to the invention can be prepared, for instance, as injectables such as liquid solutions, suspensions, and emulsions.

The terms "pharmaceutically acceptable vehicle", "pharmaceutically acceptable diluent" or "pharmaceutically acceptable excipient", used interchangeably herein, is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Acceptable excipients or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN^{™}, PLURONICS^{™} or polyethylene glycol (PEG). Suitable pharmaceutical vehicles are described in "Remington's Pharmaceutical Sciences" by E.W. Martin, 21st Edition, 2005.

The term "therapeutically effective amount", as used herein, in relation to the antibody of the invention or the carrier of the invention, or in relation to the excipient comprised by the pharmaceutical composition of the invention, relates to the sufficient amount of said antibody, carrier and/or excipient to provide the desired effect, i.e. to achieve an appreciable prevention, cure, delay, reduction of severity of, or amelioration of one or more symptoms derived from a disease or condition associated to the ICAM-1 protein expression, and will generally be determined by, among other causes, the characteristics of the agent itself and the therapeutic effect to be achieved. It will also depend on the subject to be treated, the severity of the disease suffered by said subject, the chosen dosage form, etc. For this reason, the doses that may be mentioned in this invention must be considered only as guides for the person skilled in the art, who must adjust the doses depending on the aforementioned variables. In an embodiment, the effective amount produces the amelioration of one or more symptoms of the disease that is being treated.

The antibodies of the invention may be in the same formulation or may be administered in different formulations. Administration can be concurrent or sequential, and may be effective in either order.

Supplementary active compounds can also be incorporated into the pharmaceutical composition of the invention. Thus, in an embodiment, the pharmaceutical composition of the invention further comprises at least an agent. In a more particular embodiment, the pharmaceutical composition of the invention comprises an agent selected from the group consisting of therapeutic agent, diagnostic agent, tracer agent, reporter, marker, cell, cell fragment, cell part, virus, viral fragment, viral part, conjugate and fusion protein. In a preferred embodiment of the pharmaceutical composition of the invention, the agent is a therapeutic agent. The terms "agent", "therapeutic agent", "diagnostic agent", "tracer agent", "reporter", "marker", "cell", "cell fragment", "cell part", "virus", "viral fragment", "viral part", "conjugate" and "fusion protein" and their particulars have been described in the context of the carrier of the invention and are used with the same meaning in the context of the pharmaceutical composition of the invention.

In a particular embodiment, the pharmaceutical composition of the invention may also contain more than one therapeutic agent as necessary for the particular indication being treated, preferably those with complementary activities that do not adversely affect each other. For example, it may be desirable to further provide a therapeutic agent such as, but not limited to, a chemotherapeutic agent, a cytokine, an analgesic agent, an anti-inflammatory agent, a antibiotic agent, an antiviral agent, an antithrombotic agent, a hormone, a vitamin, a cofactor, a steroid, a thrombolytic agent, a recombinant enzyme, another antibody, a gene therapy agent, a gene silencing agent, a gene editing agent, an immunosuppressive agent, a cytotoxin, or a radionuclide. The effective amount of such other active agents depends, among other things, on the amount of antibody of the invention present in the pharmaceutical composition, the type of disease or disorder or treatment, etc.

In another embodiment, the pharmaceutical composition of the invention may also contain one or more than one therapeutic agent and at least a non-therapeutic agent. Non-limitative examples of therapeutic agents have been previously described. Non-limitative examples of non-therapeutic agents include a tracer agent, a reporter or a marker. In an embodiment, the pharmaceutical composition of the invention comprising one or more than one therapeutic agent and at least a non-therapeutic agent may be used for a combination of therapy and diagnostic, or for concurrent therapy and prognosis or determination of the effect of the therapy. In a particular embodiment, said combination approach is known in the prior art as a theranostic approach.

The antibody of the invention may be on the same formulation as the therapeutic agent(s) or may be administered in different formulations. Administration can be concurrent or sequential, and may be effective in either order.

In an embodiment, the antibody of the invention is prepared with excipients that will protect said compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. These can be prepared according to methods known to those skilled in the art, for example, as described in U.S. 4,522, 811.

The administration route of the antibody (or fragment thereof) of the invention may be intratumoural or parenteral.

The term "parenteral" as used herein includes intravenous, intraperitoneal, intramuscular, subcutaneous, rectal or vaginal administration. The intravenous form of parenteral administration is generally preferred. The amount of an antibody required for therapeutic or prophylactic effect will, of course, vary with the antibody chosen, the nature and severity of the condition being treated and the patient.

In addition, the antibody may suitably be administered by pulse infusion, e.g., with increasing or declining doses of the antibody. Preferably the dosing is given by injections, most preferably intravenous or subcutaneous injections, depending in part on whether the administration is brief or chronic.

In another embodiment, the pharmaceutical compositions of the invention may be adapted for parenteral administration, such as sterile solutions, suspensions or lyophilized products in the appropriate unit dosage form. Pharmaceutical compositions suitable for injectable use include sterile aqueous solutions (where water soluble) or dispersions and sterile powders for the extemporaneous preparation of sterile injectable solutions or dispersions. For intravenous administration, suitable excipients include physiological saline, bacteriostatic water, CremophorEM (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The excipient can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and/or gelatin.

Sterile injectable solutions can be prepared by incorporating the active compound (e.g., a polypeptide or antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

In a particular embodiment, said pharmaceutical composition is administered via intravenous or intratumoural. Adequate excipients can be used, such as bulking agents, buffering agents or surfactants. The mentioned formulations will be prepared using standard methods such as those described or referred to in the European and US Pharmacopoeias and similar reference texts.

It is especially advantageous to formulate the pharmaceutical compositions, namely, oral or parenteral compositions, in dosage unit form for ease administration and uniformity of dosage. Dosage unit form as used herein refers to physically discrete units suited as unitary dosages for the subject to be treated; each unit containing a predetermined quantity of active compound (antibody of the invention) calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Generally, an effective administered amount of an antibody of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.001 to 1,000 mg/kg body weight/day, preferably about 0.01 to about 100 mg/kg body weight/day, most preferably from about 0.05 to 10 mg/kg body weight/day.

Aside from administration of antibodies to the patient, the present application contemplates administration of antibodies by gene therapy. WO 1996/07321 relates the use of gene therapy to generate intracellular antibodies.

The pharmaceutical compositions can be included in a container, pack, or dispenser together with instructions for administration.

The antibodies, carriers and pharmaceutical compositions of this invention may be used with other drugs to provide a combination therapy. The other drugs may form part of the same composition, or be provided as a separate composition for administration at the same time or at different time.

The antibodies, carriers and pharmaceutical compositions of the present invention will be useful in the treatment of medical conditions, such as diseases and conditions associated to the ICAM-1 protein expression, specially, for treating cancer, an infectious disease, an inflammatory disease, a metabolic disease, a pulmonary disease, a cardiovascular disease, a neurological disease, a genetic disease, and an immune disease.

All the terms and embodiments previously described are equally applicable to this aspect of the invention.

### Uses of the invention

The antibody of the invention, which specifically binds to an epitope of ICAM-1, can be used therapeutically, as well as in immunochemical assays, such as immunofluorescence assays, flow cytometry, Western blots, immunohistochemical assays, immunoprecipitations, or other immunochemical assays known in the art.

Thus, a fourteenth aspect of the present invention relates to the antibody of the invention, the carrier of the invention, or the pharmaceutical composition of the invention, for use in medicine, hereinafter referred to as the "first use of the invention".

The present invention also relates to the use of the antibody of the invention, the carrier of the invention, or the pharmaceutical composition of the invention in the manufacture of a medicament. Alternatively, this aspect may be reformulated as a method of treatment and/or prevention which comprises administering the antibody of the invention, the carrier of the invention, or the pharmaceutical composition of the invention.

In a fifteenth aspect, the invention relates to the antibody of the invention, the carrier of the invention, or the pharmaceutical composition of the invention, for use in the treatment and/or prevention of a disease or condition associated to the ICAM-1 protein expression, hereinafter referred to as the "second use of the invention".

The present invention also relates to the use of the antibody of the invention, the carrier of the invention, or the pharmaceutical composition of the invention in the manufacture of a medicament for treatment of a disease or condition associated to the ICAM-1 protein expression. Alternatively, this aspect may be reformulated as a method of treatment and/or prevention of a disease or condition associated to the ICAM-1 protein expression, which comprises administering the antibody of the invention, the carrier of the invention, or the pharmaceutical composition of the invention to a subject in need thereof.

The terms "antibody", "ICAM-1", "carrier", "subject", "disease or condition associated to the ICAM-1 protein expression", "pharmaceutical composition" and their particulars have been described in detail in the context of the antibody of the invention, carrier of the invention, in the first and second methods of the invention and in the pharmaceutical composition of the invention, and are used with the same meaning in the context of the first and second use of the invention.

In a particular embodiment of the second use of the invention, the disease or condition associated to the ICAM-1 protein expression is selected from the group consisting of cancer, an infectious disease, an inflammatory disease, a metabolic disease, a pulmonary disease, a cardiovascular disease, a neurological disease, a genetic disease, and an immune disease.

Thus, in an embodiment the invention relates to the first method of the invention, the second method of the invention, or the second use of the invention, wherein said disease or condition associated to the ICAM-1 protein expression is selected from the group consisting of cancer, an infectious disease, an inflammatory disease, a metabolic disease, a pulmonary disease, a cardiovascular disease, a neurological disease, a genetic disease, and an immune disease.

The present invention also relates to the use of the antibody of the invention, the carrier of the invention, or the pharmaceutical composition of the invention in the manufacture of a medicament for treatment of a disease or condition associated to the ICAM-1 protein expression, wherein said disease or condition associated to the ICAM-1 protein expression is selected from the group consisting of cancer, an infectious disease, an inflammatory disease, a metabolic disease, a pulmonary disease, a cardiovascular disease, a neurological disease, a genetic disease, and an immune disease. Alternatively, this embodiment may be reformulated as a method of treatment and/or prevention of a disease or condition associated to the ICAM-1 protein expression, wherein said disease or condition associated to the ICAM-1 protein expression is selected from the group consisting of cancer, an infectious disease, an inflammatory disease, a metabolic disease, a pulmonary disease, a cardiovascular disease, a neurological disease, a genetic disease, and an immune disease, which comprises administering the antibody of the invention, the carrier of the invention, or the pharmaceutical composition of the invention to a subject in need thereof.

The terms "cancer", "infectious disease", "inflammatory disease", "metabolic disease", "pulmonary disease", "cardiovascular disease", "neurological disease", "genetic disease" and "immune disease", and their particulars have been described in detail in the context of the first method of the invention, and are used with the same meaning in the context of the second use of the invention.

In a particular embodiment of the second use of the invention:
- the cancer is selected from the group consisting of lung cancer, mesothelioma, brain cancer, breast cancer, pancreatic cancer, colon cancer, ovarian cancer, prostate cancer, head and neck cancer, liver cancer, and leukemia;
- the infectious disease is selected from the group consisting of common cold, COVID-19, herpes, acquired immunodeficiency syndrome (AIDS), influenza, tuberculosis, malaria, and an enteric viral infection;
- the inflammatory disease is selected from the group consisting of asthma, diabetes, lupus, ulcerative colitis, psoriasis, chronic obstructive pulmonary disease, vasculitis, arthritis, hepatitis, systemic sclerosis, gout, myositis, and scleroderma;
- the metabolic disease is selected from the group consisting of glycogen storage disease, hypercholesterolemia, obesity, diabetes, atherosclerosis, lysosomal storage disorder, fatty liver disease, and inborn errors of metabolism;
- the pulmonary disease is selected from the group consisting of asthma, cystic fibrosis, acid sphingomyelinase deficiency, Fabry disease, pneumonia, bronchitis, pulmonary edema, pulmonary fibrosis, interstitial lung disease, pneumothorax, mesothelioma, lung cancers, acute respiratory distress syndrome, chronic obstructive pulmonary disease, emphysema, bronchitis, and pulmonary thrombosis;
- the cardiovascular disease is selected from the group consisting of stroke, aneurysm, hypertension, cardiomyopathy, heart failure, heart attack, coronary artery disease, peripheral arterial disease, angina, heart valve disease, atrial fibrillation, arrhythmia, thrombosis, endocarditis, cerebrovascular disease, and atherosclerosis;
- the neurological disease is selected from the group consisting of Parkinson's disease, Alzheimer's disease, spinal cord injury, amyotrophic lateral sclerosis, ataxia, epilepsy, depression, bipolar disorder, and schizophrenia, and congenital diseases that affect the central nervous system;
- the genetic disease is selected from the group consisting of cystic fibrosis, familiar hypercholesterolemia, lysosomal storage disease, Gaucher disease, Pompe disease, Muccopolysaccharidosis, Niemann-Pick disease, acid sphingomyelinase disease, Fabry disease, Duchenne muscular dystrophy, sickle cell disease, congenital glycosylation disorders, and congenital heart disease; and/or
- the immune disease is selected from the group consisting of inflammatory bowel disease, lupus, asthma, psoriasis, rheumatoid arthritis, multiple sclerosis, Hashimoto thyroiditis, Grave's disease, Type 1 diabetes, vitiligo, host versus graft disease, Crohn's disease, ulcerative colitis, celiac disease, Sjogren's syndrome, Kawasaki disease, and allergy.

The term "treatment", as used herein, refers to any type of therapy, which is aimed at terminating, preventing, ameliorating or reducing the susceptibility to a clinical condition as described herein. In a preferred embodiment, the term treatment relates to prophylactic treatment (i.e. a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter.

The term "prevention", "preventing" or "prevent", as used herein, relates to the administration of the antibody of the invention, the carrier of the invention, or the pharmaceutical composition of the invention or of a medicament comprising said combination to a subject who has not been diagnosed as possibly having the disease or condition, but who would normally be expected to develop said disease or condition, or be at increased risk for said disease or condition. The prevention intends to avoid the appearance of said disease or condition. The prevention may be complete (e.g. the total absence of a disease or condition). The prevention may also be partial, such that for example the occurrence of a disease or condition in a subject is less than that which would have occurred without the administration of the antibody of the invention, the carrier of the invention, or the pharmaceutical composition of the invention. Prevention also refers to reduced susceptibility to a clinical condition.

As the person skilled in the art will recognize, the antibody of the invention may be useful in therapeutic applications in various forms, which include without limitation, in the form of intact antibody, conjugated to another therapeutic agent, and in the form of a fusion protein with another therapeutic agent. These therapeutic applications will comprise the administration of a therapeutically effective amount of the antibody of the invention.

The term "therapeutically effective amount", and their particulars have been described in detail in the context of the pharmaceutical composition of the invention, and are used with the same meaning in the context of the first and second use of the invention.

In a particular embodiment of the first and the second use of the invention, the antibody of the invention is in the form of intact antibody, i.e., in the form of immunoglobulin. The antibody of the invention, when is in the form of immunoglobulin, typically uses a combination of mechanisms in directing cytotoxic effects to an ICAM-1-expressing cell: (i) it interacts with components of the immune system through antibody-dependent cellular cytotoxicity (ADCC) or (ii) through complement-dependent cytotoxicity (CDC).

Antibody-dependent cellular cytotoxicity (ADCC) occurs when antibodies bind to antigens on cells and the antibody Fc domains engage Fc receptors (FcR) on the surface of immune effector cells. Several families of Fc receptors have been identified, and specific cell populations characteristically express defined Fc receptors. For example, neutrophils commonly express human FcγRl (CD64), FcγRII (CD32) and the B (lipid anchored) isoform of FcγRIII (CD16). In contrast, human natural killer (NK) cells express only the A (transmembrane) isoform of CD16. This structure facilitates recruitment of adaptor proteins and activation of NK cells by antibody engagement of CD16.

Complement-dependent cytotoxicity (CDC) is another cell-killing method that can be directed by antibodies. As with ADCC, the different subclasses of antibodies have varying abilities to elicit CDC responses. IgM is the most effective isotype for complement activation, and IgG1 and IgG3 are both very effective at directing CDC via the classical complement activation pathway. In this cascade, the formation of antigen-antibody complexes results in the uncloaking of multiple C1q binding sites in close proximity on the CH2 domains of participating IgG molecules (C1q is one of three subcomponents of complement C1). These uncloaked C1q binding sites convert the previously low-affinity C1q-IgG interaction to one of high avidity, which triggers a cascade of events involving a series of other complement proteins and leads to the proteolytic release of the effector-cell chemotactic/activating agents C3a and C5a. The complement cascade ends in the formation of a membrane attack complex, which creates 100 Å pores in the cell membrane that facilitate free passage of water and solutes into and out of the cell.

In a particular embodiment, the binding of the antibody of the invention to the ICAM-1 may block or decrease or increase the binding of any natural binders of ICAM-1, including but not limited to ICAM-1 itself, since this protein can form dimers or oligomers, and/or LFA-1, Mac-1, fibrinogen- and fibrin-derived gamma 3 peptide, a peptide found in mucin 1 core protein, hyaluronan, major class human Rhinoviruses, A-type coxsackieviruses, *Plasmodium falciparum-*infected erythrocytes displaying PfEMP1, as well as membrane-envelopped viruses, extracellular vesicles, and cancer or metastatic cells that can either bind to ICAM-1 through the presence of ICAM-1 ligands on their surface or, *vice versa,* contain ICAM-1 molecules of their membranes with which they can bind to ICAM-1 ligands.

In another particular embodiment of the first and second use of the invention, the antibody of the invention or the carrier of the invention is conjugated to or in the form of a fusion protein with another therapeutic agent.

Suitable therapeutic agents for forming immunoconjugates of the present invention include, but are not limited to, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, maytansine or an analog or derivative thereof, mitoxantrone, mithramycin, actinomycin D, 1-ehydrotestosterone, glucocorticoids, procaine, tetracaine, lidocaine, propranolol, and puromycin; calicheamicin or analogs or derivatives thereof; antimetabolites (such as methotrexate, 6-mercaptopurine, 6-thioguanine, cytarabine, fludarabin, 5-fluorouracil, decarbazine, hydroxyurea, asparaginase, gemcitabine, cladribine), alkylating agents (such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin; as well as duocarmycin A, duocarmycin SA, CC-1065 (a .k.a. rachelmycin), or analogs or derivatives of CC- 1065), antibiotics (such as dactinomycin (formerly actinomycin), bleomycin, daunorubicin (formerly daunomycin), doxorubicin, idarubicin, mithramycin, mitomycin, mitoxantrone, plicamycin, anthramycin (AMC)), anti-mitotic agents (e.g ., tubulin-inhibitors) such as monomethyl auristatin E, monomethyl auristatin F, or other analogs or derivatives of dolastatin 10; diphtheria toxin and related molecules (such as diphtheria A chain and active fragments thereof and hybrid molecules); ricin toxin (such as ricin A or a deglycosylated ricin A chain toxin), cholera toxin, a Shiga-like toxin (SLT-I, SLT-II, SLT-IIV), LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, and enomycin toxins. Other suitable conjugated molecules include antimicrobial/lytic peptides such as CLIP, Magainin 2, mellitin, Cecropin, and P18; ribonuclease (RNase), DNase I, Staphylococcal enterotoxin-A, pokeweed antiviral protein, diphtherin toxin, and Pseudomonas endotoxin.

In a preferred embodiment of the first and second use of the invention, the antibody of the invention or the carrier of the invention is conjugated to or in the form of a fusion protein with another therapeutic agent, selected from the group consisting in, but not limited to, a chemotherapeutic agent, a cytokine, an analgesic agent, an anti-inflammatory agent, a antibiotic agent, an antiviral agent, an antithrombotic agent, a hormone, a vitamin, a cofactor, a steroid, a thrombolytic agent, a recombinant enzyme, another antibody, a gene therapy agent, a gene silencing agent, a gene editing agent, a cytotoxin, a radionuclide or an immunosuppressive agent.

Cytotoxic chemotherapy or radiotherapy of cancer is limited by serious, sometimes life threatening, side effects that arise from toxicities to sensitive normal cells because the therapies are not selective for malignant cells. One strategy to avoid these problems is to couple the therapeutic agent to antibodies or other ligands that recognize tumour-associated antigens. This increases the exposure of the malignant cells, and reduces the exposure of normal cells, to the ligand-targeted therapeutics.

The therapeutic agent can be a radionuclide that acts releasing a cytotoxic radiation to the cell expressing the ICAM-1 protein. When conjugated to the antibody of the invention or to the carrier of the invention, the resulting molecule is useful as a radioimmunotherapeutic agent.

In one preferred embodiment of the first and second use of the invention, the antibody of the invention or the carrier of the invention is conjugated to a radionuclide. Illustrative examples that are useful in the context of the present invention include, without limitation, beta emitters, such as ¹³¹I, ⁹⁰Y, ^{99m}Tc, ¹⁷⁷Lu, and ⁶⁷Cu, and alpha emitters, such as ²¹³Bi and ²¹¹At.

The therapeutic agent can be an immunosuppressive agent, i.e., a substance that acts to suppress or mask the immune system of the mammal being treated herein. This would include substances that suppress cytokine production, down-regulate or suppress self-antigen expression, or mask the MHC antigens.

The therapeutic agent can also be a cytotoxic agent, i.e., a substance that inhibits or prevents the function of cells and/or causes destruction of cells. The term is intended to include radioactive isotopes (as described above), chemotherapeutic agents, i.e., chemical compounds useful in the treatment of cancer, and toxins such as small molecule toxins or enzymatically active toxins of bacterial, fungal, plant or animal origin, or fragments thereof.

The therapeutic agent can also be a cytokine, a hormone, growth factor, necrosis factor, i.e., a protein or peptide released by one cell population which act on another cell as intercellular mediators or even in the same cell population. As used herein, the term cytokine includes proteins and peptides from natural sources or from recombinant cell culture and biologically active equivalents of the native sequence cytokines.

In one preferred embodiment of the first and second use of the invention, the antibody of the invention or the carrier of the invention is conjugated to one or more toxin molecules. Enzymatically active toxins and fragments thereof which can be used include diphtheria A chain, nonbinding active fragments of diphtheria toxin, exotoxin A chain (from *Pseudomonas aeruginosa*)*,* ricin A chain, abrin A chain, modeccin A chain, alpha sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes.

The present invention further contemplates the antibody of the invention or the carrier of the invention to be conjugated with a compound with nucleolytic activity (e.g. a ribonuclease or a DNA endonuclease such as a deoxyribonuclease; DNase) or other compound capable of damaging a cellular structure or organelle and therefore killing or diminishing the vitality of the cell.

Conjugates of the antibody of the invention or of the carrier of the invention and cytotoxic agent may be made using a variety of bifunctional protein coupling agents or linkers. The linker may be a "cleavable linker" facilitating release of the cytotoxic drug in the cell. For example, an acid-labile linker, peptidase-sensitive linker, dimethyl linker or disulfide-containing linker may be used.

Alternatively, a fusion protein comprising the antibody of the invention or the carrier of the invention and cytotoxic agent may be made, e.g. by recombinant techniques or peptide synthesis.

The therapeutic agent can also be a prodrug which refers to a precursor or derivative form of a pharmaceutically active substance that is less cytotoxic to tumour cells compared to the parent drug and is capable of being, for instance, enzymatically, temperature, light or pH dependent activated or converted into the more active parent form.

In one preferred embodiment of the first and second use of the invention, the antibody of the invention or the carrier of the invention may also be conjugated with a prodrug activating agent which converts a prodrug to an active anti-cancer drug. The agent component of such conjugates includes any agent capable of acting on a prodrug in such a way so as to convert it into its more active, cytotoxic form. This is particularly useful in antibody-directed enzyme prodrug therapy approaches (ADEPT).

Enzymes useful in the present invention include any enzyme that can convert a prodrug into an active therapeutic agent (drug). In a specific embodiment, the enzyme is an enzyme from a mammal, for example, a human enzyme. In another specific embodiment, the enzyme is an enzyme from an organism other than a mammal; in this last embodiment, the immunogenicity of the enzyme is optionally reduced, for example, by conjugation to polyethylene glycol (PEG) and the like. In accordance with a particular embodiment, neither the enzyme nor an enzyme with similar substrate specificity is endogenous to the subject along the route of administration or biodistribution of the prodrug. In a particular embodiment, enzymes useful in the present invention are lysosomal enzymes (such as, but not limited to alpha-galactosidase, acid sphingomyelinase, hyaluronidase, glucosidase-alpha, beta-glucocerebrosidase, etc.). Thrombolytic enzymes (such as, but not limited to heparin) and antioxidant enzymes (such as but not limited to catalase, superoxide dismutase, etc.).

Proteases, glycosidases, esterases and the like are general types of enzymes that can be used in accordance with the present invention. Specific examples of suitable enzymes include, but are not limited to, glycosidases (beta-glucuronidase, beta-glucosidase, beta-galactosidase), beta-lactamase, cellulase, dextranase, fructase, aminopeptidase, lysozyme, cytosine deaminase, carboxypeptidase, penicillin amidase, methionine γ liase, and carboxyesterase. The enzyme is selected for its ability to convert the selected prodrug into its active drug form. For example, if the prodrug comprises a conjugate of dextran and a therapeutic agent, an appropriate enzyme would be dextranase. Similarly, cellulase could be used with a prodrug comprising a cellulose substrate, and glucuronidase could be used with a prodrug comprising a glucuronide.

In a preferred embodiment, the enzyme is selected from the group consisting of glycosidases (glucuronidase, beta-glucosidase, beta-galactosidase), beta-lactamase, cellulase, dextranase, fructase, aminopeptidase, lysozyme, cytosine deaminase, carboxypeptidase, penicillin amidase, methionine γ liase, and carboxyesterase, or a functional variant or fragment thereof having catalytic activity. More preferably, the enzyme is a glycosidase (e.g. beta-galactosidase) or a cytosine deaminase.

Drugs useful in the context of this invention include toxins, antibiotic or chemotherapeutic drugs, radioisotopes, paramagnetic ions, boron addends, cytokines, photosensitizers, radiosensitizers, vasodilators, immunomodulator agents, immune-suppressive agents, (gluco)corticoids, analgesic agents, anti-inflammatory agents, antiviral agents, antithrombotic agents, hormones, vitamins, cofactors, steroids, thrombolytic agents, recombinant enzymes, another antibody, gene therapy agents, gene silencing agents, gene editing agents, cytotoxins, radionuclides, etc.

In a particular embodiment, the drug is a chemotherapeutic drug, such as a drug having a cytotoxic activity. The cytotoxic activity of a drug or a prodrug can be performed by means of assays that are well known in the art. For example, assessing cell membrane integrity is one of the most common ways to measure cell viability and cytotoxic effects, since compounds that have cytotoxic effects often compromise cell membrane integrity. Vital dyes, such as trypan blue or propidium iodide are normally excluded from the inside of healthy cells; however, if the cell membrane has been compromised, they freely cross the membrane and stain intracellular components. Alternatively, membrane integrity can be assessed by monitoring the passage of substances that are normally sequestered inside cells to the outside. One commonly measured molecule is lactate dehydrogenase (LDH).

Cytotoxicity can also be monitored using the MTT assay. This assay measures the reducing potential of the cell using a colorimetric reaction. Viable cells will reduce the dimethyl thiazolyl diphenyl tetrazolium salt (MTT) reagent to a coloured formazan product. A similar redox-based assay has also been developed using the fluorescent dye, resazurin. In addition to using dyes to indicate the redox potential of cells in order to monitor their viability, assays that use ATP content as a marker of viability can also be used. Such ATP-based assays include bioluminescent assays in which ATP is the limiting reagent for the luciferase reaction. Cytotoxicity can also be measured by the sulforhodamine B (SRB) assay, water soluble tetrazolium salt (WST) assay and clonogenic assay. A label-free approach to follow the cytotoxic response of adherent animal cells in real-time is based on electric impedance measurements when the cells are grown on gold-film electrodes. This technology is referred to as electric cell-substrate impedance sensing (ECIS).

In another particular embodiment, the drug is more cytotoxic than its corresponding prodrug from which the drug is released. The cytotoxicity of a compound is typically expressed as its IC₅₀ value. The IC₅₀ value, as used herein, refers to the half maximal inhibitory concentration and represents the concentration of a compound required for obtaining 50% of a maximum effect *in vivo.* In a particular preferred embodiment, the active drug is more cytotoxic than its corresponding prodrug. This can be estimated with the QIC₅₀ value, which is the ratio of the IC₅₀ of the prodrug / IC₅₀ of the drug. The QIC₅₀ value can be 5 or higher than 5, for example, 10 or higher than 10, 10² or higher than 10², 10³ or higher than 10³, 10⁴ or higher than 10⁴, or even higher. Preferably, the QIC₅₀ is higher than 10², and more preferably, higher than 10³.

Illustrative, non-limitative, examples of prodrugs which can be used in the context of the present invention include glycosidic prodrugs, for example, duocarmycin-derived galactosyl prodrugs, N-(beta-D galactopyranosyloxycarbonyl)-doxorubicin and N-[4-(beta-D-galactopyranosyl)-3-nitrobenzyloxycarbonyl]daunomycin; 5-fluorocytosine; cephalosporin prodrugs, such as PROTAX (cephalosporin derivative of taxol), C-DOX (cephalosporin derivative of doxorubicin), CCM (cephalosporin mustard prodrug), etc.; palytoxin prodrug NHPAP, doxorubicin prodrug DPO, combretastatin prodrugs such as combretastatin A-4 prodrug (CA-4PD), bisphosphonate prodrugs like bisphosphonamidate clodronate etc. Prodrugs can usually be prepared using well-known methods, such as those described by Burger "Medicinal Chemistry and Drug Discovery 6th ed. (Donald J. Abraham ed., 2001, Wiley) and "Design and Applications of Prodrugs" (H. Bundgaard ed., 1985, Harwood Academic Publishers).

Alternatively, fusion proteins comprising at least the antibody of the invention or at least the carrier of the invention linked to at least a functionally active portion of an enzyme of the invention can be constructed using recombinant DNA techniques well known in the art.

Although human, partially human, or humanised antibodies will be suitable for many applications, particularly those involving uses of the antibody in the preparation of a medicament for treatment in a human subject, other types of antibodies, i.e., of different origin, will be suitable for certain applications. The non-human antibodies of the invention can be, for example, derived from any antibody-producing animal, such as mouse, rat, rabbit, goat, donkey, or non-human primate such as monkey (e.g., cynomologous or rhesus monkey) or ape (e.g., chimpanzee). Non-human antibodies of the invention can be used, for example, in *in vitro* and cell-culture based applications, or any other application where an immune response to the antibody of the invention does not occur, is insignificant, can be prevented, is not a concern, or is desired.

In a sixteenth aspect, the invention relates to the antibody of the invention, the carrier of the invention, or the pharmaceutical composition of the invention, for use in a method of diagnosis and/or prognosis *in vivo* of a disease or condition associated to the ICAM-1 protein expression. Alternatively, this aspect may be reformulated as a method of diagnosis and/or prognosis *in vivo* of a disease or condition associated to the ICAM-1 protein expression, which comprises administering the antibody of the invention, the carrier of the invention, or the pharmaceutical composition of the invention to a subject in need thereof.

In an embodiment, the invention relates to the use of the antibody of the invention, the carrier of the invention, or the pharmaceutical composition of the invention in the *in vivo*/*ex vivo* diagnosis of a disease or condition associated to the ICAM-1 protein expression. Alternatively, this embodiment may be reformulated a method for the *in vivo*/*ex vivo* diagnosis of a disease or condition associated to the ICAM-1 protein expression using the antibody of the invention, the carrier of the invention, or the pharmaceutical composition of the invention. In another embodiment, the invention relates to the use of the antibody of the invention, the carrier of the invention, or the pharmaceutical composition of the invention in the *in vivo*/*ex vivo* diagnosis of a disease or condition by using imaging techniques wherein said disease or condition is associated to the ICAM-1 protein expression. Alternatively, this embodiment may be expressed as the use of the antibody of the invention, the carrier of the invention in the manufacture of a composition for *in vivo* diagnosis of a disease or condition by using imaging techniques wherein said disease or condition is associated to ICAM-1 expression. For this application, the antibody of the invention or the carrier of the invention will be preferably administered in the pharmaceutical composition of the invention, whose particulars have been described elsewhere in the present specification and which are incorporated herein by reference. In a particular embodiment, the antibody of the invention is a monoclonal antibody, such as, for example, the antibodies identified as B4 and B6 antibodies in the examples. In another particular embodiment, the imaging techniques are techniques used in nuclear medicine. These techniques include, without limitation, scintigraphy, SPECT, and PET. The person skilled in the art will understand that for performing these techniques it is necessary that the antibody, the carrier, or the pharmaceutical composition of the invention is labelled with a suitable radionuclide. Suitable radionuclides that can be used in this aspect have been described elsewhere in the present specification and which are incorporated herein by reference. In another particular embodiment, said disease or condition is selected from the group consisting of cancer, an infectious disease, an inflammatory disease, a metabolic disease, a pulmonary disease, a cardiovascular disease, a neurological disease, a genetic disease, and an immune disease.

In seventeenth aspect, the invention relates to the use of the antibody of the invention, the carrier of the invention, or the pharmaceutical composition of the invention, for targeting a drug to a specific site wherein said specific site comprises the presence of the ICAM-1 protein or said specific site comprises cells expressing ICAM-1 (ICAM-1⁺ cells). Alternatively, this aspect may be reformulated as a method of targeting a drug to a specific site wherein said specific site comprises the presence of the ICAM-1 protein or said specific site comprises cells expressing ICAM-1 (ICAM-1⁺cells), which comprises administering the antibody of the invention, the carrier of the invention, or the pharmaceutical composition of the invention to a subject in need thereof. Alternatively, this aspect may be reformulated as the use of the antibody of the invention, the carrier of the invention or the pharmaceutical composition of the invention in the manufacture of a composition for targeting a drug to a specific site wherein said specific site comprises the presence of the ICAM-1 protein or said specific site comprises cells expressing ICAM-1 (ICAM-1⁺ cells). The antibody of the invention or the carrier of the invention can be thus considered to be a vehicle, such as a nano-vehicle, to the specific site. For this application, the antibody of the invention or the carrier of the invention will be preferably administered in the pharmaceutical composition of the invention, whose particulars have been described elsewhere in the present specification and which are incorporated herein by reference. In a particular embodiment, the antibody of the invention is a monoclonal antibody, such as, for example, the antibodies identified as B4 and B6 antibodies in the examples. In another particular embodiment, the drug is selected from the group consisting of toxins, antibiotic or chemotherapeutic drugs, radioisotopes, paramagnetic ions, boron addends, cytokines, photosensitizers, radiosensitizers, vasodilators, immunomodulator agents, immune-suppressive agents, and (gluco)corticoids, analgesic agents, anti-inflammatory agents, antiviral agents, antithrombotic agents, hormones, vitamins, cofactors, steroids, thrombolytic agents, recombinant enzymes, another antibody, gene therapy agents, gene silencing agents, gene editing agents, cytotoxins, and radionuclides, whose particulars have been described above and which are incorporated herein by reference. In another particular embodiment, said specific site is a specific site of a disease or condition selected from the group consisting of cancer, an infectious disease, an inflammatory disease, a metabolic disease, a pulmonary disease, a cardiovascular disease, a neurological disease, a genetic disease, and an immune disease.

In an embodiment, the invention relates to the antibody of the invention, the carrier of the invention, or the pharmaceutical composition of the invention for killing target ICAM-1-expressing cells, or for use in a method of treatment of a disease or condition, wherein said method comprises killing target cells, i.e., cells expressing the ICAM-1 protein (ICAM-1⁺), or, alternatively expressed, according to this aspect, the invention relates to the use of the antibody of the invention, the carrier of the invention, or the pharmaceutical composition of the invention in the manufacture of a medicament for the treatment of a disease, wherein said treatment comprises killing the target cells, i.e., cells expressing the ICAM-1 protein (ICAM-1⁺). For this application, the antibody of the invention or the carrier of the invention will be preferably administered in the pharmaceutical composition of the invention, whose particulars have been described elsewhere in the present specification and which are incorporated herein by reference. In a particular embodiment, the antibody of the invention is a monoclonal antibody, such as, for example, the antibodies identified as B4 and B6 antibodies in the examples. In another particular embodiment, the target cells are tumour cells expressing the ICAM-1 protein or non-tumour cells expressing the ICAM-1 protein, comprised in a disease or condition wherein the expression and/or amount and/or distribution of the ICAM-1 protein in said cells is altered. In another particular embodiment, said disease or condition is selected from the group consisting of cancer, an infectious disease, an inflammatory disease, a metabolic disease, a pulmonary disease, a cardiovascular disease, a neurological disease, a genetic disease, and an immune disease.

In another aspect, the invention relates to the antibody of the invention, the carrier of the invention or the pharmaceutical composition of the invention, for use in the treatment of a disease or condition associated to the ICAM-1 expression by depleting cells expressing ICAM-1 or depleting ICAM-1 itself in said disease or condition; or, alternatively expressed, according to this aspect, the invention relates to the use of the antibody of the invention, the carrier of the invention in the manufacture of a pharmaceutical composition for treating a disease or condition associated to the ICAM-1 expression by depleting cells expressing ICAM-1 or depleting ICAM-1 itself in said disease or condition. The antibody of the invention or the carrier of the invention can be thus considered to be a vehicle, such as a nano-vehicle, to the disease or condition associated to the ICAM-1 expression. In an embodiment, the antibody of the invention or the carrier of the invention depletes cells expressing the ICAM-1 protein or depletes ICAM-1 itself in a disease or condition associated to the ICAM-1 expression. For this application, the antibody of the invention or carrier of the invention will be preferably administered in the pharmaceutical composition of the invention, whose particulars have been described elsewhere in the present specification and which are incorporated herein by reference. In a particular embodiment, the antibody of the invention is a monoclonal antibody, such as, for example, the antibodies identified as B4 and B6 antibodies in the examples. In another particular embodiment, the drug is selected from the group consisting of toxins, antibiotic or chemotherapeutic drugs, radioisotopes, paramagnetic ions, boron addends, cytokines, photosensitizers, radiosensitizers, vasodilators, immunomodulator agents, immunosuppressive agents, and (gluco)corticoids, analgesic agents, anti-inflammatory agents, antiviral agents, antithrombotic agents, hormones, vitamins, cofactors, steroids, thrombolytic agents, recombinant enzymes, another antibody, gene therapy agents, gene silencing agents, gene editing agents, cytotoxins, and radionuclides, whose particulars have been described above and which are incorporated herein by reference. In another particular embodiment, said disease or condition is selected from the group consisting of cancer, an infectious disease, an inflammatory disease, a metabolic disease, a pulmonary disease, a cardiovascular disease, a neurological disease, a genetic disease, and an immune disease.

In an eighteenth aspect, the invention relates to the use of the antibody of the invention or the carrier of the invention, as a tool in biotechnology techniques for detection, localization and/or quantification of the ICAM-1 protein in a sample, or in the detection, localization and/or quantification of the ICAM-1 protein, or cells expressing the ICAM-1 protein, present in a sample, hereinafter referred to as the "third use of the invention".

Thus, the antibody of the invention or the carrier of the invention can also be labelled with a detectable label or labelling agent that allows its location and/or identification by spectroscopic, photochemical, biochemical, immunochemical or chemical means. Thus, in a particular embodiment, the antibody of the invention or the carrier of the invention comprises a labelling agent.

The term "detectable label" or "labelling agent", as used herein, refers to a molecular label which allows the detection, localization and/or identification of the molecule to which it is attached, using suitable procedures and equipment for detection, either by spectroscopic, photochemical, biochemical, immunochemical or chemical means.

Thus, in a particular embodiment, the antibody of the invention or the carrier of the invention is modified so that subsequent detection possible. Thus, the invention contemplates the possibility of modifying the antibody of the invention or the carrier of the invention with a detectable agent that permits detection of the antibody. Non-limitative examples of detectable agents include radioisotopes and fluorescent groups.

In a particular embodiment, the antibody of the invention or the carrier of the invention is modified with a radioisotope. Non-limitative examples of radioisotopes suitable for the invention include ³H, ¹¹C, ¹⁴C, ¹⁸F, ³²P, ³⁵S, ⁶⁴Cu, ⁶⁸Ga, ⁸⁶Y, ⁹⁹Tc, ¹¹¹In, ¹²³I, ¹²⁴I, ¹²⁵I, ¹³¹I, ¹³³Xe, ¹⁷⁷Lu, ²¹¹At and ²¹³B. Radioisotope labelling is performed typically by using chelating ligands that are capable of complexing metal ions such as DOTA, DOTP, DOTMA, DTPA and TETA. Methods for conjugating radioisotopes to proteins are well known in the prior art.

In another particular embodiment, the antibody of the invention or the carrier of the invention is labelled with a fluorescent group. The fluorescent group can be attached to the side chains of the amino acids directly or through a linking group. Methods for conjugating polypeptides fluorescent reagents are well known in the prior art.

Suitable reagents for labelling polypeptides, such as antibodies, with fluorescent groups include chemical groups which show ability to react with the various groups listed in the side chains of the proteins, including amino groups and thiol groups. Thus, chemical groups that can be used to modify the antibodies according to the present invention include, without limitation, maleimide, haloacetyl, iodoacetamide succinimidyl ester (e.g. NHS, N-hydroxysuccinimide), isothiocyanate, sulfonyl chloride, 2,6-dichlorotriazinyl, pentafluorophenyl ester, phosphoramidite and the like. An example of suitable reactive functional group is N -hydroxysuccinimide ester (NHS) of a detectable group modified with a carboxyl group. Typically , the carboxyl group modifying the fluorescent compound is activated by the contacting of said compound with a carbodiimide reagent (for example, dicyclohexylcarbodiimide, diisopropylcarbodiimide, uronium or a reagent such as TSTU (O-(N-Succinimidyl)-N,N,N',N'-tetramethyluronium tetrafluoroborate), HBTU((O-benzotriazol-1-yl)-N,N,N',N'-tetramethyluronium hexafluorophosphate), or HATU (O-(7-azabenzotriazol-1-yl)-N,N,N',N'-tetramethyluronium hexafluorophosphate), an activator of the type of 1-hydroxybenzotriazole (HOBt) and N-hydroxysuccinimide to give the NHS ester of the label.

Fluorescent compounds suitable for use in the fourth use of the invention include, without limitation, ethidium bromide, SYBR Green, fluorescein isothiocyanate (FITC), rhodamine tetramethyl isotiol (TRIT), 5-carboxyfluorescein, 6-carboxyfluorescein, fluorescein, HEX (6-carboxy-2',4,4',5',7,7'-hexachlorofluorescein), Oregon Green 488, Oregon Green 500, Oregon Green 514, Joe (6-carboxy-4',5'-dichloro-2',7'-dimethoxyfluorescein), 5-carboxy-2',4',5',7'-tetrachlorofluorescein,5-carboxyrhodamine, rhodamine, tetramethylrhodamine (Tamra), Rox (carboxy-X-rhodamine), R6G (rhodamine 6G), phthalocyanines, azometazinas, cyanines (Cy2,Cy3 and Cy5), Texas Red, Princeston Red, BODIPY FL-Br2, BODIPY 530/550, BODIPY TMR, BODIPY 558/568, BODIPY 564/570, BODIPY 576/589, BODIPY 581/591, BODIPY TR, BODIPY 630/650, BODIPY 650/665, DABCYL, eosin, erythrosin, ethidium bromide, green fluorescent protein (GFP) and its analogues, inorganic-based fluorescent semiconductor nanocrystals (Quantum Dot), fluorescent labels based onlanthanide such as Eu³⁺ and Sm³⁺ and the like.

In another particular embodiment, the antibody of the invention or the carrier of the invention is labelled by conjugation to a first member of a binding pair. In a preferred embodiment, this modification is covalent biotinylation. The term "biotinylation", as used herein, refers to the covalent attachment of biotin to a molecule (typically a protein). Biotinylation is performed using biotin reagents capable of conjugating to the side chain of the proteins, wherein said conjugation occurs primarily on the primary amino groups and thiol groups contained in the side chains of proteins. Suitable reagents for biotinylation of amino groups include molecules containing biotin and a group capable of reacting with amino groups such as succinimide esters, pentafluorophenyl ester or alkyl halides, wherein the biotin moiety and the reactive group separated by a spacer of any length (for example, 8-40 A in length). Some examples of these agents include agents biotinylation NHS -biotin (containing an ester bond of five carbon atoms between biotin and NHS group), sulfo-NHS-biotin, NHS-LC-biotin, sulfo-NHS-LC-biotin, NHS-LC-LC-biotin, sulfo-NHS-LC-LC-biotin, sulfo-NHS-SS-biotin, NHS-PEO4-biotin, PFP-biotin, TFP-PEO-biotin and the like, where "NHS" indicates an N-hydroxysuccinimide, "LC" refers to an amide bond of 6 carbon atoms located between NHS and biotin group, "PEO" refers to a etileneoxyde group, where the subscript indicates the number of units PEO, "PFP" refers to a pentafluorophenyl group "TFP" refers to a tetrafluorophenyl group, "sulfo" refers to a sulfonate group (SOS) and "SS" refers to a disulfide group. Examples of biotinylation reagents with thiol groups include molecules comprising a group of biotin and maleimide or alkyl halide type, separated by a spacer of any length. Examples of biotinylation reagents include maleimide-PEG-biotin, biotin-BMCC (containing a maleimido group N-terminal and a cyclohexyl group, 2 amide and 9 carbon atom linkers), PEO-iodoacetil biotin, iodoacetil-LC-biotin, biotin-HPDP (containing a pyridyl disulfide) and the like.

In another particular embodiment, the antibody of the invention or the carrier of the invention is labelled with metal ions such as gold (Au) or iron (Fe), including colloidal gold nanoparticles or ferromagnetic nanoparticles, which can be attached directly to the antibody via electrostatic interactions. In another particular embodiment, the colloidal gold nanoparticles or ferromagnetic nanoparticles are pre-coupled to biotin and can be covalently attached to the antibody.

It is another aspect of the invention the use of the antibody of the invention or the carrier of the invention in the detection and/or quantification of the ICAM-1 protein, or cells expressing the ICAM-1 protein, present in a sample. The detection and/or quantification of the ICAM-1 protein, or cells expressing the ICAM-1 protein, present in a sample, by the antibody of the invention, can be carried out *in vitro.* Using the antibody of the invention or the carrier of the invention can be carried with any immunochemical or immunofluorescent assay for the detection, identification and/or quantification of the presence of the ICAM-1 protein, or cells expressing the ICAM-1 protein, in a sample. Examples of immunochemical or immunofluorescent assays include, without limitation, immunosensors, immunoprecipitation, Western blot, dot blot, radioimmunoassay, immunofluorescence, immunocytochemistry, immunohistochemistry, immunogold detection for electron microscopy and flow cytometry.

Using the antibody of the invention or the carrier of the invention for the detection, identification and/or quantification of the presence of the ICAM-1 protein, or cells expressing the ICAM-1 protein, in a sample requires to put into practice the third method of the invention, whose particulars have been described elsewhere in the present specification and which are incorporated herein by reference.

As the person skilled in the art will realise, the detection, identification and/or quantification of the presence of the ICAM-1 protein in a sample enables the use of the antibody of the invention or the carrier of the invention in a number of applications including the detection of ICAM-1⁺ circulating tumour cells, the phenotypic analysis of ICAM-1⁺ cellular subpopulations, in particular, of ICAM-1⁺ stem cells.

The detection of ICAM-1⁺ circulating tumour cells usually involves a step of circulating tumour cell enrichment, which includes a large panel of technologies based on the different properties of circulating tumour cells that distinguish them from the surrounding normal blood cells, including physical properties (size, density, electric charges, deformability) and biological properties (surface protein expression, mostly EpCAM expression, as well as ICAM-1 expression). After enrichment, the circulating tumour cells fraction usually still contains a substantial number of leukocytes, and circulating tumour cells need to be, therefore, identified by a method that can distinguish tumor cells from normal blood cells at the single cell level. Among the protein-based strategies, cells are fluorescently stained for cytokeratins (CK; positive marker), the common leukocyte antigen CD45 (negative marker), and a nuclear dye (DAPI); circulating tumour cells are identified as CK+/CD45-/DAPI+ cells. The sample enriched in circulating tumour cells can be then tested for the presence of ICAM-1⁺ cells through a number of different assays, e.g. by direct detection with the anti-ICAM-1 antibody of the invention, or with a circulating tumour cell-chip consisting of an array of microposts coated with the anti-ICAM-1 antibody of the invention

The antibody of the invention or the carrier of the invention can also be used in the phenotypic analysis of ICAM-1⁺ cellular subpopulations, in particular, of ICAM-1⁺ stem cells. e.g. as part of a microarray for phenotyping mammalian cells.

The antibody of the invention or carrier of the invention may also be used in the isolation or purification of cells expressing the ICAM-1 protein, for example mesenchymal stem cells. Thus, it is another aspect of the present invention the use of the antibody of the invention or the carrier of the invention in the isolation and/or purification of cells expressing the ICAM-1 protein, for example mesenchymal stem cells. The person skilled in the art will realise that the mesenchymal stem cells isolated using this application are useful in regenerative cell therapies.

One can use a wide variety of formats, either in solid or soluble phase, for the isolation and/or purification of cells expressing the ICAM-1 protein.

A particular application of the antibody of the invention or the carrier of the invention is in techniques such as leukopheresis. As used herein, the term "leukopheresis" refers to the procedure in which white blood cells are separated from the blood, and the remaining components are returned to the blood circulation. It is thus an extracorporeal therapy (a medical procedure performed outside the body). One way to carry out the leukopheresis is by using a selective leukopheresis column coupled with the antibody of the invention. The person skilled in the art will realise that this application will be useful, for example, in the treatment of inflammatory bowel disease (Eberhardson et al., 2013, Clin Immunol. 149:73-82).

All the terms and embodiments previously described are equally applicable to this aspect of the invention.

### Kits of the invention and uses thereof

The present invention also provides kits for carrying out the above-described methods and uses.

Thus, in a nineteenth aspect, the invention relates to a kit, hereinafter referred to as the "kit of the invention", comprising at least one antibody of the invention, the carrier of the invention, or the pharmaceutical composition of the invention.

The terms "antibody", "ICAM-1", "carrier", "subject", "disease or condition associated to the ICAM-1 protein expression" and their particulars have been described in detail in the context of the antibody of the invention, the carrier of the invention and in the first method of the invention and are used with the same meaning in the context of the kit of the invention.

In a particular embodiment, the kit of the invention further comprises at least an agent. In a more particular embodiment, the pharmaceutical composition of the invention comprises an agent selected from the group consisting of therapeutic agent, diagnostic agent, tracer agent, reporter, marker, cell, cell fragment, cell part, virus, viral fragment, viral part, conjugate and fusion protein. In a preferred embodiment of the kit of the invention, the agent is a therapeutic agent. The terms "agent", "therapeutic agent", "diagnostic agent", "tracer agent", "reporter", "marker", "cell", "cell fragment", "cell part", "virus", "viral fragment", "viral part", "conjugate" and "fusion protein" and their particulars have been described in the context of the carrier of the invention and are used with the same meaning in the context of the pharmaceutical composition of the invention. In a particular embodiment, said therapeutic agent can be conjugated to, or forming a fusion protein with, the antibody of the invention.

In the context of the present invention, "kit" is understood as a product containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

The kit of the invention can be used for diagnosing and/or prognosing a disease or condition associated to the ICAM-1 protein expression, including, among others, ICAM-1⁺ tumour diagnosis by using imaging techniques; or for monitoring the response to a treatment of a disease or condition associated to the ICAM-1 protein expression in a subject under treatment; or for the detection and/or quantification of the ICAM-1 protein, or cells expressing the ICAM-1 protein, present in a sample; or for the treatment and/or prevention of a disease or condition associated to the ICAM-1 protein expression, including, among others, treatment methods comprising killing the target cells (ICAM-1⁺) as well as treatment methods of a disease or condition associated to the ICAM-1 protein expression by depleting cells expressing the ICAM-1 or depleting ICAM-1 itself in said disease or condition, or for targeting a drug to a specific site wherein said specific site comprises the presence of the ICAM-1 protein, or cells expressing ICAM-1 (ICAM-1⁺ cells); or as a tool in biotechnology techniques, such as for example, biotechnology techniques, for detection, localization and/or quantification of the ICAM-1 protein protein in a sample.

This, in a twentieth aspect, the invention relates to the use of the kit of the invention:
- for the diagnosis and/or prognosis of a disease or condition associated to the ICAM-1 protein expression; or
- for monitoring the response to a treatment of a disease or condition associated to the ICAM-1 protein expression in a subject under treatment; or
- for detecting, localizing and/or quantifying the ICAM-1 protein, or cells expressing the ICAM-1 protein, present in a sample; or
- for the treatment and/or prevention of a disease or condition associated to the ICAM-1 protein expression; or
- for targeting a drug to a specific site wherein said specific site comprises the presence of the ICAM-1 protein or said specific site comprises cells expressing ICAM-1 (ICAM-1⁺ cells); or
- as a tool in biotechnology techniques for detection, localization and/or quantification of the ICAM-1 protein in a sample, or in the detection, localization and/or quantification of the ICAM-1 protein, or cells expressing the ICAM-1 protein, present in a sample.

In a preferred embodiment of the kit of the invention, the antibody of the invention is selected from the group consisting of:
(a) an antibody or an antigen-binding fragment thereof, wherein the antibody comprises the CDR1, CDR2 and CDR3 within the heavy chain of the variable region comprising respectively the sequences set forth in SEQ ID NO: 5, 6 and 7, or a functionally equivalent variant thereof, and the CDR1, CDR2 and CDR3 within the light chain comprising respectively the sequences set forth in SEQ ID NO: 8, 9 and 10, or a functionally equivalent variant thereof, which corresponds to the antibody identified as B4 in the examples; and
(b) an antibody or an antigen-binding fragment thereof, wherein the antibody comprises the CDR1, CDR2 and CDR3 within the heavy chain of the variable region comprising respectively the sequences set forth in SEQ ID NO: 11, 12 and 13, or a functionally equivalent variant thereof, and the CDR1, CDR2 and CDR3 within the light chain comprising respectively the sequences set forth in SEQ ID NO: 14, 15 and 16, or a functionally equivalent variant thereof, which corresponds to the antibody identified as B6 in the examples.

In a preferred embodiment of the kit of the invention, the antibody of the invention is selected from the group consisting of:
(a) an antibody or an antigen-binding fragment thereof, wherein the antibody comprises the FR1, FR2, FR3 and FR4 regions (FRs) within the heavy chain of the variable region comprising respectively the sequences set forth in SEQ ID NO: 17, 18, 19 and 20, or a functionally equivalent variant thereof, and the FR1, FR2, FR3 and FR4 regions (FRs) within the light chain of the variable region comprising respectively the sequences set forth in SEQ ID NO: 21, 22, 23 and 24, or a functionally equivalent variant thereof, which corresponds to the antibody identified as B4 in the examples; and
(b) an antibody or an antigen-binding fragment thereof, wherein the antibody comprises the FR1, FR2, FR3 and FR4 regions (FRs) within the heavy chain of the variable region comprising respectively the sequences set forth in SEQ ID NO: 25, 26, 27 and 28, or a functionally equivalent variant thereof, and the FR1, FR2, FR3 and FR4 regions within the light chain of the variable region comprising respectively the sequences set forth in SEQ ID NO: 29, 30, 31 and 32, or a functionally equivalent variant thereof, which corresponds to the antibody identified as B6 in the examples.

In a preferred embodiment of the kit of the invention, the antibody of the invention is selected from the group consisting of:
(a) an antibody or an antigen-binding fragment thereof, wherein the antibody comprises at least a heavy chain of the variable region (VH) comprising the sequence set forth in SEQ ID NO: 1, or a functionally equivalent variant thereof, and at least a light chain of the variable region (VL) comprising the sequence set forth in SEQ ID NO: 2, or a functionally equivalent variant thereof, which corresponds to the antibody identified as B4 in the examples; and
(b) an antibody or an antigen-binding fragment thereof, wherein the antibody comprises at least a heavy chain of the variable region (VH) comprising the sequence set forth in SEQ ID NO: 3, or a functionally equivalent variant thereof, and at least a light chain of the variable region (VL) comprising the sequence set forth in SEQ ID NO: 4, or a functionally equivalent variant thereof, which corresponds to the antibody identified as B6 in the examples.

All the terms and embodiments previously described are equally applicable to this aspect of the invention.

The invention is defined below by means of the following aspects:
1. An antibody binding specifically to the ICAM-1 protein or an antigen-binding fragment thereof, selected from the group consisting of:
   (a) an antibody or an antigen-binding fragment thereof, wherein the antibody comprises the CDR1, CDR2 and CDR3 within the heavy chain of the variable region comprising respectively the sequences set forth in SEQ ID NO: 5, 6 and 7, or a functionally equivalent variant thereof, and the CDR1, CDR2 and CDR3 within the light chain comprising respectively the sequences set forth in SEQ ID NO: 8, 9 and 10, or a functionally equivalent variant thereof; and
   (b) an antibody or an antigen-binding fragment thereof, wherein the antibody comprises the CDR1, CDR2 and CDR3 within the heavy chain of the variable region comprising respectively the sequences set forth in SEQ ID NO: 11, 12 and 13, or a functionally equivalent variant thereof, and the CDR1, CDR2 and CDR3 within the light chain comprising respectively the sequences set forth in SEQ ID NO: 14, 15 and 16, or a functionally equivalent variant thereof.
2. The antibody or antigen-binding fragment thereof according to aspect 1, wherein:
   (a) the FR1, FR2, FR3 and FR4 regions within the heavy chain of the variable region of the antibody or antigen-binding fragment thereof according to aspect 1(a) comprise respectively the sequences set forth in SEQ ID NO: 17, 18, 19 and 20, or a functionally equivalent variant thereof, and the FR1, FR2, FR3 and FR4 regions within the light chain of the variable region of the antibody or antigen-binding fragment thereof according to aspect 1(a) comprise respectively the sequences set forth in SEQ ID NO: 21, 22, 23 and 24, or a functionally equivalent variant thereof; and
   (b) the FR1, FR2, FR3 and FR4 within the heavy chain of the variable region of the antibody or antigen-binding fragment thereof according to aspect 1(b) comprise respectively the sequences set forth in SEQ ID NO: 25, 26, 27 and 28, or a functionally equivalent variant thereof, and the FR1, FR2, FR3 and FR4 regions within the light chain of the variable region of the antibody or antigen-binding fragment thereof according to aspect 1(b) comprise respectively the sequences set forth in SEQ ID NO: 29, 30, 31 and 32, or a functionally equivalent variant thereof.
3. The antibody or antigen-binding fragment thereof according to aspect 1 or 2, wherein:
   (a) at least a heavy chain of the variable region (VH) of the antibody or antigen-binding fragment thereof according to aspect 1(a) comprises the sequence set forth in SEQ ID NO: 1 or a functionally equivalent variant thereof, and at least a light chain of the variable region (VL) of the antibody or antigen-binding fragment thereof according to aspect 1(a) comprises the sequence set forth in SEQ ID NO: 2 or a functionally equivalent variant thereof; and
   (b) at least a heavy chain of the variable region (VH) of the antibody or antigen-binding fragment thereof according to aspect 1(b) comprises the sequence set forth in SEQ ID NO: 3 or a functionally equivalent variant thereof, and at least a light chain of the variable region (VL) of the antibody or antigen-binding fragment thereof according to aspect 1(b) comprises the sequence set forth in SEQ ID NO: 4 or a functionally equivalent variant thereof.
4. The antibody or antigen-binding fragment thereof according to any one of aspects 1 to 3, wherein said antibody is an animal antibody, a chimeric antibody, or a humanized antibody.
5. The antibody or antigen-binding fragment thereof according to any one of aspects 1 to 4, wherein said fragment is selected from the group consisting of Fv, Fab, F(ab')₂, Fab', scFv, (scFv)₂, scFv-Fc, minibody, nanobody, and diabody.
6. A carrier further comprising the antibody or antigen-binding fragment thereof according to any one of aspects 1 to 5.
7. The carrier according to aspect 6, wherein the antibody or antigen-binding fragment thereof is displayed on the surface of said carrier.
8. The carrier according to aspect 6 or 7, wherein said carrier further comprises at least an agent.
9. The carrier according to aspect 8, wherein the agent is selected from the group consisting of therapeutic agent, diagnostic agent, tracer agent, reporter, marker, cell, cell fragment, cell part, virus, viral fragment, viral part, conjugate and fusion protein.
10. The carrier according to aspect 9, wherein the agent is a therapeutic agent.
11. The carrier according to any one of aspects 6 to 10, wherein the carrier is selected from the group consisting of delivery system, conjugate, fusion protein, cell, cell-derived membrane, cell-derived vesicle, virus, viral-like particle and bacteria.
12. The carrier according to aspect 11, wherein said carrier is a delivery system.
13. The carrier according to aspect 12, wherein the delivery system is selected from the group consisting of polymeric nanoparticle, lipid nanoparticle, protein nanoparticle, nucleic acid nanoparticle, inorganic nanoparticle, polymeric microparticle, lipid microparticle, protein microparticle, nucleic acid microparticle, inorganic microparticle, liposome, polymersome, micelle and dendrimer.
14. The carrier according to aspect 12 or 13, wherein the delivery system is a polymeric nanoparticle, and wherein the polymeric nanoparticle is preferably a polystyrene nanoparticle.
15. The carrier according to aspect 14, wherein the delivery system is a polystyrene nanoparticle.
16. The carrier according to any one of aspects 6 to 15, wherein the delivery system is a polystyrene nanoparticle and the agent is a therapeutic agent
17. The carrier according to any one of aspects 12 to 16, wherein the antibody or antigen-binding fragment thereof according to any one of aspects 1 to 5 is adsorbed on the surface of the delivery system.
18. A nucleic acid selected form the group consisting of:
   (i) a nucleic acid coding for the antibody or antigen-binding fragment thereof according to any one of aspects 1 to 5, and
   (ii) a complementary nucleic acid of a nucleic acid as defined in (i).
19. A gene construct comprising a nucleic acid according to aspect 18.
20. An expression cassette comprising the nucleic acid according to aspect 18 or the gene construct according to aspect 19.
21. A vector comprising the nucleic acid according to aspect 18, the gene construct according to aspect 19, or the expression cassette according to aspect 20.
22. The vector according to aspect 21, wherein said vector is an expression vector.
23. A cell comprising the nucleic acid according to aspect 18, the gene construct according to aspect 19, the expression cassette according to aspect 20, or the vector according to aspect 21 or 22.
24. A living organism comprising the nucleic acid according to aspect 18, the gene construct according to aspect 19, the expression cassette according to aspect 20, or the vector according to aspect 21 or 22.
25. A method for producing the antibody or antigen-binding fragment thereof according to any one of aspects 1 to 5, comprising growing the cell according to aspect 20, or the living organism according to aspect 21, under conditions permitting the production of said antibody or antigen-binding fragment thereof.
26. An *in vitro* method for the diagnosis and/or prognosis of a disease or condition associated to the ICAM-1 protein expression in a subject, comprising:
   (a) contacting the antibody or antigen-binding fragment thereof according to any one of aspects 1 to 5, or the carrier according to any one of aspects 6 to 17, with a sample from said subject;
   (b) detecting and/or quantifying the level and/or location of said antibody or antigen-binding fragment thereof, or said carrier bound to the ICAM-1 protein in said sample;
   (c) comparing the level and/or location of said antibody or antigen-binding fragment thereof, or said carrier bound to the ICAM-1 protein detected in step (b) with that of a reference value; and
   (d) correlating the result obtained with the presence of a disease or condition associated to the ICAM-1 protein expression.
27. An *in vitro* method for monitoring the response to a treatment of a disease or condition associated to the ICAM-1 protein expression in a subject under treatment, comprising:
   (a) contacting the antibody or antigen-binding fragment thereof according to any one of aspects 1 to 5, or the carrier according to any one of aspects 6 to 17, with a first sample from said subject taken at a first time-point;
   (b) detecting and/or quantifying the level and/or location of said antibody or antigen-binding fragment thereof, or said carrier bound to the ICAM-1 protein in said first sample;
   (c) contacting the antibody or antigen-binding fragment thereof according to any one of aspects 1 to 5, or the carrier according to any one of aspects 6 to 17, with a second sample from said subject taken at a second time-point;
   (d) detecting and/or quantifying the level and/or location of said antibody or antigen-binding fragment thereof, or said carrier bound to the ICAM-1 protein in said second sample;
   (e) comparing the level and/or location of said antibody or antigen-binding fragment thereof, or said carrier bound to the ICAM-1 protein detected in step (b) with the level and/or location of said antibody or antigen-binding fragment thereof, or said carrier bound to the ICAM-1 protein detected in step (d); and
   (f) correlating the result obtained with the response to the treatment of a disease or condition associated to the ICAM-1 protein expression.
28. An *in vitro* method for detecting, localizing and/or quantifying the presence of the ICAM-1 protein, or cells expressing the ICAM-1 protein, in a sample, comprising:
   (i) contacting the test sample with an antibody or antigen-binding fragment thereof according to any one of aspects 1 to 5, or the carrier according to any one of aspects 6 to 17; and
   (ii) detecting, localizing and/or quantifying the formation of immune complexes with said antibody or antigen-binding fragment thereof, or said carrier.
29. A pharmaceutical composition comprising the antibody or antigen-binding fragment thereof according to any one of aspects 1 to 5, or the carrier according to any of aspects 6 to 17, and a pharmaceutically acceptable excipient.
30. The pharmaceutical composition according to aspect 29, wherein said pharmaceutical composition further comprises at least an agent.
31. The pharmaceutical composition according to aspect 30, wherein the agent is selected from the group consisting of therapeutic agent, diagnostic agent, tracer agent, reporter, marker, cell, cell fragment, cell part, virus, viral fragment, viral part, conjugate and fusion protein.
32. The pharmaceutical composition according to aspect 31, wherein the agent is a therapeutic agent.
33. The antibody or antigen-binding fragment thereof according to any one of aspects 1 to 5, the carrier according to any one of aspects 6 to 17, or the pharmaceutical composition according to any one of aspects 29 to 32, for use in medicine.
34. The antibody or antigen-binding fragment thereof according to any one of aspects 1 to 5, the carrier according to any one of aspects 6 to 17, or the pharmaceutical composition according to any one of aspects 29 to 32, for use in the treatment and/or prevention of a disease or condition associated to the ICAM-1 protein expression.
35. The *in vitro* method for diagnosis and/or prognosis according to aspect 26, the *in vitro* method for monitoring according to aspect 27, or the antibody or antigen-binding fragment thereof, the carrier, or the pharmaceutical composition for use according to aspect 34, wherein said disease or condition associated to the ICAM-1 protein expression is selected from the group consisting of cancer, an infectious disease, an inflammatory disease, a metabolic disease, a pulmonary disease, a cardiovascular disease, a neurological disease, a genetic disease, and an immune disease.
36. The *in vitro* method for diagnosis and/or prognosis, the *in vitro* method for monitoring, the antibody or antigen-binding fragment thereof, the carrier, or the pharmaceutical composition for use according to aspect 35, wherein:
   - the cancer is selected from the group consisting of lung cancer, mesothelioma, brain cancer, breast cancer, pancreatic cancer, colon cancer, ovarian cancer, prostate cancer, head and neck cancer, liver cancer, and leukemia;
   - the infectious disease is selected from the group consisting of common cold, COVID-19, herpes, acquired immunodeficiency syndrome (AIDS), influenza, tuberculosis, malaria, and an enteric viral infection;
   - the inflammatory disease is selected from the group consisting of asthma, diabetes, lupus, ulcerative colitis, psoriasis, chronic obstructive pulmonary disease, vasculitis, arthritis, hepatitis, systemic sclerosis, gout, myositis, and scleroderma;
   - the metabolic disease is selected from the group consisting of glycogen storage disease, hypercholesterolemia, obesity, diabetes, atherosclerosis, lysosomal storage disorder, fatty liver disease, and inborn errors of metabolism;
   - the pulmonary disease is selected from the group consisting of asthma, cystic fibrosis, acid sphingomyelinase deficiency, Fabry disease, pneumonia, bronchitis, pulmonary edema, pulmonary fibrosis, interstitial lung disease, pneumothorax, mesothelioma, lung cancers, acute respiratory distress syndrome, chronic obstructive pulmonary disease, emphysema, bronchitis, and pulmonary thrombosis;
   - the cardiovascular disease is selected from the group consisting of stroke, aneurysm, hypertension, cardiomyopathy, heart failure, heart attack, coronary artery disease, peripheral arterial disease, angina, heart valve disease, atrial fibrillation, arrhythmia, thrombosis, endocarditis, cerebrovascular disease, and atherosclerosis;
   - the neurological disease is selected from the group consisting of Parkinson's disease, Alzheimer's disease, spinal cord injury, amyotrophic lateral sclerosis, ataxia, epilepsy, depression, bipolar disorder, and schizophrenia, and congenital diseases that affect the central nervous system;
   - the genetic disease is selected from the group consisting of cystic fibrosis, familiar hypercholesterolemia, lysosomal storage disease, Gaucher disease, Pompe disease, Muccopolysaccharidosis, Niemann-Pick disease, acid sphingomyelinase disease, Fabry disease, Duchenne muscular dystrophy, sickle cell disease, congenital glycosylation disorders, and congenital heart disease; and/or
   - the immune disease is selected from the group consisting of inflammatory bowel disease, lupus, asthma, psoriasis, rheumatoid arthritis, multiple sclerosis, Hashimoto thyroiditis, Grave's disease, Type 1 diabetes, vitiligo, host versus graft disease, Crohn's disease, ulcerative colitis, celiac disease, Sjogren's syndrome, Kawasaki disease, and allergy.
37. The antibody or antigen-binding fragment thereof according to any one of aspects 1 to 5, the carrier according to any one of aspects 6 to 17, or the pharmaceutical composition according to any one of aspects 29 to 32, for use in a method of diagnosis and/or prognosis *in vivo* of a disease or condition associated to the ICAM-1 protein expression.
38. The use of the antibody or antigen-binding fragment thereof according to any one of aspects 1 to 5, the carrier according to any one of aspects 6 to 17, or the pharmaceutical composition according to any one of aspects 29 to 32, for targeting a drug to a specific site wherein said specific site comprises the presence of the ICAM-1 protein or said specific site comprises cells expressing ICAM-1 (ICAM-1⁺ cells).
39. Use of the antibody or antigen-binding fragment thereof according to any one of aspects 1 to 5, or the carrier according to any one of aspects 6 to 17, as a tool in biotechnology techniques for detection, localization and/or quantification of the ICAM-1 protein in a sample, or in the detection, localization and/or quantification of the ICAM-1 protein, or cells expressing the ICAM-1 protein, present in a sample.
40. A kit comprising at least one antibody or antigen-binding fragment thereof according to any one of aspects 1 to 5, the carrier according to any one of aspects 6 to 17, or the pharmaceutical composition according to any one of aspects 29 to 32.
41. The kit according to aspect 40, wherein said kit further comprises at least an agent.
42. The kit according to aspect 41, wherein the agent is selected from the group consisting of therapeutic agent, diagnostic agent, tracer agent, reporter, marker, cell, cell fragment, cell part, virus, viral fragment, and viral part.
43. The kit according to aspect 42, wherein the agent is a therapeutic agent.
44. Use of the kit according to any one of aspects 40 to 43:
   - for the diagnosis and/or prognosis of a disease or condition associated to the ICAM-1 protein expression; or
   - for monitoring the response to a treatment of a disease or condition associated to the ICAM-1 protein expression in a subject under treatment; or
   - for detecting, localizing and/or quantifying the ICAM-1 protein, or cells expressing the ICAM-1 protein, present in a sample; or
   - for the treatment and/or prevention of a disease or condition associated to the ICAM-1 protein expression; or
   - for targeting a drug to a specific site wherein said specific site comprises the presence of the ICAM-1 protein or said specific site comprises cells expressing ICAM-1 (ICAM-1⁺ cells); or
   - as a tool in biotechnology techniques for detection, localization and/or quantification of the ICAM-1 protein in a sample, or in the detection, localization and/or quantification of the ICAM-1 protein, or cells expressing the ICAM-1 protein, present in a sample.

The invention is described below by way of the following examples, which are merely illustrative and do not limit the scope of the invention.

### EXAMPLES

### Example 1: Identification of new anti-ICAM-1 antibodies

The new anti-ICAM-1 sequences were identified by several rounds of biopanning of an antibody (Ab) library (phage-display format) against purified, recombinant ICAM-1-Fc fusion protein used and an antigen (Ag). This was pursued by a biopanning procedure, consisting of several successive rounds of library enrichment against a mixture of the Ag corresponding to ICAM-1 sequences. The biopanning was followed by polyclonal (phage pools) and then monoclonal (single phage binders) ELISA screenings, finally rendering identification of 2 single clones (thereafter called B4 and B6), which bound specifically to recombinant ICAM-1 compared to saline control (Table 2).

**Table 2. Absorbance at 450 nm to detect binding of monoclonal (single phage) binders to ICAM-1-coated versus saline-coated (control) wells using anti-phage-HRP Ab.**

| **Coating** | **Clone ID (abbreviation)** | |
|---|---|---|
| | B4 | B6 |
| ICAM-1 | 1.05 | 2.25 |
| Saline | 0.04 | 0.02 |

Thereafter, the sequence of the variable heavy chain (VHC) and variable light chain (VLC) of these 2 unique binders was obtained (SEQ ID NO: 1 to 4, sequences in Table 1) and compared by multiple alignment to identify any possible identity or large sequence overlap among them.

### Example 2: Cellular interactions of new anti-ICAM-1 antibodies compared to commercial counterparts

Data described in Table 2 above demonstrate the ability of new anti-ICAM-1 Abs to recognize soluble ICAM-1, which is relevant for diagnostic purposes as this form of ICAM-1 is often considered a pathological marker. However, most ICAM-1 in the body is present as a protein anchored to the cell membrane. Hence, as shown in Figure 1, the sequences described above were recombinantly expressed as IgG-type Ab proteins (thereafter called new Abs B4 and B6 for simplicity), purified, tested for their ability to recognize specifically ICAM-1-expressing cells, and compared to previously existing commercial anti-ICAM-1 Abs (namely G-5 and H-4). For this purpose, HEK-293T cells recombinantly modified to express human ICAM-1 were incubated for 1 hour at 37 °C with each one of the new Abs, commercial Abs or with a non-specific IgG control. Then, cells were washed with cell medium to remove non-bound Abs, fixed with 2% paraformaldehyde, and incubated for 30 minutes with a secondary antibody linked to a red fluorophore to visualize the new and commercial Abs bound on the surface of cells. Next, cells were permeabilized with 0.1% Triton X-100 and incubated with a secondary Ab linked to a green fluorophore, so that the new Ab located both on the surface and inside cells could be visualized. Analysis was performed by confocal fluorescence microscopy. The total green fluorescence intensity, which is indicative of total Ab interaction with cells (Figure 1-left), showed that all four Abs tested associated specifically with cells (* indicates statistically significant values above IgG control levels). Yet, unexpectedly, both new Abs surpassed the two commercial Abs, despite the fact that these new Abs have not been optimized yet to maximize these parameters, contrary to most Abs commercially available. Additionally, in the same experiment described above, the green fluorescence that did not colocalize with cell-surface red fluorescence was examined, as this is indicative of Ab molecules internalized in cells (Figure 1-right). This analysis showed that all Abs were also specifically taken up by cells compared to IgG control and, again, the new anti-ICAM-1 Abs outperformed commercial Abs in this function.

Furthermore, not only the new anti-ICAM-1 Abs interacted with regular full-length ICAM-1 composed of five extracellular domains better than commercial counterparts (described above), but also with other ICAM-1 variants or isoforms of shorter molecular size (Figure 2). For instance, both new Abs B4 and B6 interacted more profusely than commercial Abs with cells recombinantly modified to express an ICAM-1 variant lacking one extracellular domain, herein called 4D ICAM-1 (Figure 2-left), or ICAM-1 lacking two extracellular domains, herein called 3D ICAM-1 (Figure 2-right).

Thus, altogether, this set of data demonstrates the unexpected superiority of the new anti-ICAM-1 Abs claimed in this invention (identified as B4 and B6) over commercial Abs (namely G-5 and H-4), when presented in solution.

### Example 3: Cellular interactions of nanoparticles coated with new anti-ICAM-1 antibodies compared to commercial counterparts

To then determine whether ICAM-1 could also be detected by the new anti-ICAM-1 Abs after being complexed to larger constructs, each one of these Abs was adsorbed on nanoparticles, to serve as models for diagnostic or therapeutic nanocarriers (NCs). This model consisted of 100 nm diameter polystyrene nanoparticles, onto which Abs were coated by surface adsorption. Polystyrene was selected since it is not biodegradable and its cellular transport can be tracked without confounding effects of degradation.

Briefly, nanoparticles were incubated for 1 h at room temperature with either new or commercial anti-ICAM-1 Abs or non-specific Ab (IgG control). Then, non-coated Ab was removed by centrifugation and the Ab-coated NC pellet was resuspended, sonicated, and analyzed by dynamic light scattering to measure their diameter, polydispersity index (PDI), and ζ-potential. Coating was determined by using Abs labeled with ¹²⁵Iodine and standard procedures, followed by analysis in a gamma counter as published (Ghaffarian R, et al., J Control Release, 2012). As shown in Table 3, uncoated NCs had 114 nm diameter, 0.03 PDI, and -25 mV ζ-potential and coating Abs on these NCs increased their diameter, PDI, and ζ-potential, as expected and validating successful coating. Indeed, coating efficiency was statistically similarly for all Abs, ranging from 134 to 179 Ab molecules per NC.

**Table 3. Characterization of model nanocarriers.**

| **NCs coating** | **Diameter (nm)** | **PDI** | **ζ-potential (mV)** | **Coating Ab molec./NCs** |
|---|---|---|---|---|
| **None** | 114 ± 0.5 | 0.03 ± 0.0 | -25.2 ± 0.7 | N/A |
| **IgG** | 223.2 ± 11.3 | 0.2 ± 0.03 | -19.8 ± 1.0 | 172.2 ± 17.2 |
| **B4** | 229.9 ± 7.5 | 0.2 ± 0.01 | -13.0 ± 0.4 | 134.5 ± 1.1 |
| **B6** | 289.4 ± 9.9 | 0.3 ± 0.01 | -12.3 ± 0.1 | 151.4 ± 1.3 |
| **G-5** | 241.2 ± 9.7 | 0.2 ± 0.01 | -18.6 ± 2.2 | 179.0 ± 41.8 |
| **H-4** | 223.3 ± 11.2 | 0.2 ± 0.02 | -20.6 ±1.9 | 167.5 ± 64.0 |

| | | | | |
|---|---|---|---|---|
| Molec. = molecules; N/A = not applicable; NCs = nanocarriers; PDI = polydispersity index. Data shown are average ± SEM. | | | | |

Using these nanoparticles, a similar experiment to the one conducted with Abs alone (Figure 1) was performed (Figure 3). The only difference was that nanoparticles contained a green fluorophore so that all nanoparticles located on the cell surface and those internalized could be directly visible by confocal microscopy without needing cell permeabilization. In cells recombinantly expressing ICAM-1 (Figure 3-left), all anti-ICAM-1 nanoparticles interacted specifically compared to control IgG. Those coated with new Ab B4 were slightly better than those coated with commercial Abs and, surprisingly, nanoparticles coated with new Ab B6 markedly surpassed commercial counterparts. Importantly, in cells naturally expressing ICAM-1 in high amounts under inflammatory signals (i.e. TNFα), which mimics a pathological situation aimed to be treated (Figure 3-right), both B4 nanoparticles and B6 nanoparticles largely outperformed those coated by commercial Abs. Therefore, the new anti-ICAM-1 Abs have improved function both when presented as soluble forms (Figure 1) and when presented as larger multimeric/multimolecular complexes, such as nanoparticles described here (Figure 3).

Next, cellular uptake of Ab-coated nanoparticles was examined (Figure 4). All Ab coated nanoparticles were also specifically internalized by cells compared to non-specific IgG NCs. Again, in cells recombinantly expressing ICAM-1, nanoparticles coated with new Ab B4 were slightly better than those coated with commercial Abs and new B6 nanoparticles markedly surpassed them (Figure 4-left). And again, both new Abs facilitated entrance of NCs into cells that naturally express ICAM-1 in pathological situations at a much higher extent than commercial Abs (Figure 4-right).

### Example 4: Subcellular transport of nanoparticles coated with new anti-ICAM-1 antibodies compared to commercial counterparts

Additionally to interacting with ICAM-1-expressing cells and being internalized by them, different transport routes were studied, which may provide different destinations. Particularly, trafficking into membrane-bound intracellular vesicles such as lysosomes was assessed since this transport route would retain Abs within cells for intracellular delivery. In contrast, trafficking across cells would provide a means to penetrate through stacks of cells in tissue culture or cellular barriers that separate body compartments, such as the one separating the circulation from the brain (blood-brain barrier; BBB).

First, to test lysosomal trafficking (Figure 5), cells were incubated for either 1 hour at 37 °C with green-labeled nanoparticles coated with new Abs B4 or B6 vs. a commercial counterpart as an example. Then, cells were washed with cell medium to remove non-interacting nanoparticles, fixed with 2% paraformaldehyde, permeabilized with 0.1% Triton X-100, and incubated with an Ab specific for LAMP-1 (a lysosomal marker) linked to a red fluorophore. Thus, nanoparticles that had trafficked to lysosomes would appear yellow (green+red) when visualized by confocal microscopy, compared to non-lysosomal nanoparticles (green alone). Quantification of microscopy images showed that both the rate of transport to lysosomes (Figure 5-left) and the absolute number of nanoparticles found in this compartment of cells (Figure 5-right) were superior in the case of new Abs compared to the commercial one.

With regards to transport across cells, an experiment was performed where human brain endothelial cells were grown to confluency on a porous Transwell filter, which renders a cellular layer that separates an apical chamber from a basolateral chamber. New Abs B4 and B6, compared to a commercial Ab (G-5 as an example) were labeled with ¹²⁵Iodine for radiotracing, then coated on nanoparticles, and next added to the apical chamber above the cells. After 1 hour incubation at 37 °C, non-interacting nanoparticles were removed from the apical cell medium and those interacting with cells on the filter or transported to the basolateral chamber were independently extracted and analyzed using a gamma counter. The rate of transport, understood as the percentage of transported nanoparticles from the total found in the system after 1 hour incubation, was greater for the formulations coated with new Abs B4 or B6 compared to the commercial counterpart (Figure 6-left). Given this, after 24 hours of transport, the total amount of Ab-coated nanoparticles transported across cells into the basolateral chamber was superior for new vs. commercial Abs (Figure 6-right). Therefore, the new Abs may be more useful to cross the BBB and enter the brain from the circulation.

A similar experiment was then conducted using human pulmonary endothelial cells grown to confluency on a porous Transwell filter separating apical and basolateral chambers (Figure 7), to generate a model to study whether Ab-coated nanoparticles may cross from the circulation into the lungs. Once again, Abs B4 and B6 were superior regarding both the rate of nanoparticle transport after 1 hour incubation (Figure 7-left) and total amount of nanoparticles transported after 24 hours of transport (Figure 7-right) compared to a commercial Ab (G-5 as an example).

### Example 5: Interactions of anti-ICAM-1 Ab-coated nanoparticles with cells expressing animal ICAM-1

Moreover, additionally to testing the ability of new anti-ICAM-1 Abs to interact in different manners with cells expressing human ICAM-1 shown in all figures above, their potential to target ICAM-1 from other species (cross-reactivity) was tested (Figure 8). In particular, dog and monkey ICAM-1 were capitalized since they are often used as animal models in pre-clinical trials during the development of diagnostic and therapeutic products, and they also hold relevance regarding veterinary medicine. For this purpose, HEK-293T cells were recombinantly modified to express ICAM-1 with sequences derived from these species and incubated with nanoparticles coated with new anti-ICAM-1 Abs B4 or B6 compared to those coated with a commercial Ab, namely G-5 as an example. All nanoparticles interacted specifically with cells expressing dog-derived ICAM-1 (Figure 8-left) and monkey-derived ICAM-1 (Figure 8-right). In the case of dog, B6-coated nanoparticles slightly surpassed those coated with the commercial Ab and B4-coated counterparts greatly outperformed them. In the case of monkey both new Abs markedly surpassed the commercial counterpart. Therefore, the new anti-ICAM-1 Abs claimed in this invention hold great potential for applications involving not only human ICAM-1 but also ICAM-1 from these other species.

### Conclusions

Altogether, in view of all these results, two new Ab sequences (particularly, VHC and VLC) have been identified which: (a) recognize ICAM-1 as a purified protein, therefore a protein in solution, or a protein expressed on/in cells, (b) can bind to ICAM-1 from human and several animal species, (c) recognize also various ICAM-1 variants, (d) are additionally capable of transport into cells, into subcellular compartments such as lysosomes, and/or across cells, (e) are active as single molecules or as nanosized constructs, and (f) they surpass the efficiency of commercial Abs in all these functions.

On the whole, these new Abs recognizing ICAM-1 could be used in a variety of ways. For instance, according to the functions and performance described above, they could be used as tools to detect, measure, and/or alter either human ICAM-1 or ICAM-1 from respective species described above, thus, as markers and/or modifiers of the patho-physiological states associated with ICAM-1, for either research, diagnostic, and/or therapeutic purposes. They could also be employed to induce transport into cells, to lysosomes, and/or across cells, which in turn could be valuable to transport diagnostic and/or therapeutic agents to these destinations. Additionally, these new Abs could be used for blocking or occupying ICAM-1 binding sites to modulate its functions, and/or to address other bioactive agents to disease sites where ICAM-1 is expressed, for either preventive or therapeutic intervention, in the form of linking such agents to these Abs directly or by loading such agents in drug delivery systems coupled to these Abs. An advantage of these new Abs sequences is that they outperformed commercial ones in all these functions and cross-reactivities, which could not be predicted a priori.

## Claims

1. An antibody binding specifically to the ICAM-1 protein or an antigen-binding fragment thereof, selected from the group consisting of:
(a) an antibody or an antigen-binding fragment thereof, wherein the antibody comprises the CDR1, CDR2 and CDR3 within the heavy chain of the variable region comprising respectively the sequences set forth in SEQ ID NO: 5, 6 and 7, or a functionally equivalent variant thereof, and the CDR1, CDR2 and CDR3 within the light chain comprising respectively the sequences set forth in SEQ ID NO: 8, 9 and 10, or a functionally equivalent variant thereof; and
(b) an antibody or an antigen-binding fragment thereof, wherein the antibody comprises the CDR1, CDR2 and CDR3 within the heavy chain of the variable region comprising respectively the sequences set forth in SEQ ID NO: 11, 12 and 13, or a functionally equivalent variant thereof, and the CDR1, CDR2 and CDR3 within the light chain comprising respectively the sequences set forth in SEQ ID NO: 14, 15 and 16, or a functionally equivalent variant thereof.

2. A carrier further comprising the antibody or antigen-binding fragment thereof according to claim 1.

3. The carrier according to claim 2, further comprising at least an agent selected from the group consisting of therapeutic agent, diagnostic agent, tracer agent, reporter, marker, cell, cell fragment, cell part, virus, viral fragment, viral part, conjugate and fusion protein, preferably the agent is a therapeutic agent, preferably wherein the agent is a therapeutic agent.

4. The carrier according to claim 2 or 3, wherein the carrier is selected from the group consisting of delivery system, conjugate, fusion protein, cell, cell-derived membrane, cell-derived vesicle, virus, viral-like particle and bacteria, preferably wherein the delivery system is a polymeric nanoparticle, and more preferably wherein the polymeric nanoparticle is a polystyrene nanoparticle.

5. A nucleic acid selected form the group consisting of:
(i) a nucleic acid coding for the antibody or antigen-binding fragment thereof according to claim 1, and
(ii) a complementary nucleic acid of a nucleic acid as defined in (i).

6. A cell comprising the nucleic acid according to claim 5.

7. An *in vitro* method for the diagnosis and/or prognosis of a disease or condition associated to the ICAM-1 protein expression in a subject, comprising:
(a) contacting the antibody or antigen-binding fragment thereof according to claim 1, or the carrier according to any one of claims 2 to 4, with a sample from said subject;
(b) detecting and/or quantifying the level and/or location of said antibody or antigen-binding fragment thereof, or said carrier bound to the ICAM-1 protein in said sample;
(c) comparing the level and/or location of said antibody or antigen-binding fragment thereof, or said carrier bound to the ICAM-1 protein detected in step (b) with that of a reference value; and
(d) correlating the result obtained with the presence of a disease or condition associated to the ICAM-1 protein expression.

8. An *in vitro* method for monitoring the response to a treatment of a disease or condition associated to the ICAM-1 protein expression in a subject under treatment, comprising:
(a) contacting the antibody or antigen-binding fragment thereof according to claim 1, or the carrier according to any one of claims 2 to 4, with a first sample from said subject taken at a first time-point;
(b) detecting and/or quantifying the the level and/or location of said antibody or antigen-binding fragment thereof, or said carrier bound to ICAM-1 protein in said first sample;
(c) contacting the antibody or antigen-binding fragment thereof according to claim 1, or the carrier according to any one of claims 2 to 4, with a second sample from said subject taken at a second time-point;
(d) detecting and/or quantifying the level and/or location of said antibody or antigen-binding fragment thereof, or said carrier bound to the ICAM-1 protein in said second sample;
(e) comparing the level and/or location of said antibody or antigen-binding fragment thereof, or said carrier bound to the ICAM-1 protein detected in step (b) with the level and/or location of said antibody or antigen-binding fragment thereof, or said carrier bound to the ICAM-1 protein detected in step (d); and
(f) correlating the result obtained with the response to the treatment of a disease or condition associated to the ICAM-1 protein expression.

9. An *in vitro* method for detecting, localizing and/or quantifying the presence of the ICAM-1 protein, or cells expressing the ICAM-1 protein, in a sample, comprising:
(i) contacting the test sample with an antibody or antigen-binding fragment thereof according to claim 1, or the carrier according to any one of claims 2 to 4; and
(ii) detecting, localizing and/or quantifying the formation of immune complexes with said antibody or antigen-binding fragment thereof, or said carrier.

10. A pharmaceutical composition comprising the antibody or antigen-binding fragment thereof according to claim 1, or the carrier according to any of claims 2 to 4, and a pharmaceutically acceptable excipient.

11. The antibody or antigen-binding fragment thereof according to claim 1, the carrier according to any one of claims 2 to 4, or the pharmaceutical composition according to claim 10, for use in medicine.

12. The antibody or antigen-binding fragment thereof according to claim 1, the carrier according to any one of claims 2 to 4, or the pharmaceutical composition according to claim 10, for use in the treatment and/or prevention of a disease or condition associated to the ICAM-1 protein expression.

13. The *in vitro* method for diagnosis and/or prognosis according to claim 7, the *in vitro* method for monitoring according to claim 8, or the antibody or antigen-binding fragment thereof, the carrier, or the pharmaceutical composition for use according to claim 12, wherein said disease or condition associated to the ICAM-1 protein expression is selected from the group consisting of cancer, an infectious disease, an inflammatory disease, a metabolic disease, a pulmonary disease, a cardiovascular disease, a neurological disease, a genetic disease, and an immune disease.

14. A kit comprising at least one antibody or antigen-binding fragment thereof according to claim 1, the carrier according to any one of claims 2 to 4, or the pharmaceutical composition according to claim 10.

15. Use of the kit according to claim 14:
- for the diagnosis and/or prognosis of a disease or condition associated to the ICAM-1 protein expression; or
- for monitoring the response to a treatment of a disease or condition associated to the ICAM-1 protein expression in a subject under treatment; or
- for detecting, localizing and/or quantifying the ICAM-1 protein, or cells expressing the ICAM-1 protein, present in a sample; or
- for the treatment and/or prevention of a disease or condition associated to the ICAM-1 protein expression; or
- for targeting a drug to a specific site wherein said specific site comprises the presence of the ICAM-1 protein or said specific site comprises cells expressing ICAM-1 (ICAM-1⁺ cells); and/or
- as a tool in biotechnology techniques for detection, localization and/or quantification of the ICAM-1 protein in a sample, or in the detection, localization and/or quantification of the ICAM-1 protein, or cells expressing the ICAM-1 protein, present in a sample.
